# EUROPÄISCHE PATENTSCHRIFT

(11) **EP 2 831 050 B1**
(45) Veröffentlichungstag und Bekanntmachung des Hinweises auf die Patenterteilung: **23.03.2016**
(21) Anmeldenummer: 13712777.5
(22) Anmeldetag: 27.03.2013
(51) Int. Cl.: C07D 239/42, C07D 401/12, C07D 403/12, C07D 405/12, C07D 409/12, A01N 43/48

(54) **5-AMINO-PYRIMIDINDERIVATE UND DEREN VERWENDUNG ZUR BEKÄMPFUNG UNERWÜNSCHTEN PFLANZENWACHSTUMS**
5-AMINO PYRIMIDINE DERIVATIVES AND USE THEREOF FOR COMBATING UNDESIRED PLANT GROWTH
DÉRIVÉS DE 5-AMINO-PYRIMIDINE ET LEUR UTILISATION POUR LUTTER CONTRE LA CROISSANCE INDÉSIRABLE DE PLANTES

(30) Priorität: 29.03.2012 EP 12162187
(43) Veröffentlichungstag der Anmeldung: 04.02.2015
(73) Patentinhaber: Bayer Intellectual Property GmbH, 40789 Monheim (DE)
(72) Erfinder: MINN, Klemens, 65795 Hattersheim (DE); HOFFMANN, Michael, Gerhard, 65439 Flörsheim (DE); GATZWEILER, Elmar, 61231 Bad Nauheim (DE); HEINEMANN, Ines, 65719 Hofheim (DE); HÄUSER-HAHN, Isolde, 51375 Leverkusen (DE); ROSINGER, Christopher, 65719 Hofheim am Taunus (DE)
(74) Vertreter: BIP Patents
(86) Internationale Anmeldenummer: PCT/EP2013/056483
(87) Internationale Veröffentlichungsnummer: WO 2013/144187

(56) Entgegenhaltungen:
- WO-A1-2010/076009
- WO-A1-2010/076010

## Beschreibung

Die Erfindung betrifft das technische Gebiet der Pflanzenschutzmittel, insbesondere das der Herbizide zur selektiven Bekämpfung von Unkräutern und Ungräsern in Nutzpflanzenkulturen sowie im Ziergartenbereich und zur generellen Bekämpfung von Unkräutern und Ungräsern in Umweltbreichen, in denen Pflanzenwuchs störend ist.

Insbesondere betrifft die Erfindung substituierte 5-Amino-Pyrimidine, Verfahren zu deren Herstellung sowie deren Verwendung zur Bekämpfung von Schadpflanzen.

Aus dem Stand der Technik sind Diaminopyrimidine bekannt, welche eine herbizide Wirkung aufweisen. So werden 2,4-Diaminopyrimidine und deren Anwendung im Bereich des Pflanzenschutzes beispielsweise durch die Dokumente EP 0523533 A1 WO 2010/076009 und WO 2010/076010 offenbart.

Die Anwendung der Derivate dieses Typs als selektive Herbizide zur Schadpflanzenbekämpfung oder als Pflanzenwachstumsregulatoren in verschiedenen Nutzpflanzenkulturen erfordert jedoch häufig eine hohe Kosten verursachende Aufwandmenge oder führt zu unerwünschten Schädigungen der Nutzpflanzen. Zudem ist die Anwendung der Wirkstoffe in vielen Fällen wegen verhältnismäßig hoher Herstellkosten unwirtschaftlich.

Es ist deshalb erstrebenswert, alternative chemische Wirkstoffe auf Basis von Pyrimidinderivaten bereitzustellen, die als Herbizide oder Pflanzenwachstumsregulatoren eingesetzt werden können und mit welchen bestimmte Vorteile im Vergleich zu aus dem Stand der Technik bekannten Systemen verbunden sind.

Die Aufgabe der vorliegenden Erfindung besteht in der Bereitstellung alternativer Pyrimidin-Derivate, welche als Herbizide oder Pflanzenwachstumsregulatoren, mit einer zufriedenstellenden herbiziden Wirkung und einem breiten Wirkspektrum gegenüber Schadpflanzen und/oder mit einer hohen Selektivität in Nutzpflanzenkulturen, eingesetzt werden können.

Diese Pyrimidin-Derivate sollen im Vergleich zu den aus dem Stand der Technik bekannten Pyrimidinderivate ein besseres Eigenschaftsprofil, insbesondere eine bessere herbizide Wirkung gegen Schadpflanzen, ein breiteres Spektrum gegenüber Schadpflanzen und/oder eine höhere Selektivität in Nutzpflanzenkulturen zeigen.

Erfindungsgemäß wurden nun speziell substituierte 5-Amino-pyrimidine der Formel (I) gefunden, welche vorteilhaft als Herbizide und Pflanzenwachstumsregulatoren eingesetzt werden können.

Die erfindungsgemäßen Verbindungen der Formel (I) weisen, in der 5-Positon des Pyrimidines einen in der alpha Position zum Aromaten über ein Amin gebundenen teilhydrierten bicyclischen Substituienten auf.

Die erfindungsgemäßen 5-Aminopyrimidinderivate der Formel (I) unterscheiden sich von den bekannten Herbiziden mit 2,4-Diaminopyrimidin-Struktur in der Positionierung und in der Zahl der Aminogruppen. Die erfindungsgemäßen 5-Aminopyrimidine zeichnen sich dadurch aus, dass sie nur durch eine Aminogruppe substituiert sind, wobei sich diese durch einen bicyclischen Rest substituierte Aminogruppe in 5-Position des Pyrimidins befindet.

Somit stehen die Reste R¹, R^{2a} und R^{2b} in den Verbindungen der Formel (I) nicht für eine Aminogruppe, d.h. die R¹, R^{2a} und R^{2b} sind nicht über ein Stickstoffatom an das Pryimidin gebunden. Gleichwohl können die Reste R¹, R^{2a} und R^{2b} eine Aminogruppe, bzw. ein Amidgruppen (z.B. Aminocarbonyl-(C₁-C₆)-alkyl, Di-(C₁-C₆)-Alkylaminocarbonyl-(C₁-C₆)-alkyl) enthalten.

Gegenstand der vorliegenden Erfindung sind Verbindungen der Formel (I) 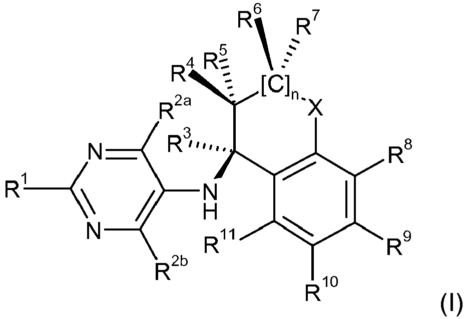 und deren agrochemisch verträglichen Salze, in welchen
R¹ ausgewählt ist aus der Gruppe, bestehend aus
   - Cyano, Nitro, C(O)OH, C(O)NH₂;
   - (C₁-C₆)-Alkyl, (C₁-C₆)-Halogenalkyl, (C₁-C₆)-Alkylcarbonyl, (C₁-C₆)-Halo-alkylcarbonyl, (C₁-C₆)-Alkylcarbonyloxy, (C₁-C₆)-Halogenalkylcarbonyloxy, (C₁-C₆)-Alkylcarbonyl-(C₁-C₄)-alkyl;
   - (C₁-C₆)-Alkoxy, (C₁-C₆)-Halogenalkoxy, (C₁-C₆)-Alkoxycarbonyl, (C₁-C₆)-Haloalkoxycarbonyl, (C₁-C₆)-Alkoxycarbonyl-(C₁-C₆)-alkyl, (C₁-C₆)-Halo-alkoxycarbonyl-(C₁-C₆)-alkyl, (C₁-C₆)-Alkoxycarbonyl-(C₁-C₆)-halogenalkyl, (C₁-C₆)-Halogenalkoxycarbonyl-(C₁-C₆)-halogenalkyl;
   - (C₂-C₆)-Alkenyl, (C₂-C₆)-Halogenalkenyl, (C₂-C₆)-Alkenylcarbonyl, (C₂-C₆)-Haloalkenylcarbonyl, (C₂-C₆)-Alkenyloxy, (C₂-C₆)-Haloalkenyloxy, (C₂-C₆)-Alkenyloxycarbonyl, (C₂-C₆)-Haloalkenyloxycarbonyl;
   - (C₂-C₆)-Alkinyl, (C₂-C₆)-Halogenalkinyl, (C₂-C₆)-Alkinylcarbonyl, (C₂-C₆)-Haloalkinylcarbonyl, (C₂-C₆)-Alkinyloxy, (C₂-C₆)-Haloalkinyloxy, (C₂-C₆)-Alkinyloxycarbonyl, (C₂-C₆)-Halogenalkinyloxycarbonyl;
   - Tri-(C₁-C₆)-alkylsilyl-(C₂-C₆)-alkinyl, Di-(C₁-C₆)-alkylsilyl-(C₂-C₆)-alkinyl, Mono-(C₁-C₆)-alkylsilyl-(C₂-C₆)-alkinyl; Phenylsilyl-(C₂-C₆)-alkinyl;
   - (C₆-C₁₄)-Aryl, (C₆-C₁₄)-Aryloxy, (C₆-C₁₄)-Arylcarbonyl und (C₆-C₁₄)-Aryl-oxycarbonyl, welche jeweils am Arylteil mit Halogen, (C₁-C₆)-Alkyl und/oder (C₁-C₆)-Haloalkyl substituiert sein können;
   - (C₆-C₁₄)-Aryl-(C₁-C₆)-alkyl, (C₆-C₁₄)-Aryl-(C₁-C₆)-alkoxy, (C₆-C₁₄)-Aryl-(C₁-C₆)-alkyl-carbonyl, (C₆-C₁₄)-Aryl-(C₁-C₆)-alkyl-carbonyloxy, (C₆-C₁₄)-Aryl-(C₁-C₆)-alkoxycarbonyl, (C₆-C₁₄)-Aryl-(C₁-C₆)-alkoxycarbonyloxy;
   - Aminocarbonyl-(C₁-C₆)-alkyl, Di-(C₁-C₆)-Alkylaminocarbonyl-(C₁-C₆)-alkyl;
   - Mono-((C₁-C₆)-alkyl)-amino-carbonyl, Mono-((C₁-C₆)-haloalkyl)-amino-carbonyl, Di-((C₁-C₆)-alkyl)-amino-carbonyl, Di-((C₁-C₆)-haloalkyl)-amino-carbonyl, ((C₁-C₆)-Alkyl-(C₁-C₆)-haloalkyl)-amino-carbonyl, N-((C₁-C₆)-Al-kanoyl)-amino-carbonyl, N-((C₁-C₆)-Haloalkanoyl)-amino-carbonyl, Mono-((C₆-C₁₄)-aryl)-amino-carbonyl, Di-((C₆-C₁₄)-aryl)-amino-carbonyl;
   - (C₁-C₆)-Alkoxy-(C₁-C₆)-alkyl, (C₁-C₆)-Alkoxy-(C₁-C₆)-alkoxy, (C₁-C₆)-Alk-oxycarbonyl-(C₁-C₆)-alkoxy;
   - (C₃-C₈)-Cycloalkyl, welches gegebenenfalls am Cycloalkylrest durch (C₁-C₆)-Alkyl und/oder Halogen substituiert sein kann; (C₃-C₈)-Cycloalkoxy, (C₃-C₈)-Cycloalkyl-(C₁-C₆)-alkyl, (C₃-C₈)-Cycloalkyl-(C₁-C₆)-haloalkyl, (C₃-C₈)-Cycloalkyl-(C₁-C₆)-alkoxy, (C₃-C₈)-Cycloalkyl-(C₁-C₆)-haloalkoxy, (C₃-C₈)-Cycloalkylcarbonyl, (C₃-C₈)-Cycloalkoxycarbonyl, (C₃-C₈)-Cycloalkyl-(C₁-C₆)-alkylcarbonyl, (C₃-C₈)-Cycloalkyl-(C₁-C₆)-haloalkylcarbonyl, (C₃-C₈)-Cycloalkyl-(C₁-C₆)-alkoxycarbonyl, (C₃-C₈)-Cycloalkyl-(C₁-C₆)-haloalkoxycarbonyl, (C₃-C₈)-Cycloalkylcarbonyloxy, (C₃-C₈)-Cycloalkoxycarbonyloxy, (C₃-C₈)-Cycloalkyl-(C₁-C₆)-alkylcarbonyloxy, (C₃-C₈)-Cycloalkyl-(C₁-C₆)-halogenalkylcarbonyloxy, (C₃-C₈)-Cycloalkyl-(C₁-C₆)-alkoxycarbonyloxy, (C₃-C₈)-Cycloalkyl-(C₁-C₆)-haloalkoxycarbonyloxy;
   - (C₃-C₈)-Cycloalkenyl, (C₃-C₈)-Cycloalkenyloxy, (C₃-C₈)-Cycloalkenyl-(C₁-C₆)-alkyl, (C₃-C₈)-Cycloalkenyl-(C₁-C₆)-halogenalkyl, (C₃-C₈)-Cycloalkenyl-(C₁-C₆)-alkoxy, (C₃-C₈)-Cycloalkenyl-(C₁-C₆)-halogenalkoxy, (C₃-C₈)-Cycloalkenylcarbonyl, (C₃-C₈)-Cycloalkenyloxycarbonyl, (C₃-C₈)-Cycloalkenyl-(C₁-C₆)-alkylcarbonyl, (C₃-C₈)-Cycloalkenyl-(C₁-C₆)-halogenalkylcarbonyl, (C₃-C₈)-Cycloalkenyl-(C₁-C₆)-alkoxycarbonyl, (C₃-C₈)-Cycloalkenyl-(C₁-C₆)-haloalkoxycarbonyl, (C₃-C₈)-Cycloalkenylcarbonyloxy, (C₃-C₈)-Cycloalkenyloxycarbonyloxy, (C₃-C₈)-Cycloalkenyl-(C₁-C₆)-alkylcarbonyloxy, (C₃-C₈)-Cycloalkenyl-(C₁-C₆)-halogenalkylcarbonyloxy, (C₃-C₈)-Cycloalkenyl-(C₁-C₆)-alkoxycarbonyloxy, (C₃-C₈)-Cycloalkenyl-(C₁-C₆)-haloalkoxycarbonyloxy;
   - Hydroxy-(C₁-C₆)-alkyl, Hydroxy-(C₁-C₆)-alkoxy Cyano-(C₁-C₆)-alkoxy, Cyano-(C₁-C₆)-alkyl;
   - (C₁-C₆)-Alkylsulfonyl, (C₁-C₆)-Alkylthio, (C₁-C₆)-Alkylsulfinyl, (C₁-C₆)-Haloalkylsulfonyl, (C₁-C₆)-Halogenalkylthio, (C₁-C₆)-Halogenalkylsulfinyl, (C₁-C₆)-Alkylsulfonyl-(C₁-C₆)-alkyl, (C₁-C₆)-Alkylthio-(C₁-C₆)-alkyl, (C₁-C₆)-Alkylsulfinyl-(C₁-C₆)-alkyl, (C₁-C₆)-Haloalkylsulfonyl-(C₁-C₆)-alkyl, (C₁-C₆)-Haloalkylthio-(C₁-C₆)-alkyl, (C₁-C₆)-Haloalkylsulfinyl-(C₁-C₆)-alkyl, (C₁-C₆)-Alkylsulfonyl-(C₁-C₆)-haloalkyl, (C₁-C₆)-Alkylthio-(C₁-C₆)-haloalkyl, (C₁-C₆)-Alkylsulfinyl-(C₁-C₆)-haloalkyl, (C₁-C₆)-Haloalkylsulfonyl-(C₁-C₆)-haloalkyl, (C₁-C₆)-Haloalkylthio-(C₁-C₆)-haloalkyl, (C₁-C₆)-Haloalkylsulfinyl-(C₁-C₆)-haloalkyl, (C₁-C₆)-Alkylsulfonyloxy, (C₁-C₆)-Halogenalkylsulfonyloxy, (C₁-C₆)-Alkylthiocarbonyl, (C₁-C₆)-Haloalkylthiocarbonyl, (C₁-C₆)-Alkylthiocarbonyloxy, (C₁-C₆)-Haloalkyl-thiocarbonyloxy, (C₁-C₆)-Alkylthio-(C₁-C₆)-alkyl, (C₁-C₆)-Alkylthio-(C₁-C₆)-alkoxy, (C₁-C₆)-Alkylthio-(C₁-C₆)-alkylcarbonyl, (C₁-C₆)-Alkylthio-(C₁-C₆)-alkylcarbonyloxy; (C₄-C₁₄)-Arylsulfonyl, (C₆-C₁₄)-Arylthio, (C₆-C₁₄)-Arylsulfinyl, (C₃-C₈)-Cycloalkylthio, (C₃-C₈)-Alkenylthio, (C₃-C₈)-Cycloalkenylthio, (C₃-C₆)-Alkinylthio; und
R^{2a} und R^{2b}, jeweils unabhängig voneinander, ausgewählt sind aus der Gruppe, bestehend aus
   - Wasserstoff, Halogen, Hydroxy, Cyano, Nitro, C(O)OH, C(O)NH₂;
   - (C₁-C₆)-Alkyl, (C₁-C₆)-Halogenalkyl, (C₁-C₆)-Alkylcarbonyl, (C₁-C₆)-Halo-alkylcarbonyl, (C₁-C₆)-Alkylcarbonyloxy, (C₁-C₆)-Halogenalkylcarbonyloxy, (C₁-C₆)-Alkylcarbonyl-(C₁-C₄)-alkyl;
   - (C₁-C₆)-Alkoxy, (C₁-C₆)-Halogenalkoxy, (C₁-C₆)-Alkoxycarbonyl, (C₁-C₆)-Haloalkoxycarbonyl, (C₁-C₆)-Alkoxycarbonyl-(C₁-C₆)-alkyl, (C₁-C₆)-Halo-alkoxycarbonyl-(C₁-C₆)-alkyl, (C₁-C₆)-Alkoxycarbonyl-(C₁-C₆)-halogenalkyl, (C₁-C₆)-Halogenalkoxycarbonyl-(C₁-C₆)-halogenalkyl;
   - (C₂-C₆)-Alkenyl, (C₂-C₆)-Halogenalkenyl, (C₂-C₆)-Alkenylcarbonyl, (C₂-C₆)-Haloalkenylcarbonyl, (C₂-C₆)-Alkenyloxy, (C₂-C₆)-Haloalkenyloxy, (C₂-C₆)-Alkenyloxycarbonyl, (C₂-C₆)-Haloalkenyloxycarbonyl;
   - (C₂-C₆)-Alkinyl, (C₂-C₆)-Halogenalkinyl, (C₂-C₆)-Alkinylcarbonyl, (C₂-C₆)-Haloalkinylcarbonyl, (C₂-C₆)-Alkinyloxy, (C₂-C₆)-Haloalkinyloxy, (C₂-C₆)-Alkinyloxycarbonyl, (C₂-C₆)-Halogenalkinyloxycarbonyl;
   - Tri-(C₁-C₆)-alkylsilyl-(C₂-C₆)-alkinyl, Di-(C₁-C₆)-alkylsilyl-(C₂-C₆)-alkinyl, Mono-(C₁-C₆)-alkylsilyl-(C₂-C₆)-alkinyl; Phenylsilyl-(C₂-C₆)-alkinyl;
   - (C₆-C₁₄)-Aryl, (C₆-C₁₄)-Aryloxy, (C₆-C₁₄)-Arylcarbonyl und (C₆-C₁₄)-Aryl-oxycarbonyl, welche jeweils am Arylteil mit Halogen, (C₁-C₆)-Alkyl und/oder (C₁-C₆)-Haloalkyl substituiert sein können;
   - (C₆-C₁₄)-Aryl-(C₁-C₆)-alkyl, (C₆-C₁₄)-Aryl-(C₁-C₆)-alkoxy, (C₆-C₁₄)-Aryl-(C₁-C₆)-alkyl-carbonyl, (C₆-C₁₄)-Aryl-(C₁-C₆)-alkyl-carbonyloxy, (C₆-C₁₄)-Aryl-(C₁-C₆)-alkoxycarbonyl, (C₆-C₁₄)-Aryl-(C₁-C₆)-alkoxycarbonyloxy;
   - Aminocarbonyl-(C₁-C₆)-alkyl, Di-(C₁-C₆)-Alkylaminocarbonyl-(C₁-C₆)-alkyl;
   - N-((C₁-C₆)-Haloalkanoyl)-amino-carbonyl, Mono-((C₆-C₁₄)-aryl)-amino-carbonyl, Di-((C₆-C₁₄)-aryl)-amino-carbonyl;
   - (C₁-C₆)-Alkoxy-(C₁-C₆)-alkyl, (C₁-C₆)-Alkoxy-(C₁-C₆)-alkoxy, (C₁-C₆)-Alk-oxycarbonyl-(C₁-C₆)-alkoxy;
   - (C₃-C₈)-Cycloalkyl, welches gegebenenfalls am Cycloalkylrest durch (C₁-C₆)-Alkyl und/oder Halogen substituiert sein kann; (C₃-C₈)-Cycloalkoxy, (C₃-C₈)-Cycloalkyl-(C₁-C₆)-alkyl, (C₃-C₈)-Cycloalkyl-(C₁-C₆)-haloalkyl, (C₃-C₈)-Cycloalkyl-(C₁-C₆)-alkoxy, (C₃-C₈)-Cycloalkyl-(C₁-C₆)-haloalkoxy, (C₃-C₈)-Cycloalkylcarbonyl, (C₃-C₈)-Cycloalkoxycarbonyl, (C₃-C₈)-Cycloalkyl-(C₁-C₆)-alkylcarbonyl, (C₃-C₈)-Cycloalkyl-(C₁-C₆)-haloalkylcarbonyl, (C₃-C₈)-Cycloalkyl-(C₁-C₆)-alkoxycarbonyl, (C₃-C₈)-Cycloalkyl-(C₁-C₆)-haloalkoxycarbonyl, (C₃-C₈)-Cycloalkylcarbonyloxy, (C₃-C₈)-Cycloalkoxycarbonyloxy, (C₃-C₈)-Cycloalkyl-(C₁-C₆)-alkylcarbonyloxy, (C₃-C₈)-Cycloalkyl-(C₁-C₆)-halogenalkylcarbonyloxy, (C₃-C₈)-Cycloalkyl-(C₁-C₆)-alkoxycarbonyloxy, (C₃-C₈)-Cycloalkyl-(C₁-C₆)-haloalkoxycarbonyloxy;
   - (C₃-C₈)-Cycloalkenyl, (C₃-C₈)-Cycloalkenyloxy, (C₃-C₈)-Cycloalkenyl-(C₁-C₆)-alkyl, (C₃-C₈)-Cycloalkenyl-(C₁-C₆)-halogenalkyl, (C₃-C₈)-Cycloalkenyl-(C₁-C₆)-alkoxy, (C₃-C₈)-Cycloalkenyl-(C₁-C₆)-halogenalkoxy, (C₃-C₈)-Cycloalkenylcarbonyl, (C₃-C₈)-Cycloalkenyloxycarbonyl, (C₃-C₈)-Cycloalkenyl-(C₁-C₆)-alkylcarbonyl, (C₃-C₈)-Cycloalkenyl-(C₁-C₆)-halogenalkylcarbonyl, (C₃-C₈)-Cycloalkenyl-(C₁-C₆)-alkoxycarbonyl, (C₃-C₈)-Cycloalkenyl-(C₁-C₆)-haloalkoxycarbonyl, (C₃-C₈)-Cycloalkenylcarbonyloxy, (C₃-C₈)-Cycloalkenyloxycarbonyloxy, (C₃-C₈)-Cycloalkenyl-(C₁-C₆)-alkylcarbonyloxy, (C₃-C₈)-Cycloalkenyl-(C₁-C₆)-halogenalkylcarbonyloxy, (C₃-C₈)-Cycloalkenyl-(C₁-C₆)-alkoxycarbonyl-oxy, (C₃-C₈)-Cycloalkenyl-(C₁-C₆)-haloalkoxycarbonyloxy;
   - Hydroxy-(C₁-C₆)-alkyl, Hydroxy-(C₁-C₆)-alkoxy Cyano-(C₁-C₆)-alkoxy, Cyano-(C₁-C₆)-alkyl_{;}
   - (C₁-C₆)-Alkylsulfonyl, (C₁-C₆)-Alkylthio, (C₁-C₆)-Alkylsulfinyl, (C₁-C₆)-Haloalkylsulfonyl, (C₁-C₆)-Halogenalkylthio, (C₁-C₆)-Halogenalkylsulfinyl, (C₁-C₆)-Alkylsulfonyl-(C₁-C₆)-alkyl, (C₁-C₆)-Alkylthi₀-(C₁-C₆)-alkyl, (C₁-C₆)-Alkylsulfinyl-(C₁-C₆)-alkyl, (C₁-C₆)-Haloalkylsulfonyl-(C₁-C₆)-alkyl, (C₁-C₆)-Haloal kylthio-(C₁-C₆)-alkyl, (C₁-C₆)-Haloalkylsulfinyl-(C₁-C₆)-alkyl, (C₁-C₆)-Alkylsulfonyl-(C₁-C₆)-haloalkyl, (C₁-C₆)-Alkylthio-(C₁-C₆)-haloalkyl, (C₁-C₆)-Alkylsulfinyl-(C₁-C₆)-haloalkyl, (C₁-C₆)-Haloalkylsulfonyl-(C₁-C₆)-haloalkyl, (C₁-C₆)-Haloalkylthio-(C₁-C₆)-haloalkyl, (C₁-C₆)-Haloalkylsulfinyl-(C₁-C₆)-haloalkyl, (C₁-C₆)-Alkylsulfonyloxy, (C₁-C₆)-Halogenalkylsulfonyloxy, (C₁-C₆)-Alkylthiocarbonyl, (C₁-C₆)-Haloalkylthiocarbonyl, (C₁-C₆)-Alkylthiocarbonyloxy, (C₁-C₆)-Haloalkylthiocarbonyloxy, (C₁-C₆)-Alkylthio-(C₁-C₆)-alkyl, (C₁-C₆)-Alkylthio-(C₁-C₆)-alkoxy, (C₁-C₆)-Alkylthio-(C₁-C₆)-alkylcarbonyl, (C₁-C₆)-Alkylthio-(C₁-C₆)-alkylcarbonyloxy; (C₄-C₁₄)-Arylsulfonyl, (C₆-C₁₄)-Arylthio, (C₆-C₁₄)-Arylsulfinyl, (C₃-C₈)-Cycloalkylthio, (C₃-C₈)-Alkenylthio, (C₃-C₈)-Cycloalkenylthio, (C₃-C₆)-Alkinylthio; und
R³ ausgewählt ist aus der Gruppe, bestehend aus
   - Wasserstoff, Cyano, C(O)OH, C(O)NH₂;
   - (C₁-C₆)-Alkyl und (C₁-C₆)-Haloalkyl,
   - (C₁-C₆)-Alkoxycarbonyl, und
R⁴ und R⁵, jeweils unabhängig voneinander, ausgewählt sind aus der Gruppe, bestehend aus Wasserstoff, (C₁-C₆)-Alkyl, (C₁-C₆)-Haloalkyl, Hydroxy, (C₁-C₆)-Alkoxy und (C₁-C₆)-Halogenalkoxy, oder die Reste R⁴ und R⁵ zusammen mit dem Kohlenstoffatom, an welchem sie gebunden sind, einen drei- bis siebengliedrigen Ring bilden; und
R⁶ und R⁷, jeweils unabhängig voneinander, ausgewählt sind aus der Gruppe, bestehend aus Wasserstoff, (C₁-C₆)-Alkyl, (C₁-C₆)-Haloalkyl, (C₁-C₆)-Alkoxy, (C₁-C₆)-Halogenalkoxy, (C₆-C₁₄)-Aryl, (C₆-C₁₄)-Aryloxy, (C₆-C₁₄)-Arylcarbonyl und (C₆-C₁₄)-Aryloxycarbonyl, oder die Reste R⁶ und R⁷ zusammen eine (C₁-C₇)-Alkylengruppe bilden, die ein oder mehrere Sauerstoff- und/oder Schwefelatome enthalten können, wobei die (C₁-C₇)-Alkylengruppe durch Halogen einfach oder mehrfach substituiert sein kann und die jeweilgen Halogensubstituenten gleich oder verschieden sein können, und
n die Laufzahl 0, 1 oder 2 ist und
R⁸, R⁹, R¹⁰ und R¹¹, jeweils unabhängig voneinander, ausgewählt sind aus der Gruppe, bestehend aus Wasserstoff, Halogen, Cyano, C(O)OH, C(O)NH₂, (C₁-C₆)-Alkyl, (C₁-C₆)-Alkylcarbonyl, (C₁-C₆)-Alkyloxycarbonyl, (C₁-C₆)-Alkylaminocarbonyl, (C₁-C₆)-di-Alkylaminocarbonyl, (C₁-C₆)-Haloalkyl, (C₁-C₆)-Alkoxy, (C₁-C₆)-Halogenalkoxy, (C₂-C₆)-Alkenyl, (C₂-C₆)-Alkinyl, (C₂-C₆)-Halogenalkinyl, (C₂-C₆)-Alkinylcarbonyl, (C₂-C₆)-Halo-alkinylcarbonyl, (C₂-C₆)-Alkinyloxy, (C₂-C₆)-Haloalkinyloxy, (C₂-C₆)-Alkinyloxycarbonyl und (C₂-C₆)-Halogenalkinyloxycarbonyl und Nitro;
X für eine Bindung, CH₂, O, S, Carbonyl, NH, CR¹²R¹³ und NR¹⁴ oder CH₂O, CH₂S, wobei bei den beiden letztgenannten Gruppen das Kohlenstoffatom an den aromatischen Teil und das Heteroatom O oder S an den teilhydrierten Teil des Amins gebunden ist, steht; und
R¹² und R¹³, jeweils unabhängig voneinander, ausgewählt sind aus der Gruppe, bestehend aus Wasserstoff, (C₁-C₆)-Alkyl und (C₁-C₆)-Haloalkyl, und
R¹⁴ ausgewählt ist aus der Gruppe, bestehend aus Wasserstoff, (C₁-C₆)-Alkyl, (C₁-C₆)-Haloalkyl.

Die Verbindungen der Formel (I) zeichnen sich neben einem guten Wirksamkeitsprofil und einer guten Kulturpflanzenverträglichkeit durch ihre kostengünstige Herstellung aus, da die erfindungsgemäßen Substanzen aus preiswerten und gut zugänglichen Vorstufen durch kostengünstigere Prozesse hergestellt werden können und daher auf die Verwendung teurer und schwer zugänglicher Zwischenprodukte verzichtet werden kann.

Im Folgenden werden, jeweils für die einzelnen Substituenten, bevorzugte, besonders bevorzugte und ganz besonders bevorzugte Bedeutungen beschrieben.

Eine erste Ausführungsform der vorliegenden Erfindung umfasst Verbindungen der allgemeinen Formel (I), in welchen
- R¹: bevorzugt ausgewählt ist aus der Gruppe, bestehend aus Wasserstoff, Halogen, Cyano, NO₂, (C₁-C₆)-Alkyl, (C₁-C₆)-Haloalkyl, (C₁-C₆)-Alkoxy, (C₁-C₆)-Haloalkoxy, (C1-C6)-Alkylthio, (C₁-C₆)-Alkylsulfoxid, (C₁-C₆)-Alkylsulfonyl, (C₁-C₆)-Haloalkylsulfonyl, (C₁-C₆)-Alkoxy-carbonyl, Amino-Carbonyl, Mono-((C₁-C₆)-alkyl)-amino-carbonyl, Di-((C₁-C₆)-alkyl)-amino-carbonyl, (C₆-C₁₄)-Aryl, (C₆-C₁₄)-Aryl-Haloalkyl, (C₁-C₆)Aryloxy, (C₆-C₁₄)Haloaryl-(C₁-C₆)alkyl, (C₂-C₆)-Alkinyl-(C₁-C₆)-alkoxy, (C₂-C₆)-Alkinyl-(C₁-C₆)alkyl-(C₁-C₆)-alkoxy und (C₁-C₆)Alkyl-(C₂-C₆)-Alkinyl-(C₁-C₆)-Alkoxy;
- R¹: besonders bevorzugt ausgewählt ist aus der Gruppe, bestehend aus, Cyano (CN), NO₂, (C₁-C₃)-Alkyl, (C₁-C₆)-Haloalkyl, (C₁-C₆)-Alkoxy, (C₁-C₆)-Haloalkoxy, (C₁-C₆)-Alkylthio, (C₁-C₆)-Alkylsulfoxid, (C₁-C₆)-Alkylsulfonyl, (C₁-C₆)-Haloalkylsulfonyl, (C₁-C₆)-Alkoxy-carbonyl, Amino-Carbonyl, Mono-((C₁-C₆)-alkyl)-amino-carbonyl, Di-((C₁-C₆)-alkyl)-amino-carbonyl, (C₆-C₁₄)-Aryl, (C₆-C₁₄)-Aryl-Haloalkyl, (C₁-C₆)-Aryloxy, (C₆-C₁₄)-Haloaryl-(C₁-C₆)alkyl, (C₂-C₆)-Alkinyl-(C₁-C₆)-alkoxy, (C₂-C₆)-Alkinyl-(C₁-C₆)alkyl-(C₁-C₆)-alkoxy und (C₁-C₆)Alkyl-(C₂-C₆)-Alkinyl-(C₁-C₆)-Alkoxy;
- R¹: ganz besonders bevorzugt ausgewählt ist aus der Gruppe, bestehend aus CN, NO₂, CH₃, CH₂CH₃, CH(CH₃)₂, OCH₃, CF₃ , CF₂Cl, CF₂H, CFHCH₃, CF₂CF₃, OCH₃, OCH₂CH₃, OCH₂CF₃, SCH₃, SOCH₃, SO₂CH₃, SO₂CH₂CH₃, SO₂CF₃, COOCH₃, COOCH₂CH₃, CONH₂, CONHCH₃, CON(CH₃)₂, C₆H₅-, CHF-C₆H₅, OC₆H₅, CH₂C₆H₄-4-Cl, OCH₂C≡CH, OCH(CH₃)C≡CH, OCH₂C≡CCH₃ ist; und in welchen
- R¹: am meisten bevorzugt SO₂CH₃ ist.

Eine zweite Ausführungsform der vorliegenden Erfindung umfasst Verbindungen der allgemeinen Formel (I), in welchen
R^{2a} und R^{2b}, jeweils unabhängig voneinander, bevorzugt ausgewählt sind aus der Gruppe, bestehend aus Wasserstoff, CN, Hydroxy, (C₁-C₆)-Alkyl, (C₁-C₆)-Haloalkyl, (C₁-C₆)-Cy_{C}loalkyl,(C₁-C₆)-Alkoxy, Aminocarbonyl, Hydroxycarbonyl, (C₁-C₆)-Alkoxycarbonyl, (C₁-C₆)-Alkylsulfonyl, Mono-((C₁-C₆)-alkyl)-amino-carbonyl, Di-((C₁-C₆)-alkyl)-amino-carbonyl, Mono-((C₆-C₁₄)-Aryl)-amino-carbonyl, Mono-((C₂-C₆)-Alkylen)-amino-carbonyl, Mono-((C₆-C₁₄)-Aryl-(C₁-C₆)-Alkyl)-amino-carbonyl, (C₁-C₆)-Alkoxy-(C₁-C₆)-alkoxy-carbonyl und Di-((C₁-C₆)-alkyl)-amino-(C₁-C₆)-alkoxycarbonyl;
R^{2a} und R^{2b}, jeweils unabhängig voneinander, besonders bevorzugt ausgewählt sind aus der Gruppe, bestehend aus Wasserstoff, CN, Hydroxy (OH), (C₁-C₆)-Alkyl, (C₁-C₆)-Haloalkyl, (C₁-C₆)-Cy_{C}loalkyl, (C₁-C₆)-Alkoxy, Aminocarbonyl, Hydroxycarbonyl COOH, (C₁-C₆)-Alkoxycarbonyl, (C₁-C₆)-Alkylsulfonyl, Mono-((C₁-C₆)-alkyl)-amino-carbonyl, Di-((C₁-C₆)-alkyl)-amino-carbonyl, Mono-((C₆-C₁₄)-Aryl)-amino-carbonyl, Mono-((C₂-C₆)-Alkylen)-amino-carbonyl, Mono-((C₆-C₁₄)-Aryl-(C₁-C₆)-Alkyl)-amino-carbonyl, (C₁-C₆)-Alkoxy-(C₁-C₆)-alkoxy-carbonyl und Di-((C₁-C₆)-alkyl)-amino-(C₁-C₆)-alkoxycarbonyl; und in welchen
R^{2a} und R^{2b}, jeweils unabhängig voneinander, ganz besonders bevorzugt ausgewählt sind aus der Gruppe, bestehend aus
Wasserstoff, CN, Hydroxy (OH), CH₃, CH₂CH₃, CH(CH₃)₂, C(CH₃)₃, CF₃, CF₂H, CF₂Cl, Cyclopropyl, OCH₃, CONH₂, COOH, COOCH₃, COOCH₂CH₃, SO₂CH₃, CONHCH₃, CONHcyprop, CON(CH₃)₂, CON(CH₂CH₃)₂, CONHC₆H₅, CONHCH₂CH=CH₂, CONHCH₂C₆H₅, COOCH₂CH₂OCH₃, COOCH₂CH₂N(CH₃)₂
R^{2a} und R^{2b}, jeweils unabhängig voneinander, am meisten bevorzugt ausgewählt sind aus der Gruppe, bestehend aus Verbindungen der allgemeinen Formel (I), in welchen
R^{2a} ausgewählt ist aus der Gruppe, bestehend aus Wasserstoff, CH₃, CH₂CH₃ und CF₃, und
R^{2b} ausgewählt ist aus der Gruppe, bestehend aus Wasserstoff und CH₃.

Eine dritte Ausführungsform der vorliegenden Erfindung umfasst Verbindungen der allgemeinen Formel (I), in welchen
R³ bevorzugt ausgewählt ist aus der Gruppe, bestehend aus Wasserstoff, (C₁-C₆)-Alkyl, (C₁-C₆)-Alkoxy,(C₁-C₆)-Alkoxy-(C₁-C₆)-Alkyl, (C₁-C₆)-Alkoxy-carbonyl, Aminocarbonyl, (C₆-C₁₄)-Aryl,
R³ besonders bevorzugt ausgewählt ist aus der Gruppe, bestehend aus Wasserstoff, CH₃, CH₂CH₂OCH₃, COOCH₃, CONH₂, und C₆H₅, und
R³ ganz besonders bevorzugt ausgewählt ist aus der Gruppe, bestehend aus Wasserstoff, CH₃, CH₂CH₂OCH₃; und in welchen
R³ am meisten bevorzugt Wasserstoff ist.

Eine vierte Ausführungsform der vorliegenden Erfindung umfasst Verbindungen der allgemeinen Formel (I), in welchen
- R⁴ und R⁵: jeweils unabhängig voneinander, bevorzugt ausgewählt sind aus der Gruppe, bestehend aus Wasserstoff, Cyano, Hydroxy, (C₁-C₆)-Alkyl, (C₁-C₆)-Alkylphenyl und (C₁-C₆)-Alkoxy,
- R⁴ und R⁵: jeweils unabhängig voneinander, besonders bevorzugt ausgewählt sind aus der Gruppe, bestehend aus Wasserstoff, Cyano, Hydroxy, (C₁-C₆)-Alkyl und (C₁-C₆)-Alkoxy,
- R⁴ und R⁵: jeweils unabhängig voneinander, ganz besonders bevorzugt ausgewählt sind aus der Gruppe, bestehend aus Wasserstoff, Cyano, Hydroxy, CH₃, CH₂CH₃, CH₂C₆H₅ und OCH₃; und in welchen
- R⁴ und R⁵: jeweils unabhängig voneinander, am meisten bevorzugt Methyl oder Wasserstoff sind.

In dieser vierten Ausführungsform ist somit speziell bevorzugt, wenn mindestens einer der Reste R⁴, bzw. R⁵ Wasserstoff ist. Weiter bevorzugt ist, wenn mindestens einer der Reste R⁴, bzw. R⁵ Wasserstoff und der andere Rest R⁴, bzw. R⁵ ungleich Wasserstoff, insbesondere (C₁-C₆)-Alkyl, bevorzugt Methyl (CH₃), ist.

Eine fünfte Ausführungsform der vorliegenden Erfindung umfasst Verbindungen der allgemeinen Formel (I), in welchen
- R⁴ und R⁵: bevorzugt für eine (C₁-C₇)-Alkylengruppe stehen und zusammen mit dem Kohlenstoffatom, an welches sie gebunden sind, einen drei- bis siebengliedrigen Ring bilden;
- R⁴ und R⁵: besonders bevorzugt für eine (C₁-C₃)-Alkylengruppe stehen und zusammen mit dem Kohlenstoffatom, an welches sie gebunden sind, einen drei oder viergliedrigen Ring bilden; und in welchen
- R⁴ und R⁵: ganz besonders bevorzugt für die Alkylengruppe (-CH₂-CH₂-) stehen und zusammen mit dem Kohlenstoffatom, an welches sie gebunden sind, einen dreigliedrigen Ring bilden.

Eine sechste Ausführungsform der vorliegenden Erfindung umfasst Verbindungen der allgemeinen Formel (I), in welchen
- R⁶ und R⁷: unabhängig voneinander, bevorzugt ausgewählt sind aus der Gruppe, bestehend aus Wasserstoff, (C₁-C₆)-Alkyl und (C₆-C₁₄)-Aryl,
- R⁶ und Ru⁷: unabhängig voneinander, besonders bevorzugt ausgewählt sind aus der Gruppe, bestehend aus Wasserstoff, Methyl und Phenyl; und in welchen
- R⁶ und R⁷: ganz besonders bevorzugt Wasserstoff sind.

Eine siebte Ausführungsform der vorliegenden Erfindung umfasst Verbindungen der allgemeinen Formel (I), in welchen
R⁸ bevorzugt ausgewählt ist aus der Gruppe, bestehend aus Wasserstoff, Halogen, (C₁-C₆)-Alkyl, (C₁-C₆)Alkoxy, und Di-((C₁-C₆)alkylamino;
R⁸ besonders bevorzugt ausgewählt ist aus der Gruppe, bestehend aus Wasserstoff, Halogen, (C₁-C₆)-Alkyl, (C₁-C₆)Alkoxy, und Di-((C₁-C₆)alkylamino; und
R⁸ ganz besonders bevorzugt ausgewählt ist aus der Gruppe, bestehend aus Wasserstoff, Fluor, Chlor, CH₃, OCH₃ und N(CH₃)₂; und in welchen
R⁸ am meisten bevorzugt Wasserstoff ist.

Eine achte Ausführungsform der vorliegenden Erfindung umfasst Verbindungen der allgemeinen Formel (I), in welchen
- R⁹: bevorzugt ausgewählt ist aus der Gruppe, bestehend aus Wasserstoff, Halogen, (C₁-C₆)-Alkyl, (C₁-C₆)-Alkoxy,
- R⁹: besonders bevorzugt ausgewählt ist aus der Gruppe, bestehend aus Wasserstoff, Chlor, Fluor, (C₁-C₆)-Alkyl und (C₁-C₆)-Alkoxy, und
- R⁹: ganz besonders bevorzugt ausgewählt ist aus der Gruppe, bestehend aus Wasserstoff, Fluor, Chlor, Methyl und Methoxy; und in welchen
- R⁹: am meisten bevorzugt Wasserstoff ist.

Eine neunte Ausführungsform der vorliegenden Erfindung umfasst Verbindungen der allgemeinen Formel (I), in welchen
- R¹⁰: bevorzugt ausgewählt ist aus der Gruppe, bestehend aus Wasserstoff, Halogen, Cyano, Aminocarbonyl, Hydroxycarbonyl, (C₁-C₆)-Alkyl, (C₁-C₆)-Alkoxy, (C₂-C₆)-Alkenyl, (C₂-C₆)-Alkinyl und (C₁-C₆)-Alkyl-(C₂-C₆)-alkinyl,
- R¹⁰: besonders bevorzugt ausgewählt ist aus der Gruppe, bestehend aus Wasserstoff, Fluor, Chlor, Brom, Cyano, CONH₂, COOH, Methyl (CH₃), Ethyl (CH₂CH₃), Methoxy (OCH₃), CH=CH₂, C=CH und C=CCH₃; und in welchen
- R¹⁰: ganz besonders bevorzugt Wasserstoff oder Methyl ist.

Eine zehnte Ausführungsform der vorliegenden Erfindung umfasst Verbindungen der allgemeinen Formel (I), in welchen
- R¹¹: bevorzugt ausgewählt ist aus der Gruppe, bestehend aus Wasserstoff und (C₁-C₆)-Alkyl;
- R¹¹: besonders bevorzugt ausgewählt ist aus der Gruppe, bestehend aus Wasserstoff und Methyl; und
- R¹¹: ganz besonders bevorzugt Wasserstoff ist.

Eine elfte Ausführungsform der vorliegenden Erfindung umfasst Verbindungen der allgemeinen Formel (I), in welchen
- X: bevorzugt ausgewählt ist aus der Gruppe, bestehend aus O, S, Carbonyl, CH₂, NH, CH(C₁-C₆)Alkyl, Di-(C₁-C₆)-alkyl-alkylen, O-(C₁-C₇)Alkylen, S-(C₁-C₇)Alkylen, (C₁-C₆)Alkylamino, oder in welchen X für eine chemische Bindung steht;
- X: besonders bevorzugt ausgewählt ist aus der Gruppe, bestehend aus O, S, Carbonyl, CH₂, NH, CHCH₃, NCH₃, C(CH₃)₂, OCH₂, SCH₂, oder in welchen X für eine chemische Bindung steht; und
- X: ganz besonders bevorzugt ausgewählt ist aus der Gruppe, bestehend aus O und S, oder in welchen X für eine chemische Bindung steht.

Im Rahmen der vorliegenden Erfindung ist es möglich, die einzelnen bevorzugten, besonders bevorzugten und ganz besonders bevorzugten Bedeutungen für die Substituenten R¹ bis R¹¹ und X beliebig miteinander zu kombinieren. Das heißt, dass Verbindungen der allgemeinen Formel (I) von der vorliegenden Erfindung umfasst sind, in welchen beispielsweise der Substituent R¹ eine bevorzugte Bedeutung aufweist und die Substituenten R² bis R¹⁴ die allgemeine Bedeutung aufweisen oder aber der Substituent R² eine bevorzugte Bedeutung aufweist, der Substituent R³ eine besonders bevorzugte, bzw. eine ganz besonders bevorzugte, Bedeutung aufweist und die übrigen Substituenten eine allgemeine Bedeutung aufweisen.

Drei dieser Kombinationen der oben für die Substituenten R¹ bis R¹⁰ und X gegebenen Definitionen werden nachfolgend, als weitere Ausführungsformen, beispielhaft erläutert und offenbart:
- Kombination der oben für die Substituenten R¹ bis R¹¹ und X jeweils als besonders bevorzugt bezeichneten Definitionen (zwölfte Ausführungsform),
- Kombination der oben für die Substituenten R¹ bis R¹⁰ und X jeweils als ganz besonders bevorzugt bezeichneten Definitionen (dreizehnte Ausführungsform), und
- Kombination der oben für den Substituenten R¹ als ganz besonders bevorzugt bezeichneten Definition mit den oben für die Substituenten R² bis R¹⁰ und X jeweils als besonders bevorzugt bezeichneten Definitionen (vierzehnte Ausführungsform)

Eine zwölfte Ausführungsform der vorliegenden Erfindung umfasst Verbindungen der allgemeinen Formel (I), in welchen
R¹ ausgewählt ist aus der Gruppe, bestehend aus, Cyano (CN), NO₂, (C₁-C₃)-Alkyl, (C₁-C₆)-Haloalkyl, (C₁-C₆)-Alkoxy, (C₁-C₆)-Haloalkoxy, (C₁-C₆)-Alkylthio, (C₁-C₆)-Alkylsulfoxid, (C₁-C₆)-Alkylsulfonyl, (C₁-C₆)-Haloalkylsulfonyl, (C₁-C₆)-Alkoxy-carbonyl, Amino-Carbonyl, Mono-((C₁-C₆)-alkyl)-amino-carbonyl, Di-((C₁-C₆)-alkyl)-amino-carbonyl, (C₆-C₁₄)-Aryl, (C₆-C₁₄)-Aryl-Haloalkyl, (C₁-C₆)-Aryloxy, (C₆-C₁₄)-Haloaryl-(C₁-C₆)alkyl, (C₂-C₆)-Alkinyl-(C₁-C₆)-alkoxy, (C₂-C₆)-Alkinyl-(C₁-C₆)alkyl-(C₁-C₆)-alkoxy und (C₁-C₆)Alkyl-(C₂-C₆)-Alkinyl-(C₁-C₆)-Alkoxy;
R^{2a} und R^{2b}, jeweils unabhängig voneinander, ausgewählt sind aus der Gruppe, bestehend aus Wasserstoff, CN, Hydroxy (OH), (C₁-C₆)-Alkyl, (C₁-C₆)-Haloalkyl, (C₁-C₆)-Cycloalkyl, (C₁-C₆)-Alkoxy, Aminocarbonyl, Hydroxycarbonyl COOH, (Ci-C₆)-Alkoxycarbonyl, (C₁-C₆)-Alkylsulfonyl, Mono-((C₁-C₆)-alkyl)-amino-carbonyl, Di-((C₁-C₆)-alkyl)-amino-carbonyl, Mono-((C₆-C₁₄)-Aryl)-amino-carbonyl, Mono-((C₂-C₆)-Alkylen)-amino-carbonyl, Mono-((C₆-C₁₄)-Aryl-(C₁-C₆)-Alkyl)-amino-carbonyl, (C₁-C₆)-Alkoxy-(C₁-C₆)-alkoxy-carbonyl und Di-((C₁-C₆)-alkyl)-amino-(C₁-C₆)-alkoxycarbonyl;
R³ ausgewählt ist aus der Gruppe, bestehend aus Wasserstoff, CH₃, CH₂CH₂OCH₃, COOCH₃, CONH₂, und C₆H₅;
R⁴ und R⁵ jeweils unabhängig voneinander, ausgewählt sind aus der Gruppe, bestehend aus Wasserstoff, Cyano, Hydroxy, (C₁-C₆)-Alkyl und (C₁-C₆)-Alkoxy; oder alternativ
R⁴ und R⁵ für eine (C₁-C₃)-Alkylengruppe stehen und zusammen mit dem Kohlenstoffatom, an welches sie gebunden sind, einen drei oder viergliedrigen Ring bilden;
R⁶ und R⁷ unabhängig voneinander, ausgewählt sind aus der Gruppe, bestehend aus Wasserstoff, Methyl und Phenyl;
R⁸ ausgewählt ist aus der Gruppe, bestehend aus Wasserstoff, Halogen, (C₁-C₆)-Alkyl, (C₁-C₆)Alkoxy, und Di-((C₁-C₆)alkylamino;
R⁹ ausgewählt ist aus der Gruppe, bestehend aus Wasserstoff, Chlor, Fluor, (C₁-C₆)-Alkyl und (C₁-C₆)-Alkoxy,
R¹⁰ ausgewählt ist aus der Gruppe, bestehend aus Wasserstoff, Fluor, Chlor, Brom, Cyano, CONH₂, COOH, Methyl (CH₃), Ethyl (CH₂CH₃), Methoxy (OCH₃), CH=CH₂, C=CH und C=CCH₃;
R¹¹ ausgewählt ist aus der Gruppe, bestehend aus Wasserstoff und Methyl; und in welchen
X ausgewählt ist aus der Gruppe, bestehend aus O, S, Carbonyl, CH₂, NH, CHCH₃, NCH₃, C(CH₃)2, OCH₂, SCH₂, oder in welchen X für eine chemische Bindung steht.

Eine dreizehnte Ausführungsform der vorliegenden Erfindung umfasst Verbindungen der allgemeinen Formel (I), in welchen
R¹ ausgewählt ist aus der Gruppe, bestehend aus CN, NO₂, CH₃, CH₂CH₃, CH(CH₃)₂, OCH₃, CF₃ , CF₂Cl, CF₂H, CFHCH₃, CF₂CF₃, OCH₃, OCH₂CH₃, OCH₂CF₃, SCH₃, SOCH₃, SO₂CH₃, SO₂CH₂CH₃, SO₂CF₃, COOCH₃, COOCH₂CH₃, CONH₂, CONHCH₃, CON(CH₃)₂, C₆H₅₋, CHF-C₆H₅, OC₆H₅, CH₂C₆H₄-4-Cl, OCH₂C≡CH, OCH(CH₃)C≡CH, OCH₂C≡CCH₃ ist;
R^{2a} und R^{2b}, jeweils unabhängig voneinander, ausgewählt sind aus der Gruppe, bestehend aus
Wasserstoff, CN, Hydroxy (OH), CH₃, CH₂CH₃, CH(CH₃)₂, C(CH₃)₃, CF₃, CF₂H, CF₂Cl, Cyclopropyl, OCH₃, CONH₂, COOH, COOCH₃, COOCH₂CH₃, SO₂CH₃, CONHCH₃, CONHcyprop, CON(CH₃)₂, CON(CH₂CH₃)₂, CONHC₆H₅, CONHCH₂CH=CH₂, CONHCH₂C₆H₅, COOCH₂CH₂≡CH₃, COOCH₂CH₂N(CH₃)₂;
R³ ausgewählt ist aus der Gruppe, bestehend aus
Wasserstoff, CH₃, CH₂CH₂OCH₃; und in welchen
R⁴ und R⁵ jeweils unabhängig voneinander, ausgewählt sind aus der Gruppe, bestehend aus Wasserstoff, Cyano, Hydroxy, CH₃, CH₂CH₃, CH₂C₆H₅ und OCH₃; oder alternativ
R⁴ und R⁵ für die Alkylengruppe (-CH₂-CH₂-) stehen und zusammen mit dem Kohlenstoffatom, an welches sie gebunden sind, einen dreigliedrigen Ring bilden;
R⁶ und R⁷ unabhängig voneinander, ausgewählt sind aus der Gruppe, bestehend aus Wasserstoff, Methyl und Phenyl;
R⁸ ausgewählt ist aus der Gruppe, bestehend aus Wasserstoff, Fluor, Chlor, CH₃, OCH₃ und N(CH₃)₂;
R⁹ ausgewählt ist aus der Gruppe, bestehend aus Wasserstoff, Fluor, Chlor, Methyl und Methoxy;
R¹⁰ Wasserstoff oder Methyl ist;
R¹¹ Wasserstoff ist; und in welchen
X ausgewählt ist aus der Gruppe, bestehend aus O und S, oder in welchen X für eine chemische Bindung steht.

Eine vierzehnte Ausführungsform der vorliegenden Erfindung umfasst Verbindungen der allgemeinen Formel (I), in welchen
R¹ ausgewählt ist aus der Gruppe, bestehend aus CN, NO₂, CH₃, CH₂CH₃, CH(CH₃)₂, OCH₃, CF₃ , CF₂Cl, CF₂H, CFHCH₃, CF₂CF₃, OCH₃, OCH₂CH₃, OCH₂CF₃, SCH₃, SOCH₃, SO₂CH₃, SO₂CH₂CH₃, SO₂CF₃, COOCH₃, COOCH₂CH₃, CONH₂, CONHCH₃, CON(CH₃)₂, C₆H₅-, CHF-C₆H₅, OC₆H₅, CH₂C₆H₄-4-Cl, OCH₂C≡CH, OCH(CH₃)C≡CH, OCH₂C≡CCH₃ ist;
R^{2a} und R^{2b}, jeweils unabhängig voneinander, ausgewählt sind aus der Gruppe, bestehend aus Wasserstoff, CN, Hydroxy (OH), (C₁-C₆)-Alkyl, (C₁-C₆)-Haloalkyl, (C₁-C₆)-Cycloalkyl, (C₁-C₆)-Alkoxy, Aminocarbonyl, Hydroxycarbonyl COOH, (C₁-C₆)-Alkoxycarbonyl, (C₁-C₆)-Alkylsulfonyl, Mono-((C₁-C₆)-alkyl)-aminocarbonyl, Di-((C₁-C₆)-alkyl)-amino-carbonyl, Mono-((C₆-C₁₄)-Aryl)-aminocarbonyl, Mono-((C₂-C₆)-Alkylen)-amino-carbonyl, Mono-((C₆-C₁₄)-Aryl-(C₁-C₆)-Alkyl)-amino-carbonyl, (C₁-C₆)-Alkoxy-(C₁-C₆)-alkoxy-carbonyl und Di-((C₁-C₆)-alkyl)-amino-(C₁-C₆)-alkoxycarbonyl;
R³ ist aus der Gruppe, bestehend aus Wasserstoff, CH₃, CH₂CH₂OCH₃, COOCH₃, CONH₂, und C₆H₅;
R⁴ und R⁵ jeweils unabhängig voneinander, ausgewählt sind aus der Gruppe, bestehend aus Wasserstoff, Cyano, Hydroxy, (C₁-C₆)-Alkyl und (C₁-C₆)Alkoxy; oder alternativ
R⁴ und R⁵ für eine (C₁-C₃)-Alkylengruppe stehen und zusammen mit dem Kohlenstoffatom, an welches sie gebunden sind, einen drei oder viergliedrigen Ring bilden;
R⁶ und R⁷ unabhängig voneinander, ausgewählt sind aus der Gruppe, bestehend aus Wasserstoff, Methyl und Phenyl;
R⁸ ausgewählt ist aus der Gruppe, bestehend aus Wasserstoff, Halogen, (C₁-C₆)-Alkyl, (C₁-C₆)Alkoxy, und Di-((C₁-C₆)alkylamino;
R⁹ ausgewählt ist aus der Gruppe, bestehend aus Wasserstoff, Chlor, Fluor, (C₁-C₆)-Alkyl und (C₁-C₆)-Alkoxy,
R¹⁰ ausgewählt ist aus der Gruppe, bestehend aus Wasserstoff, Fluor, Chlor, Brom, Cyano, CONH₂, COOH, Methyl (CH₃), Ethyl (CH₂CH₃), Methoxy (OCH₃), CH=CH₂, C=CH und C≡CCH₃;
R¹¹ ausgewählt ist aus der Gruppe, bestehend aus Wasserstoff und Methyl; und in welchen
X ausgewählt ist aus der Gruppe, bestehend aus O, S, Carbonyl, CH₂, NH, CHCH₃, NCH₃, C(CH₃)₂, OCH₂, SCH₂, oder in welchen X für eine chemische Bindung steht.

Im Rahmen der vorliegenden Erfindung sind von der Verbindung der allgemeinen Formel (I) auch Verbindungen umfasst, die durch a) Protonierung, b) Alkylierung oder c) Oxidation an einem Stickstoffatom quaterniert sind. Insbesondere sind diesbezüglich die entsprechenden N-Oxide zu nennnen.

Die Verbindungen der Formel (I) können Salze bilden. Salzbildung kann durch Einwirkung einer Base auf solche Verbindungen der Formel (I) erfolgen, die ein acides Wasserstoffatom tragen, z.B. im Falle dass R¹ eine COOH-Gruppe oder eine Sulfonamid-Gruppe -NHSO₂- enthält. Geeignete Basen sind beispielsweise organische Amine, wie Trialkylamine, Morpholin, Piperidin oder Pyridin sowie Ammonium-, Alkali-oder Erdalkalimetallhydroxide, -carbonate und -hydrogencarbonate, insbesondere Natrium- und Kaliumhydroxid, Natrium- und Kaliumcarbonat und Natrium- und Kaliumhydrogencarbonat. Diese Salze sind Verbindungen, in denen der acide Wasserstoff durch ein für die Landwirtschaft geeignetes Kation ersetzt wird, beispielsweise Metallsalze, insbesondere Alkalimetallsalze oder Erdalkalimetallsalze, insbesondere Natrium- und Kaliumsalze, oder auch Ammoniumsalze, Salze mit organischen Aminen oder quartäre (quaternäre) Ammoniumsalze, zum Beispiel mit Kationen der Formel [NRR'R"R"']⁺, worin R bis R'" jeweils unabhängig voneinander einen organischen Rest, insbesondere Alkyl, Aryl, Aralkyl oder Alkylaryl darstellen. Infrage kommen auch Alkylsulfonium- und Alkylsulfoxoniumsalze, wie (C₁-C₄)-Trialkylsulfonium- und (C₁-C₄)-Trialkylsulfoxoniumsalze.

Die Verbindungen der Formel (I) können durch Anlagerung einer geeigneten anorganischen oder organischen Säure, wie beispielsweise Mineralsäuren, wie beispielsweise HCl, HBr, H₂SO₄, H₃PO₄ oder HNO₃, oder organische Säuren, z. B. Carbonsäuren, wie Ameisensäure, Essigsäure, Propionsäure, Oxalsäure, Milchsäure oder Salicylsäure oder Sulfonsäuren, wie zum Beispiel p-Toluolsulfonsäure, an eine basische Gruppe, wie z.B. Amino, Alkylamino, Dialkylamino, Piperidino, Morpholino oder Pyridino, Salze bilden. Diese Salze enthalten dann die konjugierte Base der Säure als Anion.

Geeignete Substituenten, die in deprotonierter Form, wie z.B. Sulfonsäuren oder Carbonsäuren, vorliegen, können innere Salze mit ihrerseits protonierbaren Gruppen, wie Aminogruppen bilden.

Im Folgenden werden die Verbindungen der Formel (I) und ihre Salze auch kurz als erfindungsgemäß verwendete oder erfindungsgemäße "Verbindungen (I)" bezeichnet.

In der allgemeinen Formel (I) und allen übrigen Formeln in der vorliegenden Erfindung können die Reste Alkyl, Alkoxy, Haloalkyl, Haloalkoxy, Alkylamino, Alkylthio, Haloalkylthio, sowie die entsprechenden ungesättigten und/oder substituierten Reste im Kohlenstoffgerüst jeweils geradkettig oder verzweigt sein. Wenn nicht speziell angegeben, sind bei diesen Resten die niederen Kohlenstoffgerüste, z. B. mit 1 bis 6 Kohlenstoffatomen, insbesondere 1 bis 4 Kohlenstoffatomen, bzw. bei ungesättigten Gruppen mit 2 bis 6 Kohlenstoffatomen, insbesondere 2 bis 4 Kohlenstoffatomen, bevorzugt. Alkylreste, auch in den zusammengesetzten Bedeutungen wie Alkoxy, Haloalkyl usw., bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl, Heptyle, wie n-Heptyl, 1-Methylhexy und 1,4-Dimethylpentyl; Alkenyl- und Alkinylreste haben die Bedeutung der den Alkylresten entsprechenden möglichen ungesättigten Reste; wobei mindestens eine Doppelbindung bzw. Dreifachbindung, vorzugsweise eine Doppelbindung bzw. Dreifachbindung enthalten ist. Alkenyl bedeutet z.B. Vinyl, Allyl, 1-Methylprop-2-en-1-yl, 2-Methyl-prop-2-en-1-yl, But-2-en-1-yl, But-3-en-1-yl, 1-Methyl-but-3-en-1-yl und 1-Methyl-but-2-en-1-yl; Alkinyl bedeutet z.B. Ethinyl, Propargyl, But-2-in-1-yl, But-3-in-1-yl und 1-Methyl-but-3-in-1-yl.

Cycloalkyl-Gruppen sind z. B. Cyclopropyl, Cyclobutyl, Cyclopentyl, Cyclohexyl, Cycloheptyl und Cyclooctyl. Die Cycloalkyl Gruppen können in bi- oder tri-cyclischer Form vorkommen.

Wenn Haloalkylgruppen und Haloalkylreste von Haloalkoxy, Haloalkylthio, Haloalkenyl, Haloalkinyl u.a. angegeben sind, sind bei diesen Resten die niederen Kohlenstoffgerüste, z. B. mit 1 bis 6 C-Atomen oder 2 bis 6, insbesondere 1 bis 4 C-Atomen oder bevorzugt 2 bis 4 C-Atomen, sowie die entsprechenden ungesättigten und/oder substituierten Reste im Kohlenstoffgerüst jeweils geradkettig oder verzweigt. Beispiele sind Difluormethyl, 2,2,2-Trifluorethyl, Trifluorallyl, 1-Chlorprop-1-yl-3-yl.

Alkylen-Gruppen sind bei diesen Resten die niederen Kohlenstoffgerüste, z. B. mit 1 bis 10 C-Atomen, insbesondere 1 bis 6 C-Atomen oder bevorzugt 2 bis 4 C-Atomen, sowie die entsprechenden ungesättigten und/oder substituierten Reste im Kohlenstoffgerüst, die jeweils geradkettig oder verzweigt sein können. Beispiele sind Methylen, Ethylen, n- und i- Propylen und n-, s-, i-, t-Butylen.

Hydroxyalkylgruppen sind bei diesen Resten die niederen Kohlenstoffgerüste, z. B. mit 1 bis 6 C-Atomen, insbesondere 1 bis 4 C-Atomen, sowie die entsprechenden ungesättigten und/oder substituierten Resten im Kohlenstoffgerüst, die jeweils geradkettig oder verzweigt sein können. Beispiele hierzu sind 1,2-Dihydroxyethyl und 3-Hydroxypropyl.

Halogen bedeutet Fluor, Chlor, Brom oder lod. Haloalkyl, -alkenyl und -alkinyl bedeuten durch Halogen, vorzugsweise durch Fluor, Chlor oder Brom, insbesondere durch Fluor und/oder Chlor, teilweise oder vollständig substituiertes Alkyl, Alkenyl bzw. Alkinyl, z.B. Monohaloalkyl (= Monohalogenalkyl), Perhaloalkyl, CF₃, CF₂Cl, CHF₂, CH₂F, CF₃CF₂, CH₂FCHCl, CCl₃, CHCl₂, CH₂CH₂Cl; Haloalkoxy ist z.B. OCF₃, OCHF₂, OCH₂F, CF₃CF₂O, OCH₂CF₃ und OCH₂CH₂Cl; entsprechendes gilt für Haloalkenyl und andere durch Halogen substituierte Reste.

Aryl bedeutet ein mono-, bi- oder polycyclisches aromatisches System, beispielsweise Phenyl oder Naphthyl, vorzugsweise Phenyl.

Vor allem aus den Gründen der höheren herbiziden Wirkung, besseren Selektivität und/oder besseren Herstellbarkeit sind erfindungsgemäße Verbindungen der allgemeinen Formel (I) oder deren agrochemischen Salze oder quartären N-Derivate von besonderem Interesse, worin einzelne Reste eine der bereits genannten oder im Folgenden genannten bevorzugten Bedeutungen haben, oder insbesondere solche, worin eine oder mehrere der bereits genannten oder im folgenden genannten bevorzugten Bedeutungen kombiniert auftreten.

Die oben angeführten allgemeinen oder in Vorzugsbereichen angeführten Restedefinitionen gelten sowohl für die Endprodukte der allgemeinen Formel (I) als auch entsprechend für die die jeweils zur Herstellung benötigten Ausgangs- und Zwischenprodukte. Diese Restedefinitionen können untereinander, als auch zwischen den angegebenen bevorzugten Bereichen vertauscht werden.

Sofern Tautomere möglich sind, sind mit der beschriebenen Form alle möglichen tautomere Strukturen umfasst. Wie nachfolgend dargestellt, sind wenn zum Beispiel für R^{2a} = Hydroxy beschrieben ist, die möglichen Keto-Tautomeren ebenfalls erfasst. 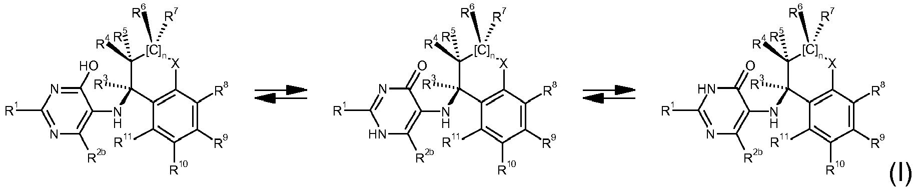 Die vorliegenden Verbindungen der allgemeinen Formel (I) weisen ein chirales Kohlenstoffatom auf, welches in der unten dargestellten Struktur durch die Kennzeichnung (*) verdeutlicht ist:

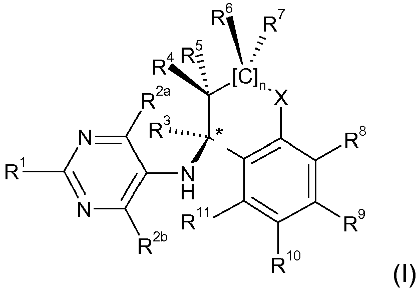

Gemäß den Regeln nach Cahn, Ingold und Prelog (CIP-Regeln) kann dieses Kohlenstoffatom sowohl eine (R)- als auch eine (S)-Konfiguration aufweisen.

Von der vorliegenden Erfindung werden Verbindungen der allgemeinen Formel (I) sowohl mit (S)- als auch mit (R)-Konfiguration erfasst, d.h., dass die vorliegende Erfindung die Verbindungen der allgemeinen Formel (I) erfasst, in welchen das betreffende Kohlenstoffatom
(1) eine (R)-Konfiguration; oder
(2) eine (S)-Konfiguration
aufweist.

Darüber hinaus werden im Rahmen der vorliegenden Erfindung auch
(3) beliebige Mischungen von Verbindungen der allgemeinen Formel (I), welche eine (R)-Konfiguation (Verbindungen der allgemeinen Formel (I-(R)) aufweisen, mit Verbindungen der allgemeinen Formel (I), welche eine (S)-Konfiguration (Verbindungen der allgemeinen Formel (I-S)) aufweisen,
   erfasst, wobei eine racemische Mischung der Verbindungen der allgemeinen Formel (I) mit (R)- und (S)-Konfiguration von der vorliegenden Erfindung ebenfalls umfasst ist. Allerdings sind im Rahmen der vorliegenden Erfindung insbesondere Verbindungen der allgemeinen Formel (I) mit (R)-Konfiguration mit einer Selektivität von 60 bis 100%, vorzugsweise 80 bis 100%, insbesondere 90 bis 100%, ganz besonders 95 bis 100%, bevorzugt, wobei die jeweilige (R)-Verbindung mit einer Enantioselektivität von jeweils mehr als 50% ee, vorzugsweise 60 bis 100% ee, insbesondere 80 bis 100% ee, ganz besonders 90 bis 100% ee, meist bevorzugt 95 bis 100% ee, bezogen auf den Gesamtgehalt an betreffender (R)-Verbindung vorliegt.

Daher betrifft die vorliegende Erfindung insbesondere Verbindungen der allgemeinen Formel (I*), in welchen die stereochemische Konfiguration an dem mit (*) gekennzeichnet Kohlenstoffatom mit einer stereochemischen Reinheit von 60 bis 100 % (R), vorzugsweise 80 bis 100 % (R), insbesondere 90 bis 100 % (R), ganz besonders 95 bis 100 % (R), vorliegt.

Unter Berücksichtigung der Regel nach Cahn, Ingold und Prelog kann es an dem mit (*) gekennzeichneten Kohlenstoffatom auch zu einer Situation kommen, in welcher aufgrund der Priorität der jeweiligen Substituenten die (S)-Konfiguration am mit (*) gekennzeichneten Kohlenstoffatom bevorzugt ist. Dieses ist beispielweise dann der Fall, wenn die Reste R⁴ und/oder R⁵ einem C₁-C₆-Alkoxyrest entsprechen.

Daher sind im Rahmen der vorliegenden Erfindung insbesondere Verbindungen der allgemeinen Formel (I) bevorzugt, die in ihrer räumlichen Anordnung den jenigen Verbindungen der allgemeinen Formel (I) mit R⁴ und R⁵ =Wasserstoff mit (R)-Konfiguration entsprechen, mit einer Selektivität von 60 bis 100 %, vorzugsweise 80 bis 100 %, insbesondere 90 bis 100 %, ganz besonders 95 bis 100 %, bevorzugt, wobei die jeweilige (R)-analoge-Verbindung mit einer Enantioselektivität von jeweils mehr als 50 % ee, vorzugsweise 60 bis 100 % ee, insbesondere 80 bis 100 % ee, ganz besonders 90 bis 100 % ee, meist bevorzugt 95 bis 100 % ee, bezogen auf den Gesamtgehalt an betreffender (R)-analogen-Verbindung, vorliegt. Daher betrifft die vorliegende Erfindung insbesondere Verbindungen der allgemeinen Formel (I), in welchen die stereochemische Konfiguration an dem mit (*) gekennzeichnet Kohlenstoffatom mit einer stereochemischen Reinheit von 60 bis 100 % (R, bzw. analog-R), vorzugsweise 80 bis 100 % (R, bzw. analog-R), insbesondere 90 bis 100 % (R, bzw. analog-R), ganz besonders 95 bis 100 % (R, bzw. analog-R), vorliegt. Insbesondere können die erfindungsgemäßen Verbindungen der allgemeinen Formel (I) noch weitere Chiralitätszentren an den mit (**) und (***) gekennzeichneten Kohlenstoffatomen aufweisen:

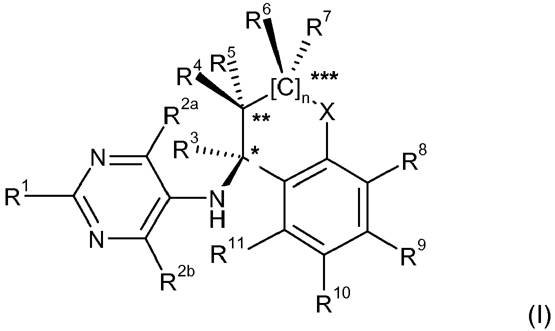

Im Rahmen der vorliegenden Erfindung sind beliebige stereochemische Konfigurationen an den mit (*), (**) und (***) gekennzeichneten Kohlenstoffatomen möglich:

| Konfiguration Kohlenstoffatom (*) | Konfiguration Kohlenstoffatom (**) | Konfiguration Kohlenstoffatom (***) |
|---|---|---|
| R | R | R |
| R | R | S |
| R | S | R |
| S | R | R |
| R | S | S |
| S | R | S |
| S | S | R |
| S | S | S |

Darüber hinaus können, je nach Wahl der jeweiligen Reste, weitere Stereoelemente in den erfindungsgemäßen Verbindungen der allgemeinen Formel (I) vorliegen.

Sind beispielsweise eine oder mehrere Alkenylgruppen vorhanden, so können Diastereomere (Z- und E-Isomere) auftreten.

Sind beispielsweise ein oder mehrere asymmetrische Kohlenstoffatome vorhanden, so können Enantiomere und Diastereomere auftreten.

Entsprechende Stereoisomere lassen sich aus den bei der Herstellung anfallenden Gemischen nach üblichen Trennmethoden, beispielsweise durch chromatographische Trennverfahren, erhalten. Ebenso können Stereoisomere durch Einsatz stereoselektiver Reaktionen unter Verwendung optisch aktiver Ausgangs- und/oder Hilfsstoffe selektiv hergestellt werden. Die Erfindung betrifft somit auch alle Stereoisomeren, die von der allgemeinen Formel (I) umfaßt werden, jedoch nicht mit ihrer spezifischen Stereoform angegeben sind und deren Gemische.

Die Kombinationsmöglichkeiten der verschiedenen Substituenten der allgemeinen Formel (I) sind so zu verstehen, dass die allgemeinen Grundsätze des Aufbaus chemischer Verbindungen zu beachten sind, d.h. die Formel (I) nicht Verbindungen umfasst, von denen der Fachmann weiß, dass sie chemisch nicht möglich sind.

Im Folgenden werden in tabellarischer Form Beispiele der Verbindungen der allgemeinen Formel (I) wiedergegeben.

In der nachfolgenden Tabelle steht:
- "StNR³" für stereochemische Anordnung am Kohlenstoffatom, an das NH und R³ gebunden sind, "StR⁴R⁵" und "StR⁶R⁷" steht analog für die Kohlenstoffatome, an die die jeweiligen Substituenten gebunden sind,
- die Bindung der Substituenten ist jeweils links,
- sofern für X zwei Bindestellen angegeben sind, bindet die linke Bindung an den Aromaten und die rechte Bindung an den hydrierten Teil des bicyclischen Amins,
- cyprop für cyclopropyl,
- ein Bindestrich "-" für eine direkte Bindung, und
- falls n=0, so enthält die Tabelle in dem entsprechenden Feld für R⁶ und R⁷ keinen Eintrag.

| Nr. | R¹ | R^{2a} | R^{2b} | R³ | St N R³ | R⁴ | R⁵ | St R⁴ R⁵ | R⁶ | R⁷ | n | St R⁶ R⁷ | R⁸ | R⁹ | R¹⁰ | R¹¹ | X |
|---|---|---|---|---|---|---|---|---|---|---|---|---|---|---|---|---|---|
| 1. | CF₃ | H | H | H | rac | H | H | | H | H | 2 | | CH₃ | H | CH₃ | H | - |
| 2. | SOCH₃ | H | H | H | rac | H | H | | H | H | 2 | | CH₃ | H | CH₃ | H | - |
| 3. | SO₂CH₃ | H | H | H | rac | H | H | | H | H | 2 | | CH₃ | H | CH₃ | H | - |
| 4. | NO₂ | H | H | H | rac | H | H | | H | H | 2 | | CH₃ | H | CH₃ | H | - |
| 5. | CN | H | H | H | rac | H | H | | H | H | 2 | | CH₃ | H | CH₃ | H | - |
| 6. | CONH₂ | H | H | H | rac | H | H | | H | H | 2 | | CH₃ | H | CH₃ | H | - |
| 7. | COOCH₃ | H | H | H | rac | H | H | | H | H | 2 | | CH₃ | H | CH₃ | H | - |
| 8. | CONH CH₃ | H | H | H | rac | H | H | | H | H | 2 | | CH₃ | H | CH₃ | H | - |
| 9. | CONHcyprop | H | H | H | rac | H | H | | H | H | 2 | | CH₃ | H | CH₃ | H | - |
| 10. | CF₂CF₃ | H | H | H | rac | H | H | | H | H | 2 | | CH₃ | H | CH₃ | H | - |
| 11. | CF₂CF₃ | H | H | H | rac | H | H | | H | H | 2 | | H | H | H | H | - |
| 12. | CF₂CF₃ | H | H | H | rac | H | H | | H | H | 1 | | H | H | H | H | O |
| 13. | CF₂CF₃ | H | H | H | rac | H | H | | H | H | 1 | | H | H | H | H | S |
| 14. | CF₂CF₃ | H | H | H | rac | H | H | | H | H | 1 | | H | H | H | H | - |
| 15. | CF₂CF₃ | H | H | H | R | CH₃ | H | S | H | H | 1 | | H | H | H | H | - |
| 16. | CF₂CF₃ | H | H | H | R | CH₃ | H | S | H | H | 1 | | H | H | CH₃ | H | - |
| 17. | CF₂CF₃ | H | H | H | rac | H | H | | H | H | 2 | | F | H | H | H | - |
| 18. | CF₂CF₃ | OCH₃ | H | H | R | H | H | | H | H | 2 | | H | H | H | H | - |
| 19. | CF₂CF₃ | OCH₃ | H | H | rac | H | H | | H | H | 2 | | CH₃ | H | CH₃ | H | - |
| 20. | CF₂CF₃ | OCH₃ | H | H | rac | H | H | | H | H | 2 | | H | H | CH₃ | H | - |
| 21. | CF₂CF₃ | H | H | H | rac | H | H | | H | H | 1 | | H | H | CH₃ | H | - |
| 22. | CF₂CF₃ | OCH₃ | H | H | R | H | H | | H | H | 1 | | H | H | CH₃ | H | - |
| 23. | CF₂CF₃ | OCH₃ | H | H | R | CH₃ | H | S | H | H | 1 | | H | H | CH₃ | H | - |
| 24. | CF₂CF₃ | OCH₃ | H | H | rac | H | H | | H | H | 2 | | H | H | F | H | - |
| 25. | CF₃ | OCH₃ | H | H | R | CH₃ | H | S | H | H | 1 | | H | H | CH₃ | H | - |
| 26. | SOCH₃ | H | H | CH₃ | rac | H | H | | H | H | 1 | | H | H | CH₃ | H | - |
| 27. | SO₂CH₃ | H | H | CH₃ | rac | H | H | | H | H | 1 | | H | H | CH₃ | H | - |
| 28. | SO₂CH₃ | H | H | CH₃ | rac | H | H | | H | H | 1 | | H | H | H | H | - |
| 29. | SO₂CH₃ | H | H | CH₃ | rac | H | H | | H | H | 2 | | H | H | H | H | - |
| 30. | SO₂CH₃ | H | H | CH₂CH₂ OCH₃ | rac | H | H | | H | H | 2 | | H | H | H | H | - |
| 31. | SO₂CH₃ | H | H | Ph | rac | H | H | | H | H | 2 | | H | H | H | H | - |
| 32. | SO₂CH₃ | H | H | COOCH₃ | rac | H | H | | H | H | 2 | | H | H | H | H | - |
| 33. | SO₂CH₃ | H | H | CONH₂ | rac | H | H | | H | H | 2 | | H | H | H | H | - |
| 34. | SO₂CH₃ | H | H | CONH₂ | rac | H | H | | H | H | 1 | | H | H | H | H | - |
| 35. | CF₃ | H | H | CONH₂ | rac | H | H | | H | H | 1 | | H | H | H | H | - |
| 36. | CN | H | H | CONH₂ | rac | H | H | | H | H | 1 | | H | H | H | H | - |
| 37. | SO₂CH₃ | H | H | H | rac | H | H | | | | 0 | | CH₃ | CH₃ | H | H | O |
| 38. | CN | H | H | H | rac | H | H | | | | 0 | | CH₃ | CH₃ | H | H | O |
| 39. | CF3 | H | H | H | rac | H | H | | | | 0 | | CH₃ | CH₃ | H | H | O |
| 40. | SO₂CH₃ | H | H | H | rac | H | H | | H | H | 1 | | H | Cl | H | H | - |
| 41. | SO₂CH₃ | H | H | H | rac | H | H | | H | H | 1 | | H | F | H | H | - |
| 42. | SO₂CH₃ | H | H | H | rac | H | H | | H | H | 1 | | H | H | F | H | - |
| 43. | SO₂CH₃ | H | H | H | rac | H | H | | H | H | 1 | | H | H | Cl | H | - |
| 44. | SO₂CH₃ | H | H | H | rac | H | H | | H | H | 1 | | H | OCH₃ | OCH₃ | H | - |
| 45. | CN | H | H | H | rac | H | H | | H | H | 1 | | H | Cl | H | H | - |
| 46. | CF₃ | H | H | H | rac | H | H | | H | H | 1 | | H | Cl | H | H | - |
| 47. | SO₂CH₃ | H | H | H | rac | H | H | | H | H | 1 | | H | H | CH₃ | H | S |
| 48. | SO₂CH₃ | H | H | H | rac | H | H | | H | H | 1 | | H | H | F | H | S |
| 49. | SO₂CH₃ | H | H | H | rac | H | H | | H | H | 1 | | CH₃ | H | CH₃ | H | S |
| 50. | SO₂CH₃ | H | H | H | rac | H | H | | H | H | 1 | | Cl | H | Cl | H | S |
| 51. | SO₂CH₃ | H | H | H | rac | H | H | | H | H | 1 | | CH₃ | H | OCH₃ | H | S |
| 52. | SO₂CH₃ | H | H | H | rac | H | H | | H | H | 1 | | OCH₃ | H | H | H | S |
| 53. | SO₂CH₃ | H | H | H | rac | H | H | | H | H | 1 | | OCH₂C H₃ | H | H | H | S |
| 54. | SO₂CH₃ | H | H | H | rac | H | H | | H | H | 1 | | N(CH₃)₂ | H | H | H | S |
| 55. | SO₂CH₃ | H | H | H | rac | H | H | | H | H | 1 | | H | H | Br | H | S |
| 56. | SO₂CH₃ | H | H | H | rac | H | H | | H | H | 1 | | H | H | C≡CH | H | S |
| 57. | SO₂CH₃ | H | H | H | rac | H | H | | H | H | 1 | | H | H | C≡CCH₃ | H | S |
| 58. | SO₂CH₃ | H | H | H | rac | H | H | | H | H | 1 | | H | H | CN | H | S |
| 59. | CF₃ | H | H | H | rac | H | H | | H | H | 1 | | H | H | CH₃ | H | S |
| 60. | CN | H | H | H | rac | H | H | | H | H | 1 | | H | H | CH₃ | H | S |
| 61. | SO₂CH₃ | H | H | H | rac | H | H | | H | H | 1 | | H | H | H | H | S |
| 62. | SO₂CH₃ | H | H | H | rac | H | H | | H | H | 1 | | CH₃ | H | CH₃ | H | O |
| 63. | CF₂CF₃ | H | H | H | R | H | H | | H | H | 2 | | H | H | H | H | - |
| 64. | CN | H | H | H | rac | H | H | | H | H | 1 | | H | H | CH₂CH₃ | H | O |
| 65. | SO₂CH₃ | H | H | H | rac | H | H | | H | H | 1 | | H | H | CH₂CH₃ | H | O |
| 66. | SO₂CH₃ | CN | H | H | rac | H | H | | H | H | 1 | | H | H | CH₂CH₃ | H | O |
| 67. | SO₂CH₃ | CONH₂ | H | H | rac | H | H | | H | H | 1 | | H | H | CH₂CH₃ | H | O |
| 68. | SO₂CH₃ | COOH | H | H | rac | H | H | | H | H | 1 | | H | H | CH₂CH₃ | H | O |
| 69. | SO₂CH₃ | COOCH₃ | H | H | rac | H | H | | H | H | 1 | | H | H | CH₂CH₃ | H | O |
| 70. | CF₃ | H | H | H | rac | H | H | | H | H | 1 | | H | H | CH₂CH₃ | H | O |
| 71. | SOCH₃ | OCH₃ | H | H | R | H | H | | H | H | 2 | | H | H | H | H | - |
| 72. | SO₂CH₃ | OCH₂CH₃ | H | H | R | H | H | | H | H | 2 | | H | H | H | H | - |
| 73. | SOCH₃ | OCH₃ | OCH₃ | H | R | H | H | | H | H | 1 | | H | H | CH₃ | H | - |
| 74. | SOCH₃ | OCH₃ | OCH₃ | H | R | CH₃ | H | S | H | H | 1 | | H | H | CH₃ | H | - |
| 75. | SO₂CH₃ | OCH₃ | OCH₃ | H | R | CH₃ | H | S | H | H | 1 | | H | H | CH₃ | H | - |
| 76. | CN | H | H | H | R | CH₃ | H | S | H | H | 1 | | H | H | CH₃ | H | - |
| 77. | COOCH₂CH₃ | H | H | H | R | CH₃ | H | S | H | H | 1 | | H | H | CH₃ | H | - |
| 78. | COOCH₃ | H | H | H | R | CH₃ | H | S | H | H | 1 | | H | H | CH₃ | H | - |
| 79. | CONH₂ | H | H | H | R | CH₃ | H | S | H | H | 1 | | H | H | CH₃ | H | - |
| 80. | CON(CH₃)₂ | H | H | H | R | CH₃ | H | S | H | H | 1 | | H | H | CH₃ | H | - |
| 81. | SO₂CH₃ | SO₂CH₃ | H | H | R | H | H | | H | H | 2 | | H | H | H | H | - |
| 82. | COOCH₂CH₃ | H | H | H | R | H | H | | H | H | 2 | | H | H | H | H | - |
| 83. | CN | H | H | H | R | H | H | | H | H | 2 | | H | H | H | H | - |
| 84. | SO₂CH₃ | H | H | H | rac | H | H | | H | H | 1 | | H | OCH₃ | H | H | O |
| 85. | SO₂CH₃ | H | H | H | rac | H | H | | H | H | 1 | | OCH₃ | OCH₃ | H | H | O |
| 86. | SO₂CH₃ | H | H | H | rac | H | H | | H | H | 1 | | H | OCH₃ | OCH₃ | H | O |
| 87. | SO₂CH₃ | CONH₂ | H | H | R | H | H | | H | H | 2 | | H | H | H | H | - |
| 88. | SO₂CH₃ | H | H | H | rac | CN | H | rac | H | H | 1 | | H | H | H | H | - |
| 89. | SO₂CH₃ | CN | H | H | rac | H | H | | | | 0 | | H | H | H | H | O |
| 90. | SO₂CH₃ | CONH₂ | H | H | rac | H | H | | H | H | 1 | | F | H | H | H | - |
| 91. | CF₃ | CONH₂ | H | H | rac | H | H | | | | 0 | | H | H | H | H | O |
| 92. | SO₂CH₃ | H | H | H | rac | H | H | | H | H | 1 | | H | H | H | H | O |
| 93. | SO₂CH₃ | CONH₂ | H | H | R | CH₃ | H | S | H | H | 1 | | H | H | CH₃ | H | - |
| 94. | SCH₃ | OH | CF₃ | H | R | CH₃ | H | S | H | H | 1 | | H | H | CH₃ | H | - |
| 95. | SCH₃ | OH | CF₂H | H | R | CH₃ | H | S | H | H | 1 | | H | H | CH₃ | H | - |
| 96. | SCH₃ | H | CF₃ | H | R | CH₃ | H | S | H | H | 1 | | H | H | CH₃ | H | - |
| 97. | SO₂CH₃ | OH | CF₃ | H | R | CH₃ | H | S | H | H | 1 | | H | H | CH₃ | H | - |
| 98. | SO₂CH₃ | OCH₃ | CF₃ | H | R | CH₃ | H | S | H | H | 1 | | H | H | CH₃ | H | - |
| 99. | CN | CN | H | H | R | H | H | | H | H | 1 | | H | H | H | H | - |
| 100. | CN | CN | CF₃ | H | R | H | H | | H | H | 1 | | H | H | H | H | - |
| 101. | CN | CN | H | H | R | H | H | | H | H | 2 | | H | H | H | H | - |
| 102. | CN | CN | CF₃ | H | R | H | H | | H | H | 2 | | H | H | H | H | - |
| 103. | CN | CN | H | H | R | CH₃ | H | S | H | H | 1 | | H | H | CH₃ | H | - |
| 104. | CN | CN | CF₃ | H | R | CH₃ | H | S | H | H | 1 | | H | H | CH₃ | H | - |
| 105. | SO₂CH₃ | CONH₂ | H | H | R | H | H | | H | H | 1 | | H | H | H | H | - |
| 106. | SO₂CH₃ | CN | H | H | R | H | H | | H | H | 1 | | H | H | H | H | - |
| 107. | SO₂CH₃ | COOCH₃ | H | H | R | H | H | | H | H | 1 | | H | H | H | H | - |
| 108. | SO₂CH₃ | CN | H | H | R | CH₃ | H | S | H | H | 1 | | H | H | CH₃ | H | - |
| 109. | SO₂CH₃ | CN | H | H | R | H | H | | H | H | 2 | | H | H | H | H | - |
| 110. | SO₂CH₂CH₃ | H | H | H | rac | H | H | | H | H | 1 | | H | H | F | H | - |
| 111. | CF₃ | H | H | H | rac | H | H | | H | H | 1 | | CH₃ | H | H | H | - |
| 112. | CF₃ | H | H | H | rac | H | H | | H | H | 1 | | Cl | H | H | H | - |
| 113. | CF₃ | H | H | H | rac | H | H | | H | H | 1 | | H | H | F | H | - |
| 114. | CF₃ | H | H | H | rac | H | H | | H | H | 1 | | H | H | CH₂CH₃ | H | - |
| 115. | CF₃ | H | H | H | R | H | H | | H | H | 1 | | H | H | H | H | - |
| 116. | CF₃ | H | H | H | R | CH₃ | H | S | H | H | 1 | | H | H | CH₃ | H | - |
| 117. | CF₃ | H | H | H | R | H | H | | H | H | 2 | | H | H | H | H | - |
| 118. | C₆H₅ | COOH | H | H | R | H | H | | H | H | 2 | | H | H | H | H | - |
| 119. | C₆H₅ | CONH₂ | H | H | R | H | H | | H | H | 2 | | H | H | H | H | - |
| 120. | C₆H₅ | H | H | H | R | H | H | | H | H | 2 | | H | H | H | H | - |
| 121. | C₆H₅ | OH | CH₃ | H | R | H | H | | H | H | 2 | | H | H | H | H | - |
| 122. | C₆H₅ | OH | cyprop | H | R | H | H | | H | H | 2 | | H | H | H | H | - |
| 123. | C₆H₅ | OH | CH₃ | H | R | H | H | | H | H | 1 | | H | H | H | H | - |
| 124. | C₆H₅ | OH | CH₃ | H | R | CH₃ | H | S | H | H | 1 | | H | H | CH₃ | H | - |
| 125. | C₆H₅ | OH | cyprop | H | R | CH₃ | H | S | H | H | 1 | | H | H | CH₃ | H | - |
| 126. | C₆H₅ | OH | cyprop | H | R | H | H | | H | H | 1 | | H | H | H | H | - |
| 127. | CH₃ | COOH | H | H | R | H | H | | H | H | 2 | | H | H | H | H | - |
| 128. | CH₃ | H | H | H | R | H | H | | H | H | 2 | | H | H | H | H | - |
| 129. | SCH₃ | COOH | H | H | R | H | H | | H | H | 2 | | H | H | H | H | - |
| 130. | SCH₃ | H | H | H | R | H | H | | H | H | 2 | | H | H | H | H | - |
| 131. | SO₂CH₃ | H | H | H | R | H | H | | H | H | 1 | | H | H | H | H | - |
| 132. | SO₂CH₃ | COOH | H | H | R | H | H | | H | H | 1 | | H | H | H | H | - |
| 133. | SO₂CH₃ | COOCH₂CH₃ | H | H | R | H | H | | H | H | 1 | | H | H | H | H | - |
| 134. | SO₂CH₃ | H | H | H | R | CH₃ | H | S | H | H | 1 | | H | H | CH₃ | H | - |
| 135. | SO₂CH₃ | CONHCH₃ | H | H | R | CH₃ | H | S | H | H | 1 | | H | H | CH₃ | H | - |
| 136. | SO₂CH₃ | CONHCH₃ | H | H | R | H | H | | H | H | 2 | | H | H | H | H | - |
| 137. | SO₂CH₃ | CONHCH₂CH =CH₂ | H | H | R | H | H | | H | H | 2 | | H | H | H | H | - |
| 138. | SO₂CH₃ | CONHcyprop | H | CH₃ | R | H | H | | H | H | 2 | | H | H | H | H | - |
| 139. | SO₂CH₃ | CONHcyprop | H | H | R | CH₃ | H | S | H | H | 1 | | H | H | CH₃ | H | - |
| 140. | SO₂CH₃ | CONHC₆H₅ | H | H | R | CH₃ | H | S | H | H | 1 | | H | H | CH₃ | H | - |
| 141. | SO₂CH₃ | CONHC₆H₅ | H | H | R | H | H | | H | H | 2 | | H | H | H | H | - |
| 142. | SO₂CH₃ | CONHCH₂C₆ H₅ | H | H | R | H | H | | H | H | 2 | | H | H | H | H | - |
| 143. | SO₂CH₃ | CF₃ | H | H | R | CH₃ | H | S | H | H | 1 | | H | H | H | H | - |
| 144. | SO₂CH₃ | CH₃ | H | H | R | CH₃ | H | S | H | H | 1 | | H | H | CH₃ | H | - |
| 145. | SO₂CH₃ | CH₃ | H | H | R | H | H | | H | H | 1 | | H | H | CH₃ | H | - |
| 146. | SO₂CH₃ | CH₃ | H | H | R | CH₃ | H | S | H | H | 1 | | H | H | H | H | - |
| 147. | SO₂CH₃ | OCH₃ | H | H | R | CH₃ | H | S | H | H | 1 | | H | H | CH₃ | H | - |
| 148. | SO₂CH₃ | CH(CH₃)₂ | H | H | R | CH₃ | H | S | H | H | 1 | | H | H | CH₃ | H | - |
| 149. | SO₂CH₃ | CH₃ | H | H | R | H | H | | H | H | 2 | | H | H | H | H | - |
| 150. | SO₂CH₃ | CF₃ | H | H | R | H | H | | H | H | 2 | | H | H | H | H | - |
| 151. | SO₂CH₃ | CF₂H | H | H | R | H | H | | H | H | 2 | | H | H | H | H | - |
| 152. | SOCH₃ | CF₃ | H | H | R | H | H | | H | H | 2 | | H | H | H | H | - |
| 153. | SOCH₃ | CN | H | H | R | H | H | | H | H | 2 | | H | H | H | H | - |
| 154. | SO₂CH₃ | CF₃ | H | H | R | H | H | | H | H | 1 | | H | H | H | H | O |
| 155. | SO₂CH₃ | CN | H | H | rac | H | H | | H | H | 1 | | H | H | H | H | O |
| 156. | SO₂CH₃ | CF₃ | H | H | rac | H | H | | H | H | 1 | | H | H | CH₃ | H | O |
| 157. | SO₂CH₃ | CH₃ | H | H | rac | H | H | | H | H | 1 | | H | H | CH₃ | H | O |
| 158. | SO₂CH₃ | CN | H | H | rac | H | H | | H | H | 1 | | H | H | CH₃ | H | O |
| 159. | SO₂CH₃ | H | H | H | rac | H | H | | H | H | 2 | | H | H | CH₃ | H | - |
| 160. | SO₂CH₃ | H | H | H | rac | H | H | | H | H | 2 | | F | H | CH₃ | H | - |
| 161. | SO₂CH₃ | H | H | H | rac | H | H | | H | H | 1 | | F | H | CH₃ | H | O |
| 162. | SO₂CH₃ | H | H | H | rac | H | H | | H | H | 1 | | CH₃ | H | F | H | O |
| 163. | SO₂CH₃ | H | H | H | rac | H | H | | H | H | 1 | | CH₃ | H | H | H | O |
| 164. | SO₂CH₃ | CN | H | H | rac | H | H | | H | H | 1 | | CH₃ | H | H | H | O |
| 165. | SO₂CH₃ | OCH₂C≡CH | H | H | rac | H | H | | H | H | 1 | | CH₃ | H | H | H | O |
| 166. | SO₂CH₃ | C(CH₃)₃ | H | H | rac | H | H | | H | H | 1 | | CH₃ | H | H | H | O |
| 167. | CN | CF₃ | H | H | rac | H | H | | H | H | 1 | | CH₃ | H | H | H | O |
| 168. | SO₂CH₃ | H | H | H | rac | H | H | | H | H | 2 | | CH₃ | H | H | H | - |
| 169. | SO₂CH₃ | H | H | H | rac | H | H | | CH₃ | CH₃ | 1 | | CH₃ | H | H | H | O |
| 170. | SO₂CH₃ | H | H | H | rac | H | H | | C₆H₅ | H | 1 | rac | H | H | H | H | O |
| 171. | SO₂CH₃ | H | H | H | rac | OH | H | rac | H | H | 1 | | H | H | H | H | - |
| 172. | SO₂CH₃ | H | H | H | rac | OCH₃ | H | rac | H | H | 1 | | H | H | H | H | - |
| 173. | SO₂CH₃ | H | H | H | rac | H | H | | H | H | 1 | | H | H | OCH₃ | H | - |
| 174. | SO₂CH₃ | H | H | H | rac | H | H | | H | H | 2 | | H | OCH₃ | OCH₃ | H | - |
| 175. | SO₂CH₃ | COOH | H | H | rac | H | H | | H | H | 2 | | H | OCH₃ | OCH₃ | H | - |
| 176. | SO₂CH₃ | CONH₂ | H | H | rac | H | H | | H | H | 2 | | H | OCH₃ | OCH₃ | H | - |
| 177. | SO₂CH₃ | COOCH₃ | H | H | rac | H | H | | H | H | 2 | | H | OCH₃ | OCH₃ | H | - |
| 178. | SO₂CH₃ | COOH | H | H | rac | H | H | | H | H | 1 | | H | H | OCH₃ | H | - |
| 179. | SO₂CH₃ | COOCH₃ | H | H | rac | H | H | | H | H | 1 | | H | H | OCH₃ | H | - |
| 180. | SO₂CH₃ | CONH₂ | H | H | rac | H | H | | H | H | 1 | | H | H | OCH₃ | H | - |
| 181. | SO₂CH₃ | CONH₂ | H | H | rac | H | H | | H | H | 1 | | H | OCH₃ | OCH₃ | H | - |
| 182. | CN | COOH | H | H | rac | H | H | | H | H | 1 | | CH₃ | H | H | H | O |
| 183. | CN | CONH₂ | H | H | rac | H | H | | H | H | 1 | | CH₃ | H | H | H | O |
| 184. | CN | COOCH₃ | H | H | rac | H | H | | H | H | 1 | | CH₃ | H | H | H | O |
| 185. | SO₂CH₃ | COOH | H | H | rac | H | H | | H | H | 1 | | CH₃ | H | H | H | O |
| 186. | SO₂CH₃ | CONH₂ | H | H | rac | H | H | | H | H | 1 | | CH₃ | H | H | H | O |
| 187. | SO₂CH₃ | COOCH₃ | H | H | rac | H | H | | H | H | 1 | | CH₃ | H | H | H | O |
| 188. | SO₂CH₃ | COOCH₃ | H | H | rac | H | H | | H | H | 1 | | F | H | H | H | O |
| 189. | SO₂CH₃ | CONHCH₃ | H | H | rac | H | H | | H | H | 1 | | F | H | H | H | O |
| 190. | SO₂CH₃ | CONH₂ | H | H | rac | H | H | | H | H | 1 | | F | H | H | H | O |
| 191. | SO₂CH₃ | COOH | H | H | rac | H | H | | H | H | 2 | | CH₃ | H | CH₃ | H | - |
| 192. | SO₂CH₃ | COOCH₃ | H | H | rac | H | H | | H | H | 2 | | CH₃ | H | CH₃ | H | - |
| 193. | SO₂CH₃ | COOCH₂CH3 | H | H | rac | H | H | | H | H | 2 | | CH₃ | H | CH₃ | H | - |
| 194. | SO₂CH₃ | COOCH₂ CH₂0CH₃ | H | H | rac | H | H | | H | H | 2 | | CH₃ | H | CH₃ | H | - |
| 195. | SO₂CH₃ | COOCH₂CH₂ N(CH₃)₂ | H | H | rac | H | H | | H | H | 2 | | CH₃ | H | CH₃ | H | - |
| 196. | SO₂CH₃ | CON(CH₂ CH₃)₂ | H | H | rac | H | H | | H | H | 2 | | CH₃ | H | CH₃ | H | - |
| 197. | SO₂CH₃ | CONH₂ | H | H | rac | H | H | | H | H | 2 | | CH₃ | H | CH₃ | H | - |
| 198. | SO₂CH₃ | CON(CH₃)₂ | H | H | rac | H | H | | H | H | 2 | | CH₃ | H | CH₃ | H | - |
| 199. | SO₂CH₃ | CONHcyprop | H | H | rac | H | H | | H | H | 2 | | CH₃ | H | CH₃ | H | - |
| 200. | SO₂CH₃ | H | H | H | rac | CH₃ | H | rac | H | H | 2 | | H | H | CH₃ | H | - |
| 201. | SO₂CH₃ | H | H | H | rac | CH₃ | H | rac | H | H | 1 | | H | H | CH₃ | H | O |
| 202. | SO₂CH₃ | H | H | H | rac | CH₃ | H | rac | H | H | 1 | | H | H | H | H | O |
| 203. | SO₂CH₃ | H | H | H | rac | H | H | | | | 0 | | H | H | H | H | -CH₂-O |
| 204. | SO₂CH₃ | H | H | H | rac | H | H | | | | 0 | | H | H | Br | H | -CH₂-O- |
| 205. | SO₂CH₃ | H | H | H | rac | H | H | | | | 0 | | H | H | CH=CH₂ | H | -CH₂-O- |
| 206. | SO₂CH₃ | H | H | H | rac | H | H | | | | 0 | | H | H | C=CH | H | -CH₂-O- |
| 207. | SO₂CH₃ | COOH | H | H | rac | H | H | | | | 0 | | H | H | H | H | -CH₂-O- |
| 208. | SO₂CH₃ | CONH₂ | H | H | rac | H | H | | | | 0 | | H | H | H | H | -CH₂-O- |
| 209. | CN | H | H | H | rac | H | H | | | | 0 | | H | H | H | H | -CH₂-O- |
| 210. | CF₃ | H | H | H | rac | H | H | | | | 0 | | H | H | H | H | -CH₂-O- |
| 211. | CF₃ | CN | H | H | rac | H | H | | | | 0 | | H | H | H | H | -CH₂-O- |
| 212. | CN | CN | H | H | rac | H | H | | | | 0 | | H | H | H | H | -CH₂-O- |
| 213. | CN | CF₃ | H | H | rac | H | H | | | | 0 | | H | H | H | H | -CH₂-O- |
| 214. | CH₃ | CH₃ | H | H | R | H | H | | H | H | 2 | | H | H | H | H | - |
| 215. | CF₂H | H | H | H | R | H | H | | H | H | 2 | | H | H | H | H | - |
| 216. | CHF-C₆H₅ | H | H | H | R | H | H | | H | H | 2 | | H | H | H | H | - |
| 217. | OC₆H₅ | H | H | H | R | H | H | | H | H | 2 | | H | H | H | H | - |
| 218. | OCH₂C≡ CCH₃ | H | H | H | R | H | H | | H | H | 2 | | H | H | H | H | - |
| 219. | OCH₂C≡CH | H | H | H | R | H | H | | H | H | 2 | | H | H | H | H | - |
| 220. | OCH₂CH₃ | H | H | H | R | H | H | | H | H | 2 | | H | H | H | H | - |
| 221. | OCH₃ | H | H | H | R | H | H | | H | H | 2 | | H | H | H | H | - |
| 222. | OCH₂CF₃ | H | H | H | R | H | H | | H | H | 2 | | H | H | H | H | - |
| 223. | CH₃ | CH₃ | H | H | R | CH₃ | H | S | H | H | 1 | | H | H | CH₃ | H | - |
| 224. | CH(CH₃)₂ | CH₃ | H | H | R | CH₃ | H | S | H | H | 1 | | H | H | CH₃ | H | - |
| 225. | SOCH₃ | H | H | H | R | CH₃ | H | S | H | H | 1 | | H | H | CH₃ | H | - |
| 226. | CH(CH₃)₂ | H | H | H | R | CH₃ | H | S | H | H | 1 | | H | H | CH₃ | H | - |
| 227. | OCH(CH₃)C≡ CH | H | H | H | R | CH₃ | H | S | H | H | 1 | | H | H | CH₃ | H | - |
| 228. | CH₂C₆H₄-4-Cl | H | H | H | R | CH₃ | H | S | H | H | 1 | | H | H | CH₃ | H | - |
| 229. | OC₆H₅ | H | H | H | R | CH₃ | H | S | H | H | 1 | | H | H | CH₃ | H | - |
| 230. | OCH₂C≡CH | H | H | H | R | CH₃ | H | S | H | H | 1 | | H | H | CH₃ | H | - |
| 231. | OCH₂CH₃ | H | H | H | R | CH₃ | H | S | H | H | 1 | | H | H | CH₃ | H | - |
| 232. | OCH₂C₆H₅ | H | H | H | R | CH₃ | H | S | H | H | 1 | | H | H | CH₃ | H | - |
| 233. | OCH₃ | H | H | H | R | CH₃ | H | S | H | H | 1 | | H | H | CH₃ | H | - |
| 234. | OCH₃ | H | H | H | R | H | H | | H | H | 1 | | H | H | CH₃ | H | - |
| 235. | OCH₃ | CONH₂ | H | H | R | CH₃ | H | S | H | H | 1 | | H | H | CH₃ | H | - |
| 236. | CONH₂ | CONH₂ | H | H | R | CH₃ | H | S | H | H | 1 | | H | H | CH₃ | H | - |
| 237. | SCF₃ | CONH₂ | H | H | R | CH₃ | H | S | H | H | 1 | | H | H | CH₃ | H | - |
| 238. | S(O)₂CF₃ | CONH₂ | H | H | R | CH₃ | H | S | H | H | 1 | | H | H | CH₃ | H | - |
| 239. | OC₆H₅ | CONH₂ | H | H | R | CH₃ | H | S | H | H | 1 | | H | H | CH₃ | H | - |
| 240. | OCH₃ | COOCH₃ | H | H | R | CH₃ | H | S | H | H | 1 | | H | H | CH₃ | H | - |
| 241. | OCH₃ | COOH | H | H | R | CH₃ | H | S | H | H | 1 | | H | H | CH₃ | H | - |
| 242. | OCH₂CH₃ | CN | H | H | R | CH₃ | H | S | H | H | 1 | | H | H | CH₃ | H | - |
| 243. | OCH₃ | CN | H | H | R | CH₃ | H | S | H | H | 1 | | H | H | CH₃ | H | - |
| 244. | OCH₂C≡CH | CN | H | H | R | CH₃ | H | S | H | H | 1 | | H | H | CH₃ | H | - |
| 245. | NO₂ | CH₃ | H | H | R | CH₃ | H | S | H | H | 1 | | H | H | CH₃ | H | - |
| 246. | NO₂ | CH₃ | H | H | R | H | H | | H | H | 1 | | H | H | CH₃ | H | - |
| 247. | NO₂ | CH₃ | H | H | R | CH₃ | H | S | H | H | 1 | | H | H | H | H | - |
| 248. | NO₂ | CH₃ | H | H | R | H | H | | H | H | 1 | | H | H | H | H | - |
| 249. | NO₂ | CH₃ | H | H | R | H | H | | H | H | 2 | | H | H | H | H | - |
| 250. | NO₂ | CH₃ | H | H | R | H | H | | H | H | 2 | | H | H | CH₃ | H | - |
| 251. | NO₂ | CH₃ | H | H | rac | H | H | | H | H | 2 | | CH₃ | H | CH₃ | H | - |
| 252. | NO₂ | CH₃ | H | H | rac | H | H | | H | H | 1 | | CH₃ | H | H | H | O |
| 253. | NO₂ | CH₃ | H | H | rac | H | H | | H | H | 1 | | F | H | H | H | O |
| 254. | NO₂ | CH₃ | H | H | rac | H | H | | H | H | 2 | | F | H | H | H | - |
| 255. | SCH₃ | CH₃ | H | H | R | CH₃ | H | S | H | H | 1 | | H | H | CH₃ | H | - |
| 256. | CF₃ | OH | CH₃ | H | R | CH₃ | H | S | H | H | 1 | | H | H | CH₃ | H | - |
| 257. | CF₃ | CH₃ | CH₃ | H | R | CH₃ | H | S | H | H | 1 | | H | H | CH₃ | H | - |
| 258. | SO₂CH₃ | CH₃ | CH₃ | H | R | CH₃ | H | S | H | H | 1 | | H | H | CH₃ | H | - |
| 259. | SO₂CH₃ | OH | CH₃ | H | R | CH₃ | H | S | H | H | 1 | | H | H | CH₃ | H | - |
| 260. | SO₂CH₃ | OH | CONH₂ | H | R | CH₃ | H | S | H | H | 1 | | H | H | CH₃ | H | - |
| 261. | SO₂CH₃ | CH₃ | CONH₂ | H | R | CH₃ | H | S | H | H | 1 | | H | H | CH₃ | H | - |
| 262. | SO₂CH₃ | CH₃ | CN | H | R | CH₃ | H | S | H | H | 1 | | H | H | CH₃ | H | - |
| 263. | SO₂CH₃ | OH | CN | H | R | CH₃ | H | S | H | H | 1 | | H | H | CH₃ | H | - |
| 264. | SO₂CH₃ | H | H | H | rac | CH₃ | CH₃ | | H | H | 1 | | H | H | H | H | - |
| 265. | SO₂CH₃ | H | H | H | rac | -CH₂-CH₂- | | | H | H | 1 | | H | H | H | H | - |
| 266. | SO₂CH₃ | H | H | H | rac | CH₂C H₃ | CH₂C H₃ | | H | H | 1 | | H | H | H | H | - |
| 267. | SO₂CH₃ | H | H | H | rac | H | H | | H | H | 1 | | H | H | H | H | C=O |
| 268. | SO₂CH₃ | H | H | H | rac | H | H | | H | H | 1 | | H | H | H | H | CHCH₃ |
| 269. | SO₂CH₃ | H | H | H | rac | H | H | | H | H | 1 | | H | H | H | H | NCH₃ |
| 270. | SO₂CH₃ | H | H | H | rac | H | H | | H | H | 1 | | H | H | H | H | NH |
| 271. | SO₂CH₃ | H | H | H | rac | H | H | | H | H | 1 | | H | H | H | H | C(CH₃)₂ |
| 272. | SO₂CH₃ | H | H | H | rac | H | H | | | | 0 | | H | H | H | H | -CH₂-S- |
| 273. | SO₂CH₃ | H | H | H | R | H | H | | H | H | 2 | | H | H | H | H | - |
| 274. | SO₂CH₃ | H | H | H | rac | H | H | | H | H | 2 | | F | H | H | H | - |

Weiterer Gegenstand der vorliegenden Erfindung sind Verfahren zur Herstellung entsprechender Verbindungen der allgemeinen Formel (I) und/oder deren Salze und/oder deren agrochemisch verträglichen quaternierten Stickstoff-Derivate:
a.) Zur Herstellung von Verbindungen der allgemeinen Formel (I) 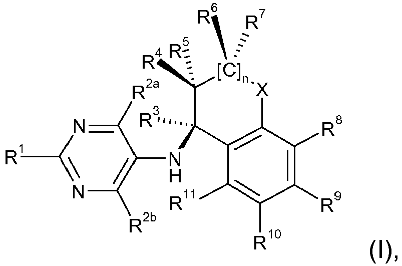
   in welchen die Reste R¹ bis R¹¹ und X sowie n die vorstehenden Bedeutungen aufweisen, wird eine Verbindung der allgemeinen Formel (II) 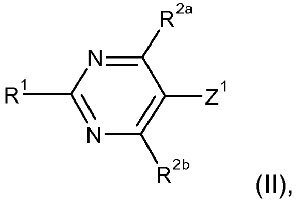
   worin R¹ und R^{2a} sowie R^{2b} die vorstehende Bedeutung aufweisen und
   Z¹ für einen austauschfähigen Rest oder eine Abgangsgruppe steht,
   mit einem Amin der allgemeinen Formel (III) oder einem Säureadditionssalz des Amins der allgemeinen Formel (III) 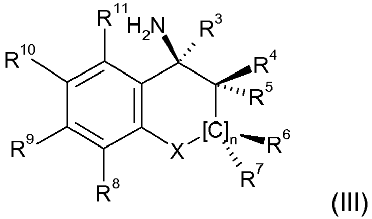
   umgesetzt, wobei die Reste R³ bis R¹¹ und X sowie n die vorstehende Bedeutung aufweisen.

Der austauschfähige Rest Z¹, bzw. die Abgangsgruppe Z¹ steht insbesondere für Fluor, Chlor, Brom, Jod, ein (C₁-C₄)-Alkylsulfonyl, ein unsubstituiertes oder ein substituiertes Phenyl-(C₁-C₄)-alkylsulfonyl oder ein (C₁-C₄)-Alkylphenyl-sulfonyl.

Besonders bevorzugt als Abgangsgruppen für Z¹ sind Brom, Chlor oder Fluor (siehe A. Synthesebeispiele, insbesondere Bsp. 64, Bsp. 92, Bsp. 115 und Bsp. 273).

Die Verbindungen der allgemeinen Formel (II) sind kommerziell verfügbar oder können nach bekannten Verfahren hergestellt werden. Gegebenenfalls können auch Verbindungen der Formel (II) mit R^{2a} gleich COOH eingesetzt werden, wobei dann unter den gewählten Reaktionsbedingungen die COOH decarboxyliert und in Wasserstoff überführt wird. Gegebenenfalls können auch Verbindungen der Formel (II) mit R^{2a} gleich COOR^{2aa} eingesetzt werden, wobei R^{2aa} für (C₁-C₄)-Alkyl, (C₁-C₄)-Alkylphenyl oder andere esterbildende Gruppen steht und dann unter den gewählten Reaktionsbedingungen die COOR^{2aa} in situ verseift oder anschließend verseift in R^{2a} gleich COOH überführt wird, dann die COOH-Gruppe decarboxyliert und in Wasserstoff überführt wird.

Gegebenenfalls kann die Umsetzung auch durch unterschiedliche Hilfsstoffe katalysiert werden, wie beispielswiese durch die Reagenzien Kaliumphosphat, Kupfer(I)iodid und N,N-diethyl-2-hydroxybenzamide oder im Sinne der Buchwald-Hartwig-Kupplung durch spezielle Übergangsmetallkatalysatorsysteme.

Beispielsweise können 2-Methylthio-5-chlor-pyrimidine-4carbonsäuren aus Mucochloric Säure und S-Methylthiuronium sulphate (Liang, Yong-min , Luo, Sheng-jun , Zhang, Zhao-xin and Yong-xiang(2002)'EFFICIENT SYNTHESIS OF A NEW PYRIMIDINE DERIVATIVE', Synthetic Communications, 32: 1, 153 - 157, http://dx.doi.org/10.1081/SCC-120001523) hergestellt werden und nachfolgend zum Methansulfonyl-Derivat oxidiert werden. Analog kann auch Mucobromic Säure eingesetzt werden (Collect. Czech. Chem. Commun. 1949, 14, 223; Tetrahedron. Lett. 1976, 693).

Die Amine der allgemeinen Formel (III) oder die Säureadditionssalz davon sind kommerziell verfügbar oder deren Synthese ist in WO2004/069814 A1 beschrieben.
b.) Gegebenenfalls können analog dem in WO2004/92166 A2, Seite 68, beschriebenen Verfahren 5-Aminopyrimidine mit kommerziell verfügbaren oder nach bekannten Methoden herstellbaren Ketonen umgesetzt und beispielswiese mit Natriumtriacetoxyborhydrid zu Verbindungen entsprechend der Formel (I) hydriert werden.
   Gegebenenfalls kann die Reaktion auch in zwei voneinander getrennten Teilschritten durchgeführt werden, indem zunächst das Amin mit kommerziell verfügbaren oder nach bekannten Methoden herstellbaren Ketonen zu einem Imin umgesetzt wird und dann das so erhaltene Intermediat hydriert wird.
c.) Zur Herstellung von Verbindungen der allgemeinen Formel (I) können Verbindungen als Vorstufen verwendet werden und in andere erfindungsgemäße Verbindungen umgewandelt werden.
   (1) Beispielsweise können Derivate der Formel (I) mit R¹, R^{2a}, R^{2b} oder R¹⁰ = Hal, insbesondere Jod oder Brom mit Acetylenen oder Trimethylsilylgeschützten Acetylen unter Übergangsmetallkatalyse z.B. mit Bis-(triphenylphosphin)-palladium-(II)-chlorid in protischen oder aprotischen Lösemitteln und Basenzusatz bei Temperaturen zwischen 20 und 150 °C zu Verbindungen der Formel (I) mit R¹, R^{2a}, R^{2b} oder R¹⁰ = Alkinyl umgesetzt werden.
   (2) Beispielsweise können Derivate der Formel (I) mit R¹ oder R^{2a} oder R^{2b} = CN sauer oder basenkatalysiert verseift werden, die so erhaltenen Carbonsäuren können nach bekannten Verfahren in Säurechloride und diese wiederum in Amide überführt werden.
   (3) Beispielsweise können Derivate der Formel (I) mit R¹ oder R^{2a} oder R^{2b} = Hal, insbesondere Chlor oder Brom oder SO₂Me oder (C₁-C₆)-Alkylsulfonyl oder Phenyl(C₁-C₆)-Alkylsulfonyl in protischen oder aprotischen Lösemitteln und Basenzusatz bei Temperaturen zwischen 100 und 200 °C durch Reaktion mit Alkoholaten oder Aminen zu Verbindungen der Formel (I) mit R¹ oder R^{2a} oder R^{2b} = Alkoxyalkyl oder Aminoalkyl oder Diaminioalkyl umgesetzt werden.
   (4) Beispielsweise können Derivate der Formel (I) mit R¹ oder R^{2a} oder R^{2b} = COOH in Säurechloride oder gemischte Anhydride überführt werden und dann mit Alkoholen oder Aminen zur Reaktion gebracht werden, so dass man dann die entsprechenden Corabonsäureester oder Amide erhält.
   (5) Beispielsweise können Derivate der Formel (I) mit R¹ oder R^{2a} oder R^{2b} = CONH₂ durch Entwässern mit Phosphoroxychlorid in die entsprechenden Nitrile überführt werden.
   (6) Beispielsweise können Derivate der Formel (I) mit R¹ oder R^{2a} oder R^{2b} = SO₂Me oder (C₁-C₆)-Alkylsulfonyl oder Phenyl(C₁-C₆)-Alkylsulfonyl mit Formaniliden (vergl.: Ihara Chem Ind (TSUB) J6-2099305 and J6-2106084) in die entsprechenden Anilide überführt werden.

Kollektionen aus Verbindungen der Formel (I) und/oder deren Salzen, die nach den oben genannten Reaktionen synthetisiert werden können, können auch in parallelisierter Weise hergestellt werden, wobei dies in manueller, teilweise automatisierter oder vollständig automatisierter Weise geschehen kann. Dabei ist es beispielsweise möglich, die Reaktionsdurchführung, die Aufarbeitung oder die Reinigung der Produkte bzw. Zwischenstufen zu automatisieren. Insgesamt wird hierunter eine Vorgehensweise verstanden, wie sie beispielsweise durch D. Tiebes in Combinatorial Chemistry - Synthesis, Analysis, Screening (Herausgeber Günther Jung), Verlag Wiley 1999, auf den Seiten 1 bis 34 beschrieben ist.

Zur parallelisierten Reaktionsdurchführung und Aufarbeitung können eine Reihe von im Handel erhältlichen Geräten verwendet werden, beispielsweise Calpyso-Reaktionsblöcke (Caylpso reaction blocks) der Firma Barnstead International, Dubuque, Iowa 52004-0797, USA oder Reaktionsstationen (reaction stations) der Firma Radleys, Shirehill, Saffron Walden, Essex, CB 11 3AZ, England oder MultiPROBE Automated Workstations der Firma Perkin Elmar, Waltham, Massachusetts 02451, USA. Für die parallelisierte Aufreinigung von Verbindungen der allgemeinen Formel (I) und deren Salzen beziehungsweise von bei der Herstellung anfallenden Zwischenprodukten stehen unter anderem Chromatographieapparaturen zur Verfügung, beispielsweise der Firma ISCO, Inc., 4700 Superior Street, Lincoln, NE 68504, USA.

Die aufgeführten Apparaturen führen zu einer modularen Vorgehensweise, bei der die einzelnen Arbeitsschritte automatisiert sind, zwischen den Arbeitsschritten jedoch manuelle Operationen durchgeführt werden müssen. Dies kann durch den Einsatz von teilweise oder vollständig integrierten Automationssystemen umgangen werden, bei denen die jeweiligen Automationsmodule beispielsweise durch Roboter bedient werden. Derartige Automationssysteme können zum Beispiel von der Firma Caliper, Hopkinton, MA 01748, USA bezogen werden.

Die Durchführung einzelner oder mehrerer Syntheseschritte kann durch den Einsatz von Polymer-supported reagents/Scavanger-Harze unterstützt werden. In der Fachliteratur sind eine Reihe von Versuchsprotokollen beschrieben, beispielsweise in ChemFiles, Vol. 4, No. 1, Polymer-Supported Scavengers and Reagents for Solution-Phase Synthesis (Sigma-Aldrich).

Neben den hier beschriebenen Methoden kann die Herstellung von Verbindungen der allgemeinen Formel (I) und deren Salzen vollständig oder partiell durch Festphasen unterstützte Methoden erfolgen. Zu diesem Zweck werden einzelne Zwischenstufen oder alle Zwischenstufen der Synthese oder einer für die entsprechende Vorgehensweise angepassten Synthese an ein Syntheseharz gebunden. Festphasenunterstützte Synthesemethoden sind in der Fachliteratur hinreichend beschrieben, z.B. Barry A. Bunin in "The Combinatorial Index", Verlag Academic Press, 1998 und Combinatorial Chemistry - Synthesis, Analysis, Screening (Herausgeber Günther Jung), Verlag Wiley, 1999. Die Verwendung von Festphasen- unterstützten Synthesemethoden erlaubt eine Reihe von literaturbekannten Protokollen, die wiederum manuell oder automatisiert ausgeführt werden können. Die Reaktionen können beispielsweise mittels IRORI-Technologie in Mikroreaktoren (microreactors) der Firma Nexus Biosystems, 12140 Community Road, Poway, CA92064, USA durchgeführt werden.

Sowohl an fester als auch in flüssiger Phase kann die Durchführung einzelner oder mehrerer Syntheseschritte durch den Einsatz der Mikrowellen-Technologie unterstützt werden. In der Fachliteratur sind eine Reihe von Versuchsprotokollen beschrieben, beispielsweise in Microwaves in Organic and Medicinal Chemistry (Herausgeber C. O. Kappe und A. Stadler), Verlag Wiley, 2005.

Die Herstellung gemäß der hier beschriebenen Verfahren liefert Verbindungen der Formel (I) und deren Salze in Form von Substanzkollektionen, die Bibliotheken genannt werden. Gegenstand der vorliegenden Erfindung sind auch Bibliotheken, die mindestens zwei Verbindungen der Formel (I) und deren Salzen enthalten.

Weiterer Gegenstand der Erfindung ist aufgrund der herbiziden Eigenschaft der Verbindungen der allgemeinen Formel (I) auch die Verwendung der erfindungsgemäßen Verbindungen der allgemeinen Formel (I) als Herbizide zur Bekämpfung von Schadpflanzen.

Herbizide werden in landwirtschaftlich genutzten Kulturen während verschiedener Anbauphasen eingesetzt. So erfolgt die Applikation einiger Produkte schon vor oder während der Saat. Andere werden ausgebracht, bevor die Kulturpflanze aufläuft, d.h. bevor der Keimling die Erdoberfläche durchbricht (Vorauflauf-Herbizide). Nachauflauf-Herbizide schließlich werden verwendet, wenn von der Kulturpflanze entweder bereits die Keimblätter oder Laubblätter ausgebildet sind.

Die erfindungsgemäßen Verbindungen können dabei sowohl im Vorlauf als auch im Nachlauf angewendet werden, wobei eine Verwendung der erfindungsgemäßen Verbindungen im Vorlauf bevorzugt ist.

Die Vorauflauf-Behandlung schließt sowohl die Behandlung der Anbaufläche vor der Aussaat (ppi = pre plant incorporation) als auch die Behandlung der angesäten, aber noch nicht bewachsenen Anbauflächen ein.

Die erfindungsgemäßen Verbindungen der Formel (I) und deren Salze, im folgenden auch synonym zusammen auch als Verbindungen der Formel (I) bezeichnet, weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es gleichgültig, ob die Substanzen im Vorsaat-, Vorauflauf- oder Nachauflaufverfahren ausgebracht werden.

Im einzelnem seien beispielhaft einige Vertreter der mono- und dikotylen Unkrautflora genannt, die durch die erfindungsgemäßen Verbindungen der allgemeinen Formel (I) kontrolliert werden können, ohne daß durch die Nennung eine Beschränkung auf bestimmte Arten erfolgen soll.

Auf der Seite der monokotylen Unkrautarten werden z. B. Agrostis, Alopecurus, Apera, Avena, Brachicaria, Bromus, Dactyloctenium, Digitaria, Echinochloa, Eleocharis, Eleusine, Festuca, Fimbristylis, Ischaemum, Lolium, Monochoria, Panicum, Paspalum, Phalaris, Phleum, Poa, Sagittaria, Scirpus, Setaria, Sphenoclea, sowie Cyperusarten vorwiegend aus der annuellen Gruppe und auf Seiten der perennierenden Spezies Agropyron, Cynodon, Imperata sowie Sorghum und auch ausdauernde Cyperusarten gut erfaßt.

Bei dikotylen Unkrautarten erstreckt sich das Wirkungsspektrum auf Arten wie z. B. Galium, Viola, Veronica, Lamium, Stellaria, Amaranthus, Sinapis, Ipomoea, Matricaria, Abutilon und Sida auf der annuellen Seite sowie Convolvulus, Cirsium, Rumex und Artemisia bei den perennierenden Unkräutern. Außerdem wird herbizide Wirkung bei dikotylen Unkräutern wie Ambrosia, Anthemis, Carduus, Centaurea, Chenopodium, Cirsium, Convolvulus, Datura, Emex, Galeopsis, Galinsoga, Lepidium, Lindernia, Papaver, Portlaca, Polygonum, Ranunculus, Rorippa, Rotala, Seneceio, Sesbania, Solanum, Sonchus, Taraxacum, Trifolium, Urtica und Xanthium beobachtet.

Werden die erfindungsgemäßen Verbindungen der allgemeinen Formel (I) vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe der allgemeinen Formel (I) auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstumsstadium stehen oder sterben nach einer gewissen Zeit ganz ab, so daß auf diese Weise eine für die Kulturpflanzen schädliche Unkrautkonkurrenz sehr früh und nachhaltig beseitigt wird.

Obgleich die erfindungsgemäßen Verbindungen der allgemeinen Formel (I) eine ausgezeichnete herbizide Aktivität gegenüber mono- und dikotylen Unkräutern aufweisen, werden Kulturpflanzen wirtschaftlich bedeutender Kulturen wie z. B. Weizen, Gerste, Roggen, Reis, Mais, Zuckerrübe, Baumwolle, Raps und Soja nur unwesentlich oder gar nicht geschädigt. Die vorliegenden Verbindungen eignen sich aus diesen Gründen sehr gut zur selektiven Bekämpfung von unerwünschtem Pflanzenwuchs in landwirtschaftlichen Nutzpflanzungen.

Darüber hinaus weisen die erfindungsgemäßen Substanzen der allgemeinen Formel (I) hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z. B. durch Auslösen von Desikkation und Wuchsstauchung eingesetzt werden. Desweiteren eignen sie sich auch zur generellen Steuerung und Hemmung von unerwünschtem vegetativem Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann.

Aufgrund ihrer herbiziden und pflanzenwachstumsregulatorischen Eigenschaften können die Wirkstoffe auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt. Weitere besondere Eigenschaften können in einer Toleranz oder Resistenz gegen abiotische Stressoren z. B. Hitze, Kälte, Trockenheit, Salz und ultraviolette Strahlung liegen.

Bevorzugt ist die Anwendung der erfindungsgemäßen Verbindungen der allgemeinen Formel (I) oder deren Salze in wirtschaftlich bedeutenden transgenen Kulturen von Nutz- und Zierpflanzen, z. B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten.

Vorzugsweise können die Verbindungen der allgemeinen Formel (I) als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z. B. EP 0221044, EP 0131624). Beschrieben wurden beispielsweise in mehreren Fällen
- gentechnische Veränderungen von Kulturpflanzen zwecks Modifikation der in den Pflanzen synthetisierten Stärke (z. B. WO 92/011376, WO 92/014827, WO 91/019806),
- transgene Kulturpflanzen, welche gegen bestimmte Herbizide vom Typ Glufosinate (vgl. z. B. EP 0242236, EP 0242246) oder Glyphosate (WO 92/000377) oder der Sulfonylharnstoffe (EP 0257993, US 5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP 0142924, EP 0193259).
- transgene Kulturpflanzen mit modifizierter Fettsäurezusammensetzung (WO 91/013972).
- gentechnisch veränderte Kulturpflanzen mit neuen Inhalts- oder Sekundärstoffen z. B. neuen Phytoalexinen, die eine erhöhte Krankheitsresistenz verursachen (EP 0309862, EP 0464461)
- gentechnisch veränderte Pflanzen mit reduzierter Photorespiration, die höhere Erträge und höhere Stresstoleranz aufweisen (EP 0305398)
- transgene Kulturpflanzen, die pharmazeutisch oder diagnostisch wichtige Proteine produzieren ("molecular pharming")
- transgene Kulturpflanzen, die sich durch höhere Erträge oder bessere Qualität auszeichnen
- transgene Kulturpflanzen die sich durch eine Kombinationen z. B. der o. g. neuen Eigenschaften auszeichnen ("gene stacking")

Zahlreiche molekularbiologische Techniken, mit denen neue transgene Pflanzen mit veränderten Eigenschaften hergestellt werden können, sind im Prinzip bekannt; siehe z. B. I. Potrykus und G. Spangenberg (eds.) Gene Transfer to Plants, Springer Lab Manual (1995), Springer Verlag Berlin, Heidelberg oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden, siehe z. B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996.

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.

Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen der allgemeinen Formel (I) in transgenen Kulturen eingesetzt werden, welche gegen Wuchsstoffe, wie z. B. Dicamba oder gegen Herbizide, die essentielle Pflanzenenzyme, z. B. Acetolactatsynthasen (ALS), EPSP Synthasen, Glutaminsynthasen (GS) oder Hydoxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, resistent sind.

Bei der Anwendung der erfindungsgemäßen Wirkstoffe der allgemeinen Formel (I) in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen der allgemeinen Formel (I) als Herbizide zur Bekämpfung von Schadpflanzen in transgenen Kulturpflanzen.

Die Verbindungen der allgemeinen Formel (I) können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie ÖI-in-Wasser- und Wasser-in-Öl-Emulsionen, versprühbare Lösungen, Suspensionskonzentrate (SC), Dispersionen auf ÖI- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.

Diese einzelnen Formulierungstypen sind im Prinzip bekannt und werden beispielsweise beschrieben in: Winnacker-Küchler, "Chemische Technologie", Band 7, C. Hanser Verlag München, 4. Aufl. 1986, Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösemittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hanser Verlag München, 4. Aufl. 1986.

Auf der Basis dieser Formulierungen lassen sich auch Kombinationen mit anderen pestizid wirksamen Stoffen, wie z. B. Insektiziden, Akariziden, Herbiziden, Fungiziden, sowie mit Safenern, Düngemitteln und/oder Wachstumsregulatoren herstellen, z. B. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z. B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-Dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösemittel z. B. Butanol, Cyclohexanon, Dimethylformamid, Xylol oder auch höhersiedenden Aromaten oder Kohlenwasserstoffen oder Mischungen der organischen Lösemittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Cadodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z. B. Sorbitanfettsäureester oder Polyoxethylensorbitanester wie z. B. Polyoxyethylensorbitanfettsäureester.

Stäubemittel erhält man durch Vermahlen des Wirkstoffes mit fein verteilten festen Stoffen, z. B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z. B. oben bei den anderen Formulierungstypen bereits aufgeführt sind, hergestellt werden.

Emulsionen, z. B. ÖI-in-Wasser-Emulsionen (EW), lassen sich beispielsweise mittels Rühren, Kolloidmühlen und/oder statischen Mischern unter Verwendung von wäßrigen organischen Lösemitteln und gegebenenfalls Tensiden, wie sie z. B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z. B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt. Zur Herstellung von Teller-, Fließbett-, Extruder- und Sprühgranulate siehe z. B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Einzelheiten zur Formulierung von Pflanzenschutzmitteln siehe z. B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0,1 bis 99 Gew.-%, insbesondere 0,1 bis 95 Gew.-%, Wirkstoff der Formel (I).

In Spritzpulvern beträgt die Wirkstoffkonzentration z. B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0,05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe u.a. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösemittel, Füll-, Träger- und Farbstoffe, Entschäumer, Verdunstungshemmer und den pH-Wert und die Viskosität beeinflussende Mittel.

Die Verbindungen der allgemeinen Formel (I) oder deren Salze können als solche oder in Form ihrer Zubereitungen (Formulierungen) mit anderen pestizid wirksamen Stoffen, wie z. B. Insektiziden, Akariziden, Nematiziden, Herbiziden, Fungiziden, Safenern, Düngemitteln und/oder Wachstumsregulatoren kombiniert eingesetzt werden, z. B. als Fertigformulierung oder als Tankmischungen.

Als Kombinationspartner für die erfindungsgemäßen Verbindungen in Mischungsformulierungen oder im Tank-Mix sind beispielsweise bekannte Wirkstoffe, die auf einer Inhibition von beispielsweise Acetolactat-Synthase, Acetyl-CoA-Carboxylase, Cellulose-Synthase, Enolpyruvylshikimat-3-phosphat-Synthase, Glutamin-Synthetase, p-Hydroxyphenylpyruvat-Dioxygenase, Phytoendesaturase, Photosystem I, Photosystem II oder Protoporphyrinogen-Oxidase beruhen, einsetzbar, wie sie z.B. aus Weed Research 26 (1986) 441-445 oder "The Pesticide Manual", 15th edition, The British Crop Protection Council and the Royal Soc. of Chemistry, 2006 und dort zitierter Literatur beschrieben sind. Nachfolgend werden beispielhaft bekannte Herbizide oder Pflanzenwachstumsregulatoren genannt, die mit den erfindungsgemäßen Verbindungen kombiniert werden können, wobei diese Wirkstoffe entweder mit dem "common name" nach der International Organization for Standardization (ISO) oder mit dem chemischen Namen bzw. mit der Codenummer bezeichnet sind. Dabei sind stets sämtliche Anwendungsformen wie beispielsweise Säuren, Salze, Ester sowie auch alle isomere Formen wie Stereoisomere und optische Isomere umfaßt, auch wenn sie nicht explizit erwähnt sind.

Beispiele für herbizide Mischungspartner sind:
Acetochlor, Acifluorfen, Acifluorfen-Natrium, Aclonifen, Alachlor, Allidochlor, Alloxydim, Alloxydim-Natrium, Ametryn, Amicarbazone, Amidochlor, Amidosulfuron, Aminocyclopyrachlor, Aminocyclopyrachlor-Kalium, Aminocyclopyrachlor-methyl, Aminopyralid, Amitrole, Ammoniumsulfamat, Anilofos, Asulam, Atrazine, Azafenidin, Azimsulfuron, Beflubutamid, Benazolin, Benazolin-ethyl, Benfluralin, Benfuresat, Bensulfuron, Bensulfuron-methyl, Bensulid, Bentazon, Benzobicyclon, Benzofenap, Bicyclopyron, Bifenox, Bilanafos, Bilanafos-Natrium, Bispyribac, Bispyribac-Natrium, Bromacil, Bromobutide, Bromofenoxim, Bromoxynil, Busoxinone, Butachlor, Butafenacil, Butamifos, Butenachlor, Butralin, Butroxydim, Butylat, Cafenstrol, Carbetamide, Carfentrazone, Carfentrazone-ethyl, Chloramben, Chlorbromuron, Chlorfenac, Chlorfenac-Natrium, Chlorfenprop, Chlorflurenol, Chlorflurenol-methyl, Chloridazon, Chlorimuron, Chlorimuron-ethyl, Chlorophthalim, Chlorotoluron, Chlorthal-dimethyl, Chlorsulfuron, Cinidon, Cinidon-ethyl, Cinmethylin, Cinosulfuron, Clethodim, Clodinafop, Clodinafop-propargyl, Clomazon, Clomeprop, Clopyralid, Cloransulam, Cloransulam-methyl, Cumyluron, Cyanamide, Cyanazine, Cycloat, Cyclosulfamuron, Cycloxydim, Cyhalofop, Cyhalofop-butyl, Cyprazin, 2,4-D, -2,4-D-butotyl, -butyl, -dimethylammonium, diolamin, -ethyl, 2-ethylhexyl, -isobutyl, -isooctyl, - Isopropylammonium, -Kalium, -Triisopropanolammonium und -Trolamine, 2,4-DB, 2,4-DB-butyl, -dimethylammonium, isooctyl, -Kalium und -Natrium, Daimuron (Dymron), Dalapon, n-Decanol, Desmedipham, Detosyl-Pyrazolate (DTP), Dicamba, Dichlobenil, Dichlorprop, Dichlorprop-P, Diclofop, Diclofop-methyl, Diclofop-P-methyl, Diclosulam, Difenzoquat, Diflufenican, Diflufenzopyr, Diflufenzopyr-Natrium, Dimefuron, Dimepiperat, Dimethachlor, Dimethametryn, Dimethenamid, Dimethenamid-P, Dimetrasulfuron, Dinitramin, Diquat, Diquat-dibromid, Dithiopyr, Diuron, DNOC, Endothal, EPTC, Esprocarb, Ethalfluralin, Ethametsulfuron, Ethametsulfuron-methyl, Ethiozin, Ethofumesate, Ethoxyfen, Ethoxyfen-ethyl, Ethoxysulfuron, Etobenzanid, F-5331, d.h. N-[2-Chlor-4-fluor-5-[4-(3-fluorpropyl)-4,5-dihydro-5-oxo-1 H-tetrazol-1-yl]-phenyl]-ethansulfonamid, F-7967, d.h. 3-[7-Chlor-5-fluor-2-(trifluormethyl)-1H-benzimidazol-4-yl]-1-methyl-6-(trifluormethyl)pyrimidin-2,4(1H,3H)-dion, Fenoxaprop, Fenoxaprop-P, Fenoxaprop-ethyl, Fenoxaprop-P-ethyl, Fenoxasulfon, Fentrazamid, Flamprop, Flamprop-M-isopropyl, Flamprop-M-methyl, Flazasulfuron, Florasulam, Fluazifop, Fluazifop-P, Fluazifop-butyl, Fluazifop-P-butyl, Flucarbazon, Flucarbazon-Natrium, Flucetosulfuron, Fluchloralin, Flufenacet (Thiafluamide), Flufenpyr, Flufenpyrethyl, Flumetsulam, Flumiclorac, Flumiclorac-pentyl, Flumioxazin, Fluometuron, Fluoroglycofen, Fluoroglycofen-ethyl, Flupropanat, Flupyrsulfuron, Flupyrsulfuronmethyl-Natrium, Fluridon, Flurochloridon, Fluroxypyr, Fluroxypyr-meptyl, Flurtamon, Fluthiacet, Fluthiacet-methyl, Fluthiamide, Fomesafen, Foramsulfuron, Fosamin, Glufosinat, Glufosinat-Ammonium, Glufosinat-P, Glufosinat-P-Ammonium, Glufosinat-P-Natrium, Glyphosat, Glyphosat-Isopropylammonium, -Diammonium, - Dimethylammonium, -Kalium, -Natrium und -Trimesium, H-9201, i.e. 0-(2,4-Dimethyl-6-nitrophenyl)-O-ethyl-isopropylphosphoramidothioat, Halosafen, Halosulfuron, Halosulfuron-methyl, Haloxyfop, Haloxyfop-P, Haloxyfop-ethoxyethyl, Haloxyfop-P-ethoxyethyl, Haloxyfop-methyl, Haloxyfop-P-methyl, Harnstoff-Sulfat, Hexazinon, HW-02, d. h. 1-(Dimethoxyphosphoryl)-ethyl-(2,4-dichlorphenoxy)acetat, Imazamethabenz, Imazamethabenz-methyl, Imazamox, Imazamox-ammonium, Imazapic, Imazapyr, Imazapyr-Isopropylammonium, Imazaquin, Imazaquin-ammonium, Imazethapyr, Imazethapyr-Ammonium, Imazosulfuron, Indanofan, Indaziflam, lodosulfuron, lodosulfuron-methyl-Natrium, loxynil, Ipfencarbazon, Isoproturon, Isouron, Isoxaben, Isoxaflutol, KUH-043, d. h. 3-({[5-(Difluormethyl)-1-methyl-3-(trifluormethyl)-1H-pyrazol-4-yl]methyl}sulfonyl)-5,5-dimethyl-4,5-dihydro-1,2-oxazol, Karbutilat, Ketospiradox, Lactofen, Lenacil, Linuron, MCPA, MCPB, MCPB-methyl, -ethyl und -natrium, Mecoprop, Mecoprop-natrium, Mecoprop-butotyl, Mecoprop-P-butotyl, Mecoprop-P-dimethylammonium, Mecoprop-P-2-ethylhexyl, Mecoprop-P-Kalium, Mefenacet, Mefluidid, Mepiquat-chlorid, Mesosulfuron, Mesosulfuron-methyl, Mesotrion, Methabenzthiazuron, Metam, Metamifop, Metamitron, Metazachlor, Metazasulfuron, Methiopyrsulfuron, Methiozolin, Methylisothiocyanat, Metobromuron, Metolachlor, S-Metolachlor, Metosulam, Metoxuron, Metribuzin, Metsulfuron, Metsulfuron-methyl, Molinat, Monolinuron, Monosulfuron, Monosulfuron-ester, MT-128, d. h. 6-Chlor-N-[(2E)-3-chlorprop-2-en-1-yl]-5-methyl-N-phenylpyridazin-3-amin, MT-5950, d. h. N-[3-Chlor-4-(1-methylethyl)-phenyl]-2-methylpentanamid, NGGC-011, Napropamid, NC-310, d.h. 4-(2,4-Dichlorbenzoyl)-1-methyl-5-benzyloxypyrazol, Neburon, Nicosulfuron, Nonansäure (Pelargonsäure), Norflurazon, Ölsäure (Fettsäuren), Orbencarb, Orthosulfamuron, Oryzalin, Oxadiargyl, Oxadiazon, Oxasulfuron, Oxaziclomefon, Oxyfluorfen, Paraquat, Paraquat-dichlorid, Pebulat, Pendimethalin, Penoxsulam, Pentachlorphenol, Pentoxazon, Pethoxamid, Petroleumöle, Phenmedipham, Picloram, Picolinafen, Pinoxaden, Piperophos, Pretilachlor, Primisulfuron, Primisulfuron-methyl, Prodiamine, Prifluraline, Profoxydim, Prometon, Prometryn, Propachlor, Propanil, Propaquizafop, Propazine, Propham, Propisochlor, Propoxycarbazon, Propoxycarbazon-Natrium, Propyrisulfuron, Propyzamide, Prosulfocarb, Prosulfuron, Pyraclonil, Pyraflufen, Pyraflufen-ethyl, Pyrasulfotol, Pyrazolynat (Pyrazolat), Pyrazosulfuron, Pyrazosulfuron-ethyl, Pyrazoxyfen, Pyribambenz, Pyribambenzisopropyl, Pyribambenz-propyl, Pyribenzoxim, Pyributicarb, Pyridafol, Pyridat, Pyriftalid, Pyriminobac, Pyriminobac-methyl, Pyrimisulfan, Pyrithiobac, Pyrithiobac-natrium, Pyroxasulfon, Pyroxsulam, Quinclorac, Quinmerac, Quinoclamin, Quizalofop, Quizalofop-ethyl, Quizalofop-P, Quizalofop-P-ethyl, Quizalofop-P-tefuryl, Rimsulfuron, Saflufenacil, Sethoxydim, Siduron, Simazin, Simetryn, Sulcotrion, Sulfentrazon, Sulfometuron, Sulfometuron-methyl, Sulfosat (Glyphosat-Trimesium), Sulfosulfuron, SW-065, SYN-523, SYP-249, d. h. 1-Ethoxy-3-methyl-1-oxobut-3-en-2-yl-5-[2-chlor-4-(trifluormethyl)phenoxy]-2-nitrobenzoat, SYP-300, d. h. 1-[7-Fluor-3-oxo-4-(prop-2-in-1-yl)-3,4-dihydro-2H-1,4-benzoxazin-6-yl]-3-propyl-2-thioxoimidazolidin-4,5-dion, TCA, TCA-Natrium, Tebuthiuron, Tefuryltrion, Tembotrion, Tepraloxydim, Terbacil, Terbucarb, Terbumeton, Terbuthylazin, Terbutryn, Thenylchlor, Thiazopyr, Thiencarbazon, Thiencarbazon-methyl, Thifensulfuron, Thifensulfuron-methyl, Thiobencarb, Topramezon, Tralkoxydim, Triafamon, Triallat, Triasulfuron, Triaziflam, Tribenuron, Tribenuron-methyl, Triclopyr, Trietazin, Trifloxysulfuron, Trifloxysulfuronnatrium, Trifluralin, Triflusulfuron, Triflusulfuron-methyl, Tritosulfuron, Vernolat, ZJ-0862, d. h. 3,4-Dichlor-N-{2-[(4,6-dimethoxypyrimidin-2-yl)oxy]benzyl}anilin, sowie die folgenden Verbindungen:

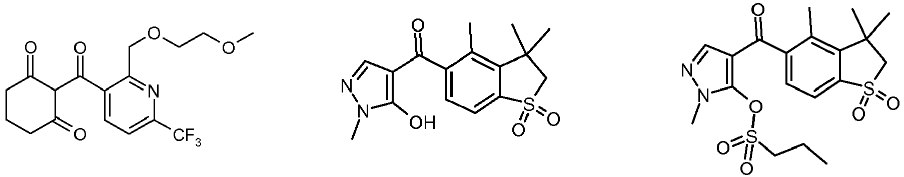

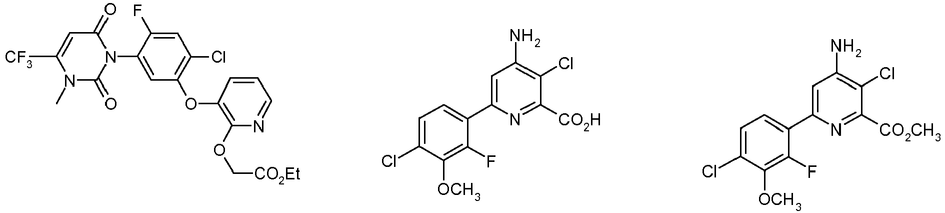

Beispiele für Pflanzenwachstumsregulatoren als mögliche Mischungspartner sind:
Acibenzolar, Acibenzolar-S-methyl, Ancymidol, 5-Aminolävulinsäure, 6-Benzylaminopurin, Chlormequat-chlorid, Cloprop, Cyclanilid, 3-(Cycloprop-1-enyl)propionsäure, Daminozid, Dazomet, Dikegulac, Dikegulac-Natrium, Ethephon, Flumetralin, Flurenol, Flurenol-butyl, Flurprimidol, Forchlorfenuron, Gibberellinsäure, Inabenfide, Indolessigsäure (IAA), 4-lndol-3-ylbuttersäure, Jasmonsäure, Jasmonsäuremethylester, Maleinsäurehydrazid, Mepiquat-chlorid, 1-Methylcyclopropen, 2-(1-Naphthyl)acetamid, 1-Naphthylessigsäure, 2-Naphthyloxyessigsäure, Nitrophenolat-Natrium (Isomerengemisch), 4-Oxo-4[(2-phenylethyl)amino]buttersäure, Paclobutrazol, N-Phenylphthalsäureamind, Prohexadion, Prohexadion-Calcium, Prohydrojasmon, Salicyclsäure, Strigolacton, Tecnazen, Thidiazuron, Triacontanol, Trinexapac, Trinexapac-ethyl, Tsitodef, Uniconazol, Uniconazol-P.

Von besonderem Interesse ist die selektive Bekämpfung von Schadpflanzen in Kulturen von Nutz- und Zierpflanzen. Obgleich die erfindungsgemäßen Verbindungen der allgemeinen Formel (I) bereits in vielen Kulturen sehr gute bis ausreichende Selektivität aufweisen, können prinzipiell in einigen Kulturen und vor allem auch im Falle von Mischungen mit anderen Herbiziden, die weniger selektiv sind, Phytotoxizitäten an den Kulturpflanzen auftreten. Diesbezüglich sind Kombinationen erfindungsgemäßer Verbindungen der allgemeinen Formel (I) von besonderem Interesse, welche die Verbindungen der allgemeinen Formel (I) bzw. deren Kombinationen mit anderen Herbiziden oder Pestiziden und Safenern enthalten. Die Safener, welche in einem antidotisch wirksamen Gehalt eingesetzt werden, reduzieren die phytotoxischen Nebenwirkungen der eingesetzten Herbizide/Pestizide, z. B. in wirtschaftlich bedeutenden Kulturen wie Getreide (Weizen, Gerste, Roggen, Mais, Reis, Hirse), Zuckerrübe, Zuckerrohr, Raps, Baumwolle und Soja, vorzugswiese Getreide. Folgende Gruppen von Verbindungen kommen beispielsweise als Safener für die Verbindungen (I) alleinig oder aber in deren Kombinationen mit wieteren Pestiziden in Frage:
Die Safener sind vorzugsweise ausgewählt aus der Gruppe bestehend aus:
   S1) Verbindungen der Formel (S1), 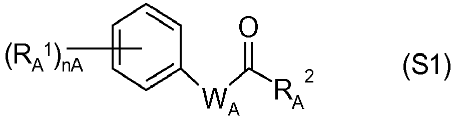 wobei die Symbole und Indizes folgende Bedeutungen haben:
      - nA: ist eine natürliche Zahl von 0 bis 5, vorzugsweise 0 bis 3;
      - R_{A}¹: ist Halogen, (C₁-C₄)Alkyl, (C₁-C₄)Alkoxy, Nitro oder (C₁-C₄)Haloalkyl,
      - W_{A}: ist ein unsubstituierter oder substituierter divalenter heterocyclischer Rest aus der Gruppe der teilungesättigten oder aromatischen Fünfring-Heterocyclen mit 1 bis 3 Heteroringatomen aus der Gruppe N und O, wobei mindestens ein N-Atom und höchstens ein O-Atom im Ring enthalten ist, vorzugsweise ein Rest aus der Gruppe (W_{A}¹) bis (W_{A}⁴),

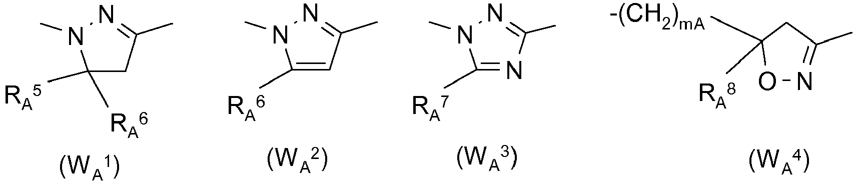

      mA ist 0 oder 1;
      R_{A}² ist OR_{A}³, SR_{A}³ oder NR_{A}³R_{A}⁴ oder ein gesättigter oder ungesättigter 3- bis 7-gliedriger Heterocyclus mit mindestens einem N-Atom und bis zu 3 Heteroatomen, vorzugsweise aus der Gruppe O und S, der über das N-Atom mit der Carbonylgruppe in (S1) verbunden ist und unsubstituiert oder durch Reste aus der Gruppe (C₁-C₄)Alkyl, (C₁-C₄)Alkoxy oder gegebenenfalls substituiertes Phenyl substituiert ist, vorzugsweise ein Rest der Formel OR_{A}³, NHR_{A}⁴ oder N(CH₃)₂, insbesondere der Formel OR_{A}³;
      R_{A}³ ist Wasserstoff oder ein unsubstituierter oder substituierter aliphatischer Kohlenwasserstoffrest, vorzugsweise mit insgesamt 1 bis 18 C-Atomen;
      R_{A}⁴ ist Wasserstoff, (C₁-C₆)Alkyl, (C₁-C₆)Alkoxy oder substituiertes oder unsubstituiertes Phenyl;
      R_{A}⁵ ist H, (C₁-C₈)Alkyl, (C₁-C₈)Haloalkyl, (C₁-C₄)Alkoxy(C₁-C₈)Alkyl, Cyano oder COOR_{A}⁹, worin R_{A}⁹ Wasserstoff, (C₁-C₈)Alkyl, (C₁-C₈)Haloalkyl, (C₁-C₄)Alkoxy-(C₁-C₄)alkyl, (C₁-C₆)Hydroxyalkyl, (C₃-C₁₂)Cycloalkyl oder Tri-(C₁-C₄)-alkyl-silyl ist;
      R_{A}⁶, R_{A}⁷, R_{A}⁸ sind gleich oder verschieden Wasserstoff, (C₁-C₈)Alkyl, (C₁-C₈)Haloalkyl, (C₃-C₁₂)Cycloalkyl oder substituiertes oder unsubstituiertes Phenyl;
vorzugsweise:
   a) Verbindungen vom Typ der Dichlorphenylpyrazolin-3-carbonsäure (S1 a), vorzugsweise Verbindungen wie 1-(2,4-Dichlorphenyl)-5-(ethoxycarbonyl)-5-methyl-2-pyrazolin-3-carbonsäure, 1-(2,4-Dichlorphenyl)-5-(ethoxycarbonyl)-5-methyl-2-pyrazolin-3-carbonsäure-ethylester (S1-1) ("Mefenpyr-diethyl"), und verwandte Verbindungen, wie sie in der WO-A-91/07874 beschrieben sind;
   b) Derivate der Dichlorphenylpyrazolcarbonsäure (S1 b), vorzugsweise Verbindungen wie 1-(2,4-Dichlorphenyl)-5-methyl-pyrazol-3-carbonsäureethylester (S1-2), 1-(2,4-Dichlorphenyl)-5-isopropyl-pyrazol-3-carbonsäureethylester (S1-3), 1-(2,4-Dichlorphenyl)-5-(1,1-dimethyl-ethyl)pyrazol-3-carbonsäureethylester (S1-4) und verwandte Verbindungen, wie sie in EP-A-333 131 und EP-A-269 806 beschrieben sind;
   c) Derivate der 1,5-Diphenylpyrazol-3-carbonsäure (S1c), vorzugsweise Verbindungen wie 1-(2,4-Dichlorphenyl)-5-phenylpyrazol-3-carbonsäureethylester (S1-5), 1-(2-Chlorphenyl)-5-phenylpyrazol-3-carbonsäuremethylester (S1-6) und verwandte Verbindungen wie sie beispielsweise in der EP-A-268554 beschrieben sind;
   d) Verbindungen vom Typ der Triazolcarbonsäuren (S1d), vorzugsweise Verbindungen wie Fenchlorazol(-ethylester), d.h. 1-(2,4-Dichlorphenyl)-5-trichlormethyl-(1H)-1,2,4-triazol-3-carbonsäureethylester (S1-7), und verwandte Verbindungen wie sie in EP-A-174 562 und EP-A-346 620 beschrieben sind;
   e) Verbindungen vom Typ der 5-Benzyl- oder 5-Phenyl-2-isoxazolin-3- carbonsäure oder der 5,5-Diphenyl-2-isoxazolin-3-carbonsäure (S1e), vorzugsweise Verbindungen wie 5-(2,4-Dichlorbenzyl)-2-isoxazolin-3-carbonsäureethylester (S1-8) oder 5-Phenyl-2-isoxazolin-3-carbonsäureethylester (S1-9) und verwandte Verbindungen, wie sie in WO-A-91/08202 beschrieben sind, bzw. 5,5-Diphenyl-2-isoxazolin-carbonsäure (S1-10) oder 5,5-Diphenyl-2-isoxazolin-carbonsäureethylester (S1-11) ("Isoxadifen-ethyl") oder -n-propylester (S1-12) oder der 5-(4-Fluorphenyl)-5-phenyl-2-isoxazolin-3-carbonsäureethylester (S1-13), wie sie in der Patentanmeldung WO-A-95/07897 beschrieben sind.

### S2) Chinolinderivate der Formel (S2),

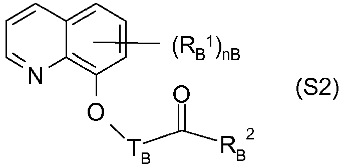

wobei die Symbole und Indizes folgende Bedeutungen haben:
- R_{B}¹: ist Halogen, (C₁-C₄)Alkyl, (C₁-C₄)Alkoxy, Nitro oder (C₁-C₄)Haloalkyl,
- nB: ist eine natürliche Zahl von 0 bis 5, vorzugsweise 0 bis 3;
- R_{B}²: ist OR_{B}³, SR_{B}³ oder NR_{B}³R_{B}⁴ oder ein gesättigter oder ungesättigter 3- bis 7-gliedriger Heterocyclus mit mindestens einem N-Atom und bis zu 3 Heteroatomen, vorzugsweise aus der Gruppe O und S, der über das N-Atom mit der Carbonylgruppe in (S2) verbunden ist und unsubstituiert oder durch Reste aus der Gruppe (C₁-C₄)Alkyl, (C₁-C₄)Alkoxy oder gegebenenfalls substituiertes Phenyl substituiert ist, vorzugsweise ein Rest der Formel OR_{B}³, NHR_{B}⁴ oder N(CH₃)₂, insbesondere der Formel OR_{B}³;
- R_{B}³: ist Wasserstoff oder ein unsubstituierter oder substituierter aliphatischer Kohlenwasserstoffrest, vorzugsweise mit insgesamt 1 bis 18 C-Atomen;
- R_{B}⁴: ist Wasserstoff, (C₁-C₆)Alkyl, (C₁-C₆)Alkoxy oder substituiertes oder unsubstituiertes Phenyl;
- T_{B}: ist eine (C₁ oder C₂)-Alkandiylkette, die unsubstituiert oder mit einem oder zwei (C₁-C₄)Alkylresten oder mit [(C₁-C₃)-Alkoxy]-carbonyl substituiert ist;

vorzugsweise:
   a) Verbindungen vom Typ der 8-Chinolinoxyessigsäure (S2a), vorzugsweise
      (5-Chlor-8-chinolinoxy)essigsäure-(1-methylhexyl)ester ("Cloquintocet-mexyl") (S2-1),
      (5-Chlor-8-chinolinoxy)essigsäure-(1,3-dimethyl-but-1-yl)ester (S2-2),
      (5-Chlor-8-chinolinoxy)essigsäure-4-allyloxy-butylester (S2-3),
      (5-Chlor-8-chinolinoxy)essigsäure-1-allyloxy-prop-2-ylester (S2-4),
      (5-Chlor-8-chinolinoxy)essigsäureethylester (S2-5),
      (5-Chlor-8-chinolinoxy)essigsäuremethylester (S2-6),
      (5-Chlor-8-chinolinoxy)essigsäureallylester (S2-7),
      (5-Chlor-8-chinolinoxy)essigsäure-2-(2-propyliden-iminoxy)-1-ethylester (S2-8), (5-Chlor-8-chinolinoxy)essigsäure-2-oxo-prop-1-ylester (S2-9) und verwandte Verbindungen, wie sie in EP-A-86 750, EP-A-94 349 und EP-A-191 736 oder EP-A-0 492 366 beschrieben sind, sowie (5-Chlor-8-chinolinoxy)essigsäure (S2-10), deren Hydrate und Salze, beispielsweise deren Lithium-, Natrium-Kalium-, Kalzium-, Magnesium-, Aluminium-, Eisen-, Ammonium-, quartäre
      Ammonium-, Sulfonium-, oder Phosphoniumsalze wie sie in der WO-A-2002/34048 beschrieben sind;
   b) Verbindungen vom Typ der (5-Chlor-8-chinolinoxy)malonsäure (S2b), vorzugsweise Verbindungen wie (5-Chlor-8-chinolinoxy)malonsäurediethylester, (5-Chlor-8-chinolinoxy)malonsäurediallylester, (5-Chlor-8-chinolinoxy)malonsäure-methyl-ethylester und verwandte Verbindungen, wie sie in EP-A-0 582 198 beschrieben sind.

### S3) Verbindungen der Formel (S3)

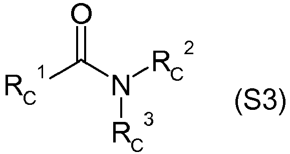

wobei die Symbole und Indizes folgende Bedeutungen haben:
R_{C}¹ ist (C₁-C₄)Alkyl, (C₁-C₄)Haloalkyl, (C₂-C₄)Alkenyl, (C₂-C₄)Haloalkenyl, (C₃-C₇)Cycloalkyl, vorzugsweise Dichlormethyl;
R_{C}², R_{C}³ sind gleich oder verschieden Wasserstoff, (C₁-C₄)Alkyl, (C₂-C₄)Alkenyl, (C₂-C₄)Alkinyl, (C₁-C₄)Haloalkyl, (C₂-C₄)Haloalkenyl, (C₁-C₄)Alkylcarbamoyl-(C₁-C₄)alkyl, (C₂-C₄)Alkenylcarbamoyl-(C₁-C₄)alkyl, (C₁-C₄)Alkoxy-(C₁-C₄)alkyl, Dioxolanyl-(C₁-C₄)alkyl, Thiazolyl, Furyl, Furylalkyl, Thienyl, Piperidyl, substituiertes oder unsubstituiertes Phenyl, oder R_{C}² und R_{C}³ bilden zusammen einen substituierten oder unsubstituierten heterocyclischen Ring, vorzugsweise einen Oxazolidin-, Thiazolidin-, Piperidin-, Morpholin-, Hexahydropyrimidin- oder Benzoxazinring;
vorzugsweise:
   Wirkstoffe vom Typ der Dichloracetamide, die häufig als Vorauflaufsafener (bodenwirksame Safener) angewendet werden, wie z. B.
   "Dichlormid" (N,N-Diallyl-2,2-dichloracetamid) (S3-1),
   "R-29148" (3-Dichloracetyl-2,2,5-trimethyl-1,3-oxazolidin) der Firma Stauffer (S3-2),
   "R-28725" (3-Dichloracetyl-2,2,-dimethyl-1,3-oxazolidin) der Firma Stauffer (S3-3),
   "Benoxacor" (4-Dichloracetyl-3,4-dihydro-3-methyl-2H-1,4-benzoxazin) (S3-4), "PPG-1292" (N-Allyl-N-[(1,3-dioxolan-2-yl)-methyl]-dichloracetamid) der Firma PPG Industries (S3-5),
   "DKA-24" (N-Allyl-N-[(allylaminocarbonyl)methyl]-dichloracetamid) der Firma Sagro-Chem (S3-6),
   "AD-67" oder "MON 4660" (3-Dichloracetyl-1-oxa-3-aza-spiro[4,5]decan) der Firma Nitrokemia bzw. Monsanto (S3-7),
   "TI-35" (1-Dichloracetyl-azepan) der Firma TRI-Chemical RT (S3-8), "Diclonon" (Dicyclonon) oder "BAS145138" oder "LAB145138" (S3-9) ((RS)-1-Dichloracetyl-3,3,8a-trimethylperhydropyrrolo[1,2-a]pyrimidin-6-on) der Firma BASF,
   "Furilazol" oder "MON 13900" ((RS)-3-Dichloracetyl-5-(2-furyl)-2,2-dimethyloxazolidin) (S3-10); sowie dessen (R)-Isomer (S3-11).

### S4) N-Acylsulfonamide der Formel (S4) und ihre Salze,

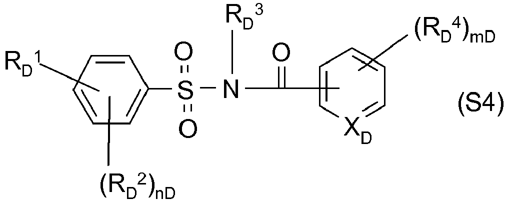

worin die Symbole und Indizes folgende Bedeutungen haben:
X_{D} ist CH oder N;
R_{D}¹ ist CO-NR_{D}⁵R_{D}⁶ oder NHCO-R_{D}⁷,
R_{D}² ist Halogen, (C₁-C₄)Haloalkyl, (C₁-C₄)Haloalkoxy, Nitro, (C₁-C₄)Alkyl, (C₁-C₄)Alkoxy, (C₁-C₄)Alkylsulfonyl, (C₁-C₄)Alkoxycarbonyl oder (C₁-C₄)Alkylcarbonyl;
R_{D}³ ist Wasserstoff, (C₁-C₄)Alkyl, (C₂-C₄)Alkenyl oder (C₂-C₄)Alkinyl;
R_{D}⁴ ist Halogen, Nitro, (C₁-C₄)Alkyl, (C₁-C₄)Haloalkyl, (C₁-C₄)Haloalkoxy, (C₃-C₆)Cycloalkyl, Phenyl, (C₁-C₄)Alkoxy, Cyano, (C₁-C₄)Alkylthio, (C₁-C₄)Alkylsulfinyl, (C₁-C₄)Alkylsulfonyl, (C₁-C₄)Alkoxycarbonyl oder (C₁-C₄)Alkylcarbonyl,
R_{D}⁵ ist Wasserstoff, (C₁-C₆)Alkyl, (C₃-C₆)Cycloalkyl, (C₂-C₆)Alkenyl, (C₂-C₆)Alkinyl, (C₅-C₆)Cycloalkenyl, Phenyl oder 3- bis 6-gliedriges Heterocyclyl enthaltend vD Heteroatome aus der Gruppe Stickstoff, Sauerstoff und Schwefel, wobei die sieben letztgenannten Reste durch vD Substituenten aus der Gruppe Halogen, (C₁-C₆)Alkoxy, (C₁-C₆)Haloalkoxy, (C₁-C₂)Alkylsulfinyl, (C₁-C₂)Alkylsulfonyl, (C₃-C₆)Cycloalkyl, (C₁-C₄)Alkoxycarbonyl, (C₁-C₄)Alkylcarbonyl und Phenyl und im Falle cyclischer Reste auch (C₁-C₄) Alkyl und (C₁-C₄)Haloalkyl substituiert sind;
R_{D}⁶ ist Wasserstoff, (C₁-C₆)Alkyl, (C₂-C₆)Alkenyl oder (C₂-C₆)Alkinyl, wobei die drei letztgenannten Reste durch vD Reste aus der Gruppe Halogen, Hydroxy, (C₁-C₄)Alkyl, (C₁-C₄)Alkoxy und (C₁-C₄)Alkylthio substituiert sind, oder
R_{D}⁵ und R_{D}⁶ gemeinsam mit dem dem sie tragenden Stickstoffatom einen Pyrrolidinyl- oder Piperidinyl-Rest bilden;
R_{D}⁷ ist Wasserstoff, (C₁-C₄)Alkylamino, Di-(C₁-C₄)alkylamino, (C₁-C₆)Alkyl, (C₃-C₆)Cycloalkyl, wobei die 2 letztgenannten Reste durch vD Substituenten aus der Gruppe Halogen, (C₁-C₄)Alkoxy, (C₁-C₆)Haloalkoxy und (C₁-C₄)Alkylthio und im Falle cyclischer Reste auch (C₁-C₄)Alkyl und (C₁-C₄)Haloalkyl substituiert sind;
nD ist 0, 1 oder 2;
mD ist 1 oder 2;
vD ist 0, 1, 2 oder 3;
davon bevorzugt sind Verbindungen von Typ der N-Acylsulfonamide, z.B. der nachfolgenden Formel (S4^{a}), die z. B. bekannt sind aus WO-A-97/45016 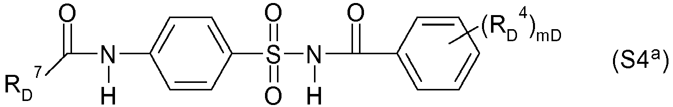 worin
R_{D}⁷ (C₁-C₆)Alkyl, (C₃-C₆)Cycloalkyl, wobei die 2 letztgenannten Reste durch vD Substituenten aus der Gruppe Halogen, (C₁-C₄)Alkoxy, (C₁-C₆)Haloalkoxy und (C₁-C₄)Alkylthio und im Falle cyclischer Reste auch (C₁-C₄)Alkyl und (C₁-C₄)Haloalkyl substituiert sind;
R_{D}⁴ Halogen, (C₁-C₄)Alkyl, (C₁-C₄)Alkoxy, CF₃;
mD 1 oder 2;
vD ist 0, 1, 2 oder 3 bedeutet;
sowie
   Acylsulfamoylbenzoesäureamide, z.B. der nachfolgenden Formel (S4^{b}), die z.B. bekannt sind aus WO-A-99/16744,

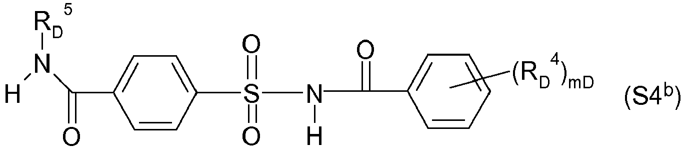

z.B. solche worin
R_{D}⁵ = Cyclopropyl und (R_{D}⁴) = 2-OMe ist ("Cyprosulfamide", S4-1),
R_{D}⁵ = Cyclopropyl und (R_{D}⁴) = 5-Cl-2-OMe ist (S4-2),
R_{D}⁵ = Ethyl und (R_{D}⁴) = 2-OMe ist (S4-3),
R_{D}⁵ = Isopropyl und (R_{D}⁴) = 5-Cl-2-OMe ist (S4-4) und
R_{D}⁵ = Isopropyl und (R_{D}⁴) = 2-OMe ist (S4-5).
sowie
   Verbindungen vom Typ der N-Acylsulfamoylphenylharnstoffe der Formel (S4^{c}), die z.B. bekannt sind aus der EP-A-365484, 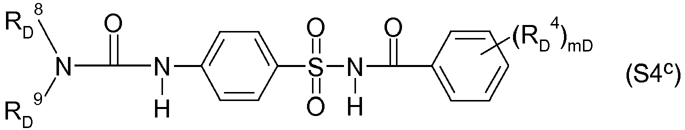
worin
   R_{D}⁸ und R_{D}⁹ unabhängig voneinander Wasserstoff, (C₁-C₈)Alkyl, (C₃-C₈)Cycloalkyl, (C₃-C₆)Alkenyl, (C₃-C₆)Alkinyl,
R_{D}⁴ Halogen, (C₁-C₄)Alkyl, (C₁-C₄)Alkoxy, CF₃
mD 1 oder 2 bedeutet;
beispielsweise
   1-[4-(N-2-Methoxybenzoylsulfamoyl)phenyl]-3-methylharnstoff,
   1-[4-(N-2-Methoxybenzoylsulfamoyl)phenyl]-3,3-dimethylharnstoff,
   1-[4-(N-4,5-Dimethylbenzoylsulfamoyl)phenyl]-3-methylharnstoff.
S5) Wirkstoffe aus der Klasse der Hydroxyaromaten und der aromatischaliphatischen Carbonsäurederivate (S5), z.B.
   3,4,5-Triacetoxybenzoesäureethylester, 3,5-Dimethoxy-4-hydroxybenzoesäure, 3,5-Dihydroxybenzoesäure, 4-Hydroxysalicylsäure, 4-Fluorsalicyclsäure, 2-Hydroxyzimtsäure, 2,4-Dichlorzimtsäure, wie sie in der WO-A-2004/084631, WO-A-2005/015994, WO-A-2005/016001 beschrieben sind.
S6) Wirkstoffe aus der Klasse der 1,2-Dihydrochinoxalin-2-one (S6), z.B. 1-Methyl-3-(2-thienyl)-1,2-dihydrochinoxalin-2-on, 1-Methyl-3-(2-thienyl)-1,2-dihydrochinoxalin-2-thion, 1-(2-Aminoethyl)-3-(2-thienyl)-1,2-dihydro-chinoxalin-2-on-hydrochlorid, 1-(2-Methylsulfonylaminoethyl)-3-(2-thienyl)-1,2-dihydrochinoxalin-2-on, wie sie in der WO-A-2005/112630 beschrieben sind.
S7) Verbindungen der Formel (S7),wie sie in der WO-A-1998/38856 beschrieben sind 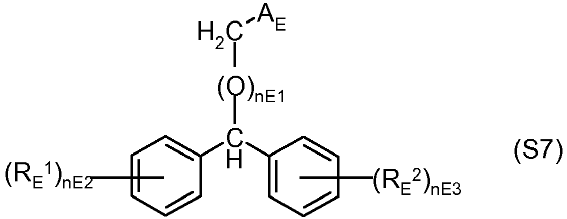 worin die Symbole und Indizes folgende Bedeutungen haben:
   - R_{E}¹, R_{E}²: sind unabhängig voneinander Halogen, (C₁-C₄)Alkyl, (C₁-C₄)Alkoxy, (C₁-C₄)Haloalkyl, (C₁-C₄)Alkylamino, Di-(C₁-C₄)Alkylamino, Nitro;
   - A_{E}: ist COOR_{E}³ oder COSR_{E}⁴
   - R_{E}³, R_{E}⁴: sind unabhängig voneinander Wasserstoff, (C₁-C₄)Alkyl, (C₂-C₆)Alkenyl, (C₂-C₄)Alkinyl, Cyanoalkyl, (C₁-C₄)Haloalkyl, Phenyl, Nitrophenyl, Benzyl, Halobenzyl, Pyridinylalkyl und Alkylammonium,
   - nE1: ist 0 oder 1
   - nE2, nE3: sind unabhängig voneinander 0, 1 oder 2,
vorzugsweise:
   Diphenylmethoxyessigsäure,
   Diphenylmethoxyessigsäureethylester,
   Diphenylmethoxyessigsäuremethylester (CAS-Reg.Nr. 41858-19-9) (S7-1).
S8) Verbindungen der Formel (S8), wie sie in der WO-A-98/27049 beschrieben sind 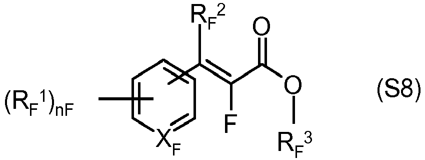 worin
   - X_{F}: CH oder N,
   - nF: für den Fall, dass X_{F}=N ist, eine ganze Zahl von 0 bis 4 und für den Fall, dass X_{F}=CH ist, eine ganze Zahl von 0 bis 5 ,
   - R_{F}¹: Halogen, (C₁-C₄)Alkyl, (C₁-C₄)Haloalkyl, (C₁-C₄)Alkoxy, (C₁-C₄)Haloalkoxy, Nitro, (C₁-C₄)Alkylthio, (C₁-C₄)-Alkylsulfonyl, (C₁-C₄)Alkoxycarbonyl, ggf. substituiertes. Phenyl, ggf. substituiertes Phenoxy,
   - R_{F}²: Wasserstoff oder (C₁-C₄)Alkyl
   - R_{F}³: Wasserstoff, (C₁-C₈)Alkyl, (C₂-C₄)Alkenyl, (C₂-C₄)Alkinyl, oder Aryl, wobei jeder der vorgenannten C-haltigen Reste unsubstituiert oder durch einen oder mehrere, vorzugsweise bis zu drei gleiche oder verschiedene Reste aus der Gruppe, bestehend aus Halogen und Alkoxy substituiert ist;bedeuten, oder deren Salze,
vorzugsweise Verbindungen worin
   - X_{F}: CH,
   - nF: eine ganze Zahl von 0 bis 2,
   - R_{F}¹: Halogen, (C₁-C₄)Alkyl, (C₁-C₄)Haloalkyl, (C₁-C₄)Alkoxy, (C₁-C₄)Haloalkoxy,
   - R_{F}²: Wasserstoff oder (C₁-C₄)Alkyl,
   - R_{F}³: Wasserstoff, (C₁-C₈)Alkyl, (C₂-C₄)Alkenyl, (C₂-C₄)Alkinyl, oder Aryl, wobei jeder der vorgenannten C-haltigen Reste unsubstituiert oder durch einen oder mehrere, vorzugsweise bis zu drei gleiche oder verschiedene Reste aus der Gruppe, bestehend aus Halogen und Alkoxy substituiert ist, bedeuten, oder deren Salze.
S9) Wirkstoffe aus der Klasse der 3-(5-Tetrazolylcarbonyl)-2-chinolone (S9), z.B. 1,2-Dihydro-4-hydroxy-1-ethyl-3-(5-tetrazolylcarbonyl)-2-chinolon (CAS-Reg.Nr. 219479-18-2), 1,2-Dihydro-4-hydroxy-1-methyl-3-(5-tetrazolyl-carbonyl)-2-chinolon (CAS-Reg.Nr. 95855-00-8), wie sie in der WO-A-1999/000020 beschrieben sind.
S10) Verbindungen der Formeln (S10^{a}) oder (S10b)
   wie sie in der WO-A-2007/023719 und WO-A-2007/023764 beschrieben sind 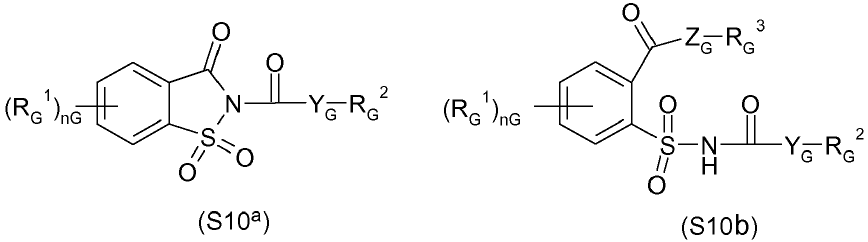
worin
R_{G}¹ Halogen, (C₁-C₄)Alkyl, Methoxy, Nitro, Cyano, CF₃, OCF₃
Y_{G}, Z_{G} unabhängig voneinander O oder S,
nG eine ganze Zahl von 0 bis 4,
R_{G}² (C₁-C₁₆)Alkyl, (C₂-C₆)Alkenyl, (C₃-C₆)Cycloalkyl, Aryl; Benzyl, Halogenbenzyl,
R_{G}³ Wasserstoff oder (C₁-C₆)Alkyl bedeutet.
S11) Wirkstoffe vom Typ der Oxyimino-Verbindungen (S11), die als Saatbeizmittel bekannt sind, wie z. B. "Oxabetrinil" ((Z)-1,3-Dioxolan-2-ylmethoxyimino(phenyl)acetonitril) (S11-1), das als Saatbeiz-Safener für Hirse gegen Schäden von Metolachlor bekannt ist, "Fluxofenim" (1-(4-Chlorphenyl)-2,2,2-trifluor-1-ethanon-O-(1,3-dioxolan-2-ylmethyl)-oxim) (S11-2), das als Saatbeiz-Safener für Hirse gegen Schäden von Metolachlor bekannt ist, und "Cyometrinil" oder "CGA-43089" ((Z)-Cyanomethoxyimino(phenyl)acetonitril) (S11-3), das als Saatbeiz-Safener für Hirse gegen Schäden von Metolachlor bekannt ist.
S12) Wirkstoffe aus der Klasse der Isothiochromanone (S12), wie z.B. Methyl-[(3-oxo-1 H-2-benzothiopyran-4(3H)-yliden)methoxy]acetate (CAS-Reg.Nr. 205121-04-6) (S12-1) und verwandte Verbindungen aus WO-A-1998/13361.
S13) Eine oder mehrere Verbindungen aus Gruppe (S13):
   "Naphthalic anhydrid" (1,8-Naphthalindicarbonsäureanhydrid) (S13-1), das als Saatbeiz-Safener für Mais gegen Schäden von Thiocarbamatherbiziden bekannt ist,
   "Fenclorim" (4,6-Dichlor-2-phenylpyrimidin) (S13-2), das als Safener für Pretilachlor in gesätem Reis bekannt ist,
   "Flurazole" (Benzyl-2-chlor-4-trifluormethyl-1,3-thiazol-5-carboxylat) (S13-3), das als Saatbeiz-Safener für Hirse gegen Schäden von Alachlor und Metolachlor bekannt ist,
   "CL 304415" (CAS-Reg.Nr. 31541-57-8)
   (4-Carboxy-3,4-dihydro-2H-1-benzopyran-4-essigsäure) (S13-4) der Firma American Cyanamid, das als Safener für Mais gegen Schäden von Imidazolinonen bekannt ist,
   "MG 191" (CAS-Reg.Nr. 96420-72-3) (2-Dichlormethyl-2-methyl-1,3-dioxolan) (S13-5) der Firma Nitrokemia, das als Safener für Mais bekannt ist,
   "MG-838" (CAS-Reg.Nr. 133993-74-5)
   (2-propenyl 1-oxa-4-azaspiro[4.5]decane-4-carbodithioate) (S13-6) der Firma Nitrokemia,
   "Disulfoton" (O,O-Diethyl S-2-ethylthioethyl phosphordithioat) (S13-7), "Dietholate" (O,O-Diethyl-O-phenylphosphorotioat) (S13-8),
   "Mephenate" (4-Chlorphenyl-methylcarbamat) (S13-9).
S14) Wirkstoffe, die neben einer herbiziden Wirkung gegen Schadpflanzen auch Safenerwirkung an Kulturpflanzen wie Reis aufweisen, wie z. B. "Dimepiperate" oder "MY-93" (S-1-Methyl-1-phenylethyl-piperidin-1-carbothioat), das als Safener für Reis gegen Schäden des Herbizids Molinate bekannt ist, "Daimuron" oder "SK 23" (1-(1-Methyl-1-phenylethyl)-3-p-tolyl-harnstoff), das als Safener für Reis gegen Schäden des Herbizids Imazosulfuron bekannt ist, "Cumyluron" = "JC-940" (3-(2-Chlorphenylmethyl)-1-(1-methyl-1-phenylethyl)harnstoff, siehe JP-A-60087254), das als Safener für Reis gegen Schäden einiger Herbizide bekannt ist,
   "Methoxyphenon" oder "NK 049" (3,3'-Dimethyl-4-methoxy-benzophenon), das als Safener für Reis gegen Schäden einiger Herbizide bekannt ist,
   "CSB" (1-Brom-4-(chlormethylsulfonyl)benzol) von Kumiai, (CAS-Reg.Nr. 54091-06-4), das als Safener gegen Schäden einiger Herbizide in Reis bekannt ist.
S15) Wirkstoffe, die vorrangig als Herbizide eingesetzt werden, jedoch auch Safenerwirkung auf Kulturpflanzen aufweisen, z.B.
   (2,4-Dichlorphenoxy)essigsäure (2,4-D),
   (4-Chlorphenoxy)essigsäure,
   (R,S)-2-(4-Chlor-o-tolyloxy)propionsäure (Mecoprop),
   4-(2,4-Dichlorphenoxy)buttersäure (2,4-DB),
   (4-Chlor-o-tolyloxy)essigsäure (MCPA),
   4-(4-Chlor-o-tolyloxy)buttersäure,
   4-(4-Chlorphenoxy)buttersäure,
   3,6-Dichlor-2-methoxybenzoesäure (Dicamba),
   1-(Ethoxycarbonyl)ethyl-3,6-dichlor-2-methoxybenzoat (Lactidichlor-ethyl).

Einige der Safener sind bereits als Herbizide bekannt und entfalten somit neben der Herbizidwirkung bei Schadpflanzen zugleich auch Schutzwirkung bei den Kulturpflanzen.

Die Gewichtsverhältnisse von Herbizid(mischung) zu Safener hängt im Allgemeinen von der Aufwandmenge an Herbizid und der Wirksamkeit des jeweiligen Safeners ab und kann innerhalb weiter Grenzen variieren, beispielsweise im Bereich von 200:1 bis 1:200, vorzugsweise 100:1 bis 1:100, insbesondere 20:1 bis 1:20. Die Safener können analog den Verbindungen der Formel (I) oder deren Mischungen mit weiteren Herbiziden/Pestiziden formuliert werden und als Fertigformulierung oder Tankmischung mit den Herbiziden bereitgestellt und angewendet werden.

Zur Anwendung werden die in handelsüblicher Form vorliegenden Formulierungen gegebenenfalls in üblicher Weise verdünnt z. B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der allgemeinen Formel (I). Sie kann innerhalb weiter Grenzen schwanken, z. B. zwischen 0,001 und 10,0 kg/ha oder mehr Aktivsubstanz, vorzugsweise liegt sie jedoch zwischen 0,005 und 5 kg/ha.

Die vorliegende Erfindung wird ahand der nachfolgenden Beispiele näher erläutert, welche die Erfindung jedoch keinesfalls beschränken.

### A. Synthesebeispiele

5-[(6-ethyl-3,4-dihydro-2H-chromen-4-yl)amino]pyrimidine-2-carbonitrile (Bsp.: 64): 0,20 g (1,09 mmol) 5-Bromo-2-cyanopyrimidine (ABCR 245244), 0,325 g (1,52 mmol) 6-ethyl-3,4-dihydro-2H-chromen-4-aminium chloride und 0,35 g (3,3 mmol) Triethylamin in 1,5 ml N,N-Dimethylacetamid werden in einer geschlossenen Küvette in der Mikrowelle 120 Minuten auf 200 °C (Biotage Initiator, http://www.biotage.com/DynPage.aspx?id= 22001) erhitzt. Das so erhaltene Rohgemisch wird auf Kieselgel aufgezogen und säulenchromatographisch mit Heptan/Essigester als Laufmittel gereinigt. Nach Einengen werden 0.03 g 5-[(6-ethyl-3,4-dihydro-2H-chromen-4-yl)amino]pyrimidine-2-carbonitrile (wachsartig) erhalten (Ausbeute 8 % bei 80 % Reinheit).

N-(3,4-dihydro-2H-chromen-4-yl)-2-(methylsulfonyl)pyrimidin-5-amine (Bsp.: 92): 0.25 g (1.29 mmol) 5-chloro-2-(methylsulfonyl)pyrimidine, 0.27 g (1.42 mmol) 3,4-dihydro-2H-chromen-4-amine hydrochlorid und 0.42 g (3.89 mmol) Triethylamin in 1.5 ml N,N-Dimethylacetamid werden in einer geschlossenen Küvette in der Mikrowelle 125 Minuten auf 180 °C (Biotage Initiator, http://www.biotage.com/DynPage.aspx?id= 22001) erhitzt. Das so erhaltene Rohgemisch wird auf Kieselgel aufgezogen und säulenchromatographisch mit Heptan/Essigester als Laufmittel gereinigt. Nach Einengen werden zunächst 0.08 g 5-chloro-2-{[(1R,2S)-2,6-dimethyl-2,3-dihydro-1H-inden-1-yl]amino}pyrimidine-4-carboxamide (wachsartig) und dann 0.09 g N-(3,4-dihydro-2H-chromen-4-yl)-2-(methylsulfonyl)pyrimidin-5-amine erhalten (wachsartig, Ausbeute 19 % bei 85 % Reinheit).

5-{[(1R,2S)-2,6-dimethyl-2,3-dihydro-1H-inden-1-yl]aminol-2-(methylsulfonyl)pyrimidine-4-carboxamide (Bsp.: 93):
Zu 40.8 g (25 ml, 0.34 mol) Thionychlorid und 0.47 g (0.5 ml, 6.5 mmol) N,N-Dimethylformamid gibt man unter Rühren portionsweise 15 g (5-chloro-2-(methylsulfanyl)pyrimidine-4-carboxylic acid und erhitzt die Reaktionsmischung anschließend solange zum Rückfluß bis keine deutliche Gasentwickung feststellbar ist. Die Mischung wird in etwas Methylenchlorid aufgenommen. Nach Einengen erhält man 15 g 5-chloro-2-(methylsulfanyl)pyrimidine-4-carbonyl chloride, das ohne weitere Reinigung in die Folgestufe eingesetzt wurde.

15 g (67.2 mmol) 5-chloro-2-(methylsulfanyl)pyrimidine-4-carbonyl chloride werden in 35 ml Dioxan gelöst und auf 5 ° C gekühlt. Zu der gekühlten Lösung werden 57,3 g (0.4 mol) 7n Ammoniak in Methanol (ca 12 %ige Lösung) langsam zugetropft. Die Reaktionstemperatur wurde dabei unter 15 °C gehalten. Nach beendetem Zutropfen wurde die Reaktionsmischung auf Raumtermperatur kommen gelassen und ca 15 Stunden gerührt. Dann wurde das Gemisch auf Wasser gegeben und das ausgefallene 5-chloro-2-(methylsulfanyl)pyrimidine-4-carboxamide isoliert (7.7 g, Ausbeute 53 % bei 95 % Reinheit).

7.7 g (37.8 mmo) 5-chloro-2-(methylsulfanyl)pyrimidine-4-carboxamide und 37.2 g (60.4 mmol) Kaliumperoxomonosulfat werden in einem Gemsich aus 30 ml Wasser, 30 ml Essigsäure und 30 ml Methanol suspendiert und ca 3 Stunden gerührt. Die Mischung wird dann in ca 100 ml Wasser aufgenommen und mit Essigester extrahiert. Nach Trocknen und Einengen des Essigesterextrakts wurden 1.5 g 5-chloro-2-(methylsulfonyl)pyrimidine-4-carboxamide (Schmelzpunkt 205 °C, 17 % Ausbeute bei 95 % Reinheit) erhalten. Aus der Mutterlauge konnte weiteres 5-chloro-2-(methylsulfonyl)pyrimidine-4-carboxamide isoliert werden.

0.30 g (1.27 mmol) 5-chloro-2-(methylsulfonyl)pyrimidine-4-carboxamide, 0.25 g (1.53 mmol) (1R,2S)-2,6-dimethyl-2,3-dihydro-1 H-inden-1-amine und 0.28 g (2.54 mmol) Triethylamin in 1,5 ml N,N-Dimethylacetamid werden in einer geschlossenen Küvette in der Mikrowelle 45 Minuten auf 120 °C (Biotage Initiator, http://www.biotage.com/DynPage.aspx?id= 22001) erhitzt. Das so erhaltene Rohgemisch wird auf Kieselgel aufgezogen und säulenchromatographisch mit Heptan/Essigester als Laufmittel gereinigt. Nach Einengen werden zunächst 0.07 g 5-chloro-2-{[(1R,2S)-2,6-dimethyl-2,3-dihydro-1H-inden-1-yl]amino}pyrimidine-4-carboxamide (wachsartig) und dann 0.252 g 5-{[(1R,2S)-2,6-dimethyl-2,3-dihydro-1H-inden-1-yl]amino}-2-(methylsulfonyl)pyrimidine-4-carboxamide erhalten (Ausbeute 52 % bei 95 % Reinheit).

N-[(1R)-2,3-dihydro-1H-inden-1-yl]-2-(trifluoromethyl)pyrimidin-5-amine (Bsp.: 115): 0.20 g (1.20 mmol) 5-fluoro-2-(trifluoromethyl)pyrimidine, 0.225 g (1.69 mmol) (1R)-2,3-dihydro-1H-inden-1-amine und 0.50 g (2,41 mmol) Dicyclohexylmethylamin in 1,5 ml 1-Methyl-2-pyrrolidon werden in einer geschlossenen Küvette in der Mikrowelle 120 Minuten auf 180 °C (Biotage Initiator, http://www.biotage.com/DynPage.aspx?id= 22001) erhitzt. Das so erhaltene Rohgemisch wird auf Kieselgel aufgezogen und säulenchromatographisch mit Heptan/Essigester als Laufmittel gereinigt. Nach Einengen werden 0.34 g N-[(1R)-2,3-dihydro-1H-inden-1-yl]-2-(trifluoromethyl)pyrimidin-5-amine (Schmelzpunkt 123.7 °C, Ausbeute 96 % bei 95 % Reinheit) erhalten.

2-(methylsulfonyl)-N-[(1R)-1,2,3,4-tetrahydronaphthalen-1-yl]pyrimidin-5-amine (Bsp.: 273):
0.20 g (0.84 mmol) 5-chloro-2-(methylsulfonyl)pyrimidine-4-carbonsäure, 0.15 g (1.01 mmol) (1 R)-1,2,3,4-tetrahydronaphthalen-1-amine und 0.33 g (1.69 mmol) Dicyclohexylmethylamin in 1.5 ml N-Methylpyrrolidone werden in einer geschlossenen Küvette in der Mikrowelle 30 Minuten auf 150 °C (Biotage Initiator, http://www.biotage.com/DynPage.aspx?id= 22001) erhitzt. Das so erhaltene Rohgemisch wird auf Kieselgel aufgezogen und säulenchromatographisch mit Heptan/Essigester als Laufmittel gereinigt. Nach Einengen werden zunächst 0.024 g 5-chloro-N-[(1R)-1,2,3,4-tetrahydronaphthalen-1-yl]pyrimidin-2-amine (fest) und dann 0.08 g 2-(methylsulfonyl)-N-[(1R)-1,2,3,4-tetrahydronaphthalen-1-yl]pyrimidin-5-amine erhalten (wachsartig, Ausbeute 31 % bei 95 % Reinheit).

**Chemisch Physikalische Charakterisierung ausgewählter Synthesebeispiele:**

| Verbindung | Beschreibung |
|---|---|
| 62 | wachsartig; logp (HCOOH): 4.06; 1H-NMR (CDCl₃, 400 MHZ, δ in ppm): 2.10-2.35 (m, 2H, 2H von CH₂); 2.15 (s, 3H, CH₃); 2.20 (s, 3H, CH₃); 4.15-4.35 (m, 2H, 2H von CH₂O); 5.05 (q, 1H, CH); 5.80 (br, 1H, NH); 6.90 (s und s, 2H, Ar-H); 8.15 (br, 2H, Pyr-H); |
| 64 | wachsartig; logp (HCOOH): 2.80; 1H-NMR (CDCl₃, 400 MHZ, δ in ppm): 1.20 (t, 3H, CH₃); 2.10 (m, 1H, 1H von CH₂); 2.30 (m, 1H, 1H von CH₂); 2.55 (t, 2H, CH₂); 4.15 (m, 1H, 1H von CH₂O); 4.30 (m, 1H, 1H von CH₂O); 5.7 (br, 1H, CH); 6.80-7.10 (m, 3H, Ar-H); 8.20 (s, 2H, Pyr-H); |
| 65 | wachsartig; logp (HCOOH): 2.54; 1H-NMR (CDCl₃, 400 MHZ, δ in ppm): 1.20 (t, 3H, CH₃); 2.10 (m, 1H, 1H von CH₂); 2.25 (m, 1H, 1H von CH₂); 2.55 (t, 2H, CH₂); 3.10 (s, 3H, SO₂CH₃); 4.15 (m, 1H, 1H von CH₂O); 4.30 (m, 1H, 1H von CH₂O); ); 5.25 (q, 1H, CH); 6.30 (br, 1H, NH); 6.80 (d, 1H, Ar-H); 7.05 (s, 1H, Ar-H); 7.10 (d, 1H, Ar-H); 8.55 und 8.80 (s, 2H, Pyr-H); |
| 66 | wachsartig; logp (HCOOH): 2.54; 1H-NMR (CDCl₃, 400 MHZ, δ in ppm): 1.15 (t, 3H, CH₃); 2.10 (m, 1H, 1H von CH₂); 2.25 (m, 1H, 1H von CH₂); 2.55 (t, 2H, CH₂); 3.25 (s, 3H, SO₂CH₃); 4.15 (m, 1H, 1H von CH₂O); 4.25 (m, 1H, 1H von CH₂O); ); 5.25 (q, 1H, CH); 6.40 (br, 1H, NH); 6.80 (d, 1H, Ar-H); 7.00 (s, 1H, Ar-H); 7.10 (d, 1H, Ar-H); 8.80 und 8.90 (s, 2H, Pyr-H); |
| 70 | wachsartig; logp (HCOOH): 3.41; 1H-NMR (CDCl₃, 400 MHZ, δ in ppm): 1.15 (t, 3H, CH₃); 2.05 (m, 1H, 1H von CH₂); 2.20 (m, 1H, 1H von CH₂); 2.55 (t, 2H, CH₂); 4.15 (m, 1H, 1H von CH₂O); 4.25 (m, 1H, 1H von CH₂O); ); 4.50 (br, 1H, NH); 4.70 (q, 1 H, CH); 6.80 (d, 1H, Ar-H); 7.05 (s, 1H, Ar-H); 7.10 (d, 1H, Ar-H); 8.25 (s, 2H, Pyr-H); |
| 76 | wachsartig; logp (HCOOH): 3.17; 1H-NMR (CDCl₃, 400 MHZ, δ in ppm): 1.25 (d, 3H, CH₃); 2.30 (s, 3H, CH₃); 2.35(jm, 1H, 1H von CH₂); 2.55 (m, 1H, 1H von CH₂); 3.15 (q, 1H, 1H von CHCH₃); 4.50 (br, 1H, NH); 4.55 (q, 1H, CH); 7.00 (s, 1H, Ar-H); 7.10 (d, 1H, Ar-H); 7.15 (d, 1H, Ar-H); 8.20 (s, 2H, Pyr-H); |
| 83 | fest; 1H-NMR (CDCl₃, 400 MHZ, δ in ppm): 1.85 (m, 2H, 2H von CH₂); 2.00 (m, 2H, 2H von CH₂); 2.80 (m, 2H, 2H von CH₂); 4.50 (d, 1H, NH); 4.70 (q, 1H, CH); 7.15-7.25 (m, 4H, Ar-H); 8.15 (s, 2H, Pyr-H); |
| 87 | wachsartig; logp (HCOOH): 1.86; 1H-NMR (CDCl₃, 400 MHZ, δ in ppm): 1.85 (m, 2H, 2H von CH₂); 2.00 (m, 2H, 2H von CH₂); 2.80 (m, 2H, 2H von CH₂); ); 3.50 (s, 3H, SO₂CH₃); 5.40 (br, 1H, CH); 5.8 (br, 1H, NH); 6.9 (br, 1H, NH₂); 7.15 - 7.25 (m, 4H, Ar-H); 8.90 und 9.00 (2s, 2H, Pyr-H); |
| 92 | Wachsartig; 1H-NMR (CDCl₃, 400 MHZ, δ in ppm): 2.10 (m, 1H, 1H von CH₂); 2.25 (m, 1H, 1H von CH₂); 3.05 (s, 3H, SO₂CH₃); 4.20 (m, 1H, 1H von CH₂O); 4.30 (m, 1H, 1H von CH₂O); ); 5.35 (q, 1H, CH); 6.30 (br, 1H, NH); 6.90 (m, 2H, Ar-H); 7.25 (m, 2H, Ar-H); 8.55 und 8.80 (s, 2H, Pyr-H); |
| 93 | wachsartig; logp (HCOOH): 2.21; 1H-NMR (CDCl₃, 400 MHZ, δ in ppm): 1.25 (d, 3H, CH₃); 2.30 (s, 3H, CH₃); 2.35 (jm, 1H, 1H von CH₂); 2.55 (m, 1H, 1H von CH₂); 3.10 (q, 1H, 1H von CHCH₃); 3.50 (s, 3H, SO₂CH₃); 5.20 und 5.40 (jeweils t, 1H, CH); 5.90 und 6.20 (jeweils d, 1H, NH);); 6.85 (br, 2H, NH₂); 7.00-7.15 (m, 3H, Ar-H); 8.90 und 9.00 (jeweils s, 1H, Pyr-H); |
| 108 | fest; logp (HCOOH): 3.29; 1H-NMR (CDCl₃, 400 MHZ, δ in ppm): 1.25 (d, 3H, CH₃); 2.35 (s, 3H, CH₃); 2.35 (m, 1H, 1H von CH₂); 2.60 (m, 1H, 1H von CH₂); 3.10 (q, 1H, 1H von CHCH₃); 3.30 (s, 3H, SO₂CH₃); 5.20 (m, 1H, CH); 6.05 (br, 1H, NH); 6.95-7.15 (m, 3H, Ar-H); 8.85 und 9.00 (jeweils s, 1H, Pyr-H); |
| 109 | fest; logp (HCOOH): 2.97; 1H-NMR (CDCl₃, 400 MHZ, δ in ppm): 1.90 (m, 3H, 1H von CH₂, 2H von CH₂); 2.15 (m, 1H, 1H von CH₂); 2.85 (m, 2H, 2H von CH₂); 3.30 (s, 3H, SO₂CH₃); 5.35 (m, 1H, CH); 6.20 (br, 1H, NH); 7.10 -7.25 (m, 4H, Ar-H); 8.80 und 9.00 (jeweils s, 1 H, Pyr-H); |
| 111 | fest; 1H-NMR (CDCl₃, 400 MHZ, δ in ppm): 1.95 (m, 1H, 1H von CH₂,); 2.30 (s, 3H, CH₃); 2.65 (m, 1H, 1H von CH₂); 2.95 (m, 1H, 1H von CH₂,); 3.05 (m, 1H, 1H von CH₂); 4.40 (d, 1H, NH); 5.05 (q, 1H, CH); 7.10 -7.2 (m, 3H, Ar-H); 8.25 (s, 2H, Pyr-H); |
| 115 | fest; Smp.: 123 - 124°C; logp (HCOOH): 3.05; 1H-NMR (CDCl₃, 400 MHZ, δ in ppm): 1.95 (m, 1H, 1H von CH₂,); 2.65 (m, 1H, 1H von CH₂); 2.90 (m, 1H, 1H von CH₂,); 3.00 (m, 1H, 1H von CH₂); 4.40 (d, 1H, NH); 5.05 (q, 1H, CH); 7.10 -7.20 (m, 4H, Ar-H); 8.20 (s, 2H, Pyr-H); |
| 116 | fest; Smp.: 83 - 84 °C; logp (HCOOH): 3.79; 1H-NMR (CDCl₃, 400 MHZ, δ in ppm): 1.25 (d, 3H, CH₃); 2.30 (s, 3H, CH₃); 2.35 (m, 1H, 1H von CH₂); 2.60 (m, 1H, 1H von CH₂); 3.10 (q, 1H, 1H von CHCH₃); 4.35 (d, 1H, NH); 4.55 (q, 1H, CH); 7.05 (s, 1H, Ar-H); 7.15 (dd, 2H, Ar-H); 8.25 (s, 2H, Pyr-H); |
| 117 | fest; Smp.: 152 - 153 °C; logp (HCOOH): 3.45; 1H-NMR (CDCl₃, 400 MHZ, δ in ppm): 1.85 (m, 2H, 2H von CH₂); 2.05 (m, 2H, 2H von CH₂); 2.85 (m, 2H, 2H von CH₂); 4.40 (d, 1H, NH); 4.70 (q, 1H, CH); 7.10 - 7.30 (m, 4H, Ar-H); 8.20 (s, 2H, Pyr-H); |
| 131 | fest; logp (HCOOH): 2.13; 1H-NMR (CDCl₃, 400 MHZ, δ in ppm): 1.95 (m, 1H, 1H von CH₂,); 2.70 (m, 1H, 1H von CH₂); 2.90 (m, 1H, 1H von CH₂,); 3.05 (m, 1H, 1H von CH₂); 3.10 (s, 3H, SO₂CH₃); 5.65 (q, 1H, CH); 6.30 (d, 1H, NH); 7.15 -7.30 (m, 4H, Ar-H); 8.50 und 8.80 (jeweils s, 1H, Pyr-H); |
| 134 | fest; Smp.: 182 - 183 °C; logp (HCOOH): 2.83; 1H-NMR (CDCl₃, 400 MHZ, δ in ppm): 1.25 (d, 3H, CH₃); 2.30 (s, 3H, CH₃); 2.35 (m, 1H, 1H von CH₂); 2.50 (m, 1H, 1H von CH₂); 3.05 (q, 1H, 1H von CHCH₃); 3.10 (s, 3H, SO₂CH₃); 5.35 (q, 1H, CH); 5.90 (d, 1H, NH); 7.00 (s, 1H, Ar-H); 7.10 (dd, 2H, Ar-H); 8.65 und 8.80 (jeweils s, 1H, Pyr-H); |

### B. Formulierungsbeispiele

a) Ein Stäubemittel wird erhalten, indem man 10 Gew.-Teile einer Verbindung der Formel (I) und/oder deren Salze und 90 Gew.-Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung der Formel (I) und/oder deren Salze, 64 Gew.-Teile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gew.-Teile einer Verbindung der Formel (I) und/oder deren Salze mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z.B. ca. 255 bis über 277 C) mischt und in einer Reibkugelmühle auf eine Feinheit von unter 5 Mikron vermahlt.
d) Ein emulgierbares Konzentrat wird erhalten aus 15 Gew.-Teilen einer Verbindung der Formel (I) und/oder deren Salze, 75 Gew.-Teilen Cyclohexanon als Lösungsmittel und 10 Gew.-Teilen oxethyliertes Nonylphenol als Emulgator.
e) Ein in Wasser dispergierbares Granulat wird erhalten indem man
   75 Gew.-Teile einer Verbindung der Formel (I) und/oder deren Salze,
   10 Gew.-Teile ligninsulfonsaures Calcium,
   5 Gew.-Teile Natriumlaurylsulfat,
   3 Gew.-Teile Polyvinylalkohol und
   7 Gew.-Teile Kaolin
   mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man 25 Gew.-Teile einer Verbindung der Formel (I) und/oder deren Salze,
   5 Gew.-Teile 2,2'-dinaphthylmethan-6,6'-disulfonsaures Natrium
   2 Gew.-Teile oleoylmethyltaurinsaures Natrium,
   1 Gew.-Teil Polyvinylalkohol,
   17 Gew.-Teile Calciumcarbonat und
   50 Gew.-Teile Wasser
   auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### Versuchsbeschreibung

### 1. Herbizide Wirkung bzw. Kulturpflanzenverträglichkeit im Vorauflauf

Samen von mono- bzw. dikotylen Unkraut- bzw. Kulturpflanzen werden in Holzfasertöpfen in sandiger Lehmerde ausgelegt und mit Erde abgedeckt. Die in Form von benetzbaren Pulvern (WP) oder als Emulsionskonzentrate (EC) formulierten erfindungsgemäßen Verbindungen werden dann als wässrige Suspension bzw. Emulsion mit einer Wasseraufwandmenge von umgerechnet 600 bis 800 I/ha unter Zusatz von 0,2% Netzmittel auf die Oberfläche der Abdeckerde appliziert.

Nach der Behandlung werden die Töpfe im Gewächshaus aufgestellt und unter guten Wachstumsbedingungen für die Testpflanzen gehalten. Die visuelle Bonitur der Schäden an den Versuchspflanzen erfolgt nach einer Versuchszeit von 3 Wochen im Vergleich zu unbehandelten Kontrollen (herbizide Wirkung in Prozent (%): 100% Wirkung = Pflanzen sind abgestorben, 0 % Wirkung = wie Kontrollpflanzen).

In den nachfolgenden Tabellen werden folgende Abkürzungen verwendet:

| | | | |
|---|---|---|---|
| ABUTH: | Abutilon theophrasti | ALOMY: | Alopecurus myosuroides |
| AMARE: | Amaranthus retroflexus | ECHCG: | Echinochloa crus-galli |
| LOLMU: | Lolium multiflorum | MATIN: | Matricaria inodora |
| POLCO: | Polygonum convolvulus | SETVI: | Setaria viridis |
| STEME: | Stellaria media | VERPE: | Veronica persica |
| VIOTR: | Viola tricolor | | |

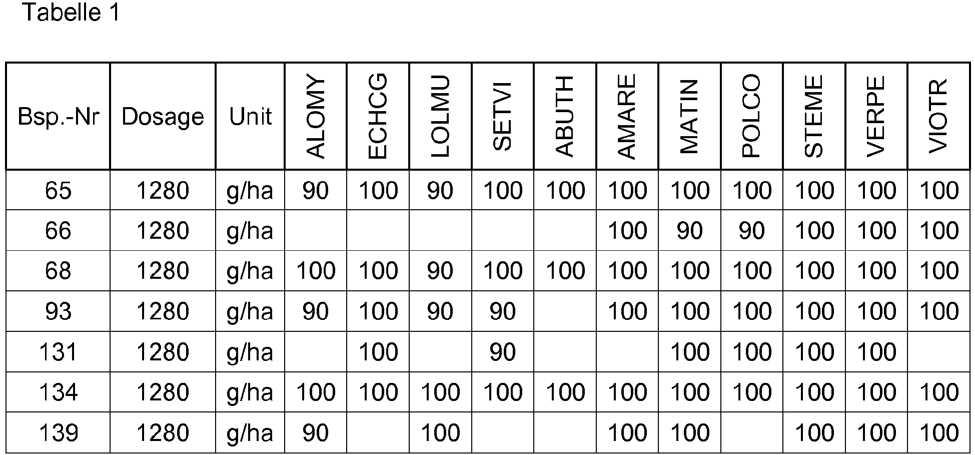

Wie die Ergebnisse zeigen, weisen erfindungsgemäße Verbindungen eine gute herbizide Vorauflaufwirksamkeit gegen ein breites Spektrum von Ungräsern und Unkräutern auf. Beispielsweise haben die Verbindungen aus Tabelle 1 sehr gute herbizide Wirkung gegen Schadpflanzen wie Avena fatua, Stellaria media, Echinochloa crus-galli, Lolium multiflorum, Setaria viridis, Abutilon theophrasti, Amaranthus retroflexus und Alopecurus myosuroides im Vorauflaufverfahren bei einer Aufwandmenge von 1,28 kg und weniger Aktivsubstanz pro Hektar. Die erfindungsgemäßen Verbindungen eignen sich deshalb im Vorauflaufverfahren zur Bekämpfung von unerwünschtem Pflanzenwuchs.

### 2. Herbizide Wirkung bzw. Kulturpflanzenverträglichkeit im Nachauflauf

Samen von mono- bzw. dikotylen Unkraut- bzw. Kulturpflanzen werden in Holzfasertöpfen in sandigem Lehmboden ausgelegt, mit Erde abgedeckt und im Gewächshaus unter guten Wachstumsbedingungen angezogen. 2 bis 3 Wochen nach der Aussaat werden die Versuchspflanzen im Einblattstadium behandelt. Die in Form von benetzbaren Pulvern (WP) oder als Emulsionskonzentrate (EC) formulierten erfindungsgemäßen Verbindungen werden dann als wässrige Suspension bzw. Emulsion mit einer Wasseraufwandmenge von umgerechnet 600 bis 800 I/ha unter Zusatz von 0,2% Netzmittel auf die grünen Pflanzenteile gesprüht. Nach ca. 3 Wochen Standzeit der Versuchspflanzen im Gewächshaus unter optimalen Wachstumsbedingungen wird die Wirkung der Präparate visuell im Vergleich zu unbehandelten Kontrollen bonitiert (herbizide Wirkung in Prozent (%): 100% Wirkung = Pflanzen sind abgestorben, 0 % Wirkung = wie Kontrollpflanzen).

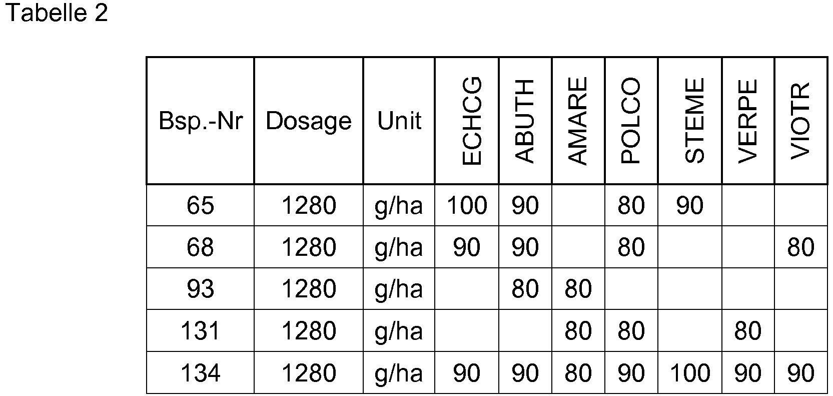

Wie die Ergebnisse zeigen, weisen erfindungsgemäße Verbindungen eine gute herbizide Nachauflaufwirksamkeit gegen ein breites Spektrum von Ungräsern und Unkräutern auf. Beispielsweise haben die Verbindungen aus Tabelle 2 sehr gute herbizide Wirkung gegen Schadpflanzen wie Avena fatua, Stellaria media, Echinochloa crus-galli, Lolium multiflorum, Setaria viridis, Abutilon theophrasti, Amaranthus retroflexus und Alopecurus myosuroides im Nachauflaufverfahren bei einer Aufwandmenge von 1,28 kg und weniger Aktivsubstanz pro Hektar. Die erfindungsgemäßen Verbindungen eignen sich deshalb im Nachauflaufverfahren zur Bekämpfung von unerwünschtem Pflanzenwuchs.

## Patentansprüche

1. Verbindungen der allgemeinen Formel (I) und deren agrochemisch verträgliche Salze 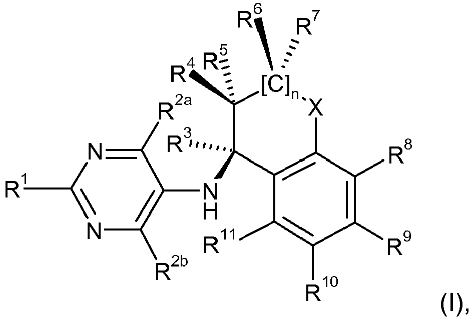 worin
R¹ ausgewählt ist aus der Gruppe, bestehend aus
- Cyano, Nitro, C(O)OH, C(O)NH₂;
- (C₁-C₆)-Alkyl, (C₁-C₆)-Halogenalkyl, (C₁-C₆)-Alkylcarbonyl, (C₁-C₆)-Haloalkylcarbonyl, (C₁-C₆)-Alkylcarbonyloxy, (C₁-C₆)-Halogenalkylcarbonyloxy, (C₁-C₆)-Alkylcarbonyl-(C₁-C₄)-alkyl;
- (C₁-C₆)-Alkoxy, (C₁-C₆)-Halogenalkoxy, (C₁-C₆)-Alkoxycarbonyl, (C₁-C₆)-Haloalkoxycarbonyl, (C₁-C₆)-Alkoxycarbonyl-(C₁-C₆)-alkyl, (C₁-C₆)-Haloalkoxycarbonyl-(C₁-C₆)-alkyl, (C₁-C₆)-Alkoxycarbonyl-(C₁-C₆)-halogenalkyl, (C₁-C₆)-Halogenalkoxycarbonyl-(C₁-C₆)-halogenalkyl;
- (C₂-C₆)-Alkenyl, (C₂-C₆)-Halogenalkenyl, (C₂-C₆)-Alkenylcarbonyl, (C₂-C₆)-Haloalkenylcarbonyl, (C₂-C₆)-Alkenyloxy, (C₂-C₆)-Haloalkenyloxy, (C₂-C₆)-Alkenyloxycarbonyl, (C₂-C₆)-Haloalkenyloxycarbonyl;
- (C₂-C₆)-Alkinyl, (C₂-C₆)-Halogenalkinyl, (C₂-C₆)-Alkinylcarbonyl, (C₂-C₆)-Haloalkinylcarbonyl, (C₂-C₆)-Alkinyloxy, (C₂-C₆)-Haloalkinyloxy, (C₂-C₆)-Alkinyloxycarbonyl, (C₂-C₆)-Halogenalklnyloxycarbonyl;
- Tri-(C₁-C₆)-alkylsilyl-(C₂-C₆)-alkinyl, Di-(C₁-C₆)-alkylsilyl-(C₂-C₆)-alkinyl, Mono-(C₁-C₆)-alkylsilyl-(C₂-C₆)-alkinyl; Phenylsilyl-(C₂-C₆)-alkinyl;
- (C₆-C₁₄)-Aryl, (C₆-C₁₄)-Aryloxy, (C₆-C₁₄)-Arylcarbonyl und (C₆-C₁₄)-Aryloxycarbonyl, welche jeweils am Arylteil mit Halogen, (C₁-C₆)-Alkyl und/oder (C₁-C₆)-Haloalkyl substituiert sein können;
- (C₆-C₁₄)-Aryl-(C₁-C₆)-alkyl, (C₆-C₁₄)-Aryl-(C₁-C₆)-alkoxy, (C₆-C₁₄)-Aryl-(C₁-C₆)-alkyl-carbonyl, (C₆-C₁₄)-Aryl-(C₁-C₆)-alkyl-carbonyloxy, (C₆-C₁₄)-Aryl-(C₁-C₆)-alkoxycarbonyl, (C₆-C₁₄)-Aryl-(C₁-C₆)-alkoxycarbonyloxy;
- Aminocarbonyl-(C₁-C₆)-alkyl, Di-(C₁-C₆)-Alkylaminocarbonyl-(C₁-C₆)-alkyl;
- Mono-((C₁-C₆)-alkyl)-amino-carbonyl, Mono-((C₁-C₆)-haloalkyl)-aminocarbonyl, Di-((C₁-C₆)-alkyl)-amino-carbonyl, Di-((C₁-C₆)-haloalkyl)-aminocarbonyl, ((C₁-C₆)-Alkyl-(C₁-C₆)-haloalkyl)-amino-carbonyl, N-((C₁-C₆)-Alkanoyl)-amino-carbonyl, N-((C₁-C₆)-Haloalkanoyl)-amino-carbonyl, Mono-((C₆-C₁₄)-aryl)-amino-carbonyl, Di-((C₆-C₁₄)-aryl)-amino-carbonyl;
- (C₁-C₆)-Alkoxy-(C₁-C₆)-alkyl, (C₁-C₆)-Alkoxy-(C₁-C₆)-alkoxy, (C₁-C₆)-Alkoxycarbonyl-(C₁-C₆)-alkoxy;
- (C₃-C₈)-Cycloalkyl, welches gegebenenfalls am Cycloalkylrest durch (C₁-C₆)-Alkyl und/oder Halogen substituiert sein kann; (C₃-C₈)-Cycloalkoxy, (C₃-C₈)-Cycloalkyl-(C₁-C₆)-alkyl, (C₃-C₈)-Cycloalkyl-(C₁-C₆)-haloalkyl, (C₃-C₈)-Cycloalkyl-(C₁-C₆)-alkoxy, (C₃-C₈)-Cycloalkyl-(C₁-C₆)-haloalkoxy, (C₃-C₈)-Cycloalkylcarbonyl, (C₃-C₈)-Cycloalkoxycarbonyl, (C₃-C₈)-Cycloalkyl-(C₁-C₆)-alkylcarbonyl, (C₃-C₈)-Cycloalkyl-(C₁-C₆)-haloalkylcarbonyl, (C₃-C₈)-Cycloalkyl-(C₁-C₆)-alkoxycarbonyl, (C₃-C₈)-Cycloalkyl-(C₁-C₆)-haloalkoxycarbonyl, (C₃-C₈)-Cycloalkylcarbonyloxy, (C₃-C₈)-Cycloalkoxycarbonyloxy, (C₃-C₈)-Cycloalkyl-(C₁-C₆)-alkylcarbonyloxy, (C₃-C₈)-Cycloalkyl-(C₁-C₆)-halogenalkylcarbonyloxy, (C₃-C₈)-Cycloalkyl-(C₁-C₆)-alkoxycarbonyloxy, (C₃-C₈)-Cycloalkyl-(C₁-C₆)-haloalkoxycarbonyloxy;
- (C₃-C₈)-Cycloalkenyl, (C₃-C₈)-Cycloalkenyloxy, (C₃-C₈)-Cycloalkenyl-(C₁-C₆)-alkyl, (C₃-C₈)-Cycloalkenyl-(C₁-C₆)-halogenalkyl, (C₃-C₈)-Cycloalkenyl-(C₁-C₆)-alkoxy, (C₃-C₈)-Cycloalkenyl-(C₁-C₆)-halogenalkoxy, (C₃-C₈)-Cycloalkenylcarbonyl, (C₃-C₈)-Cycloalkenyloxycarbonyl, (C₃-C₈)-Cycloalkenyl-(C₁-C₆)-alkylcarbonyl, (C₃-C₈)-Cycloalkenyl-(C₁-C₆)-halogenalkylcarbonyl, (C₃-C₈)-Cycloalkenyl-(C₁-C₆)-alkoxycarbonyl, (C₃-C₈)-Cycloalkenyl-(C₁-C₆)-haloalkoxycarbonyl, (C₃-C₈)-Cycloalkenylcarbonyloxy, (C₃-C₈)-Cycloalkenyloxycarbonyloxy, (C₃-C₈)-Cycloalkenyl-(C₁-C₆)-alkylcarbonyloxy, (C₃-C₈)-Cycloalkenyl-(C₁-C₆)-halogenalkylcarbonyloxy, (C₃-C₈)-Cycloalkenyl-(C₁-C₆)-alkoxycarbonyloxy, (C₃-C₈)-Cycloalkenyl-(C₁-C₆)-haloalkoxycarbonyloxy;
- Hydroxy-(C₁-C₆)-alkyl, Hydroxy-(C₁-C₆)-alkoxy Cyano-(C₁-C₆)-alkoxy, Cyano-(C₁-C₆)-alkyl;
- (C₁-C₆)-Alkylsulfonyl, (C₁-C₆)-Alkylthio, (C₁-C₆)-Alkylsulfinyl, (C₁-C₆)-Haloalkylsulfonyl, (C₁-C₆)-Halogenalkylthio, (C₁-C₆)-Halogenalkylsulfinyl, (C₁-C₆)-Alkylsulfonyl-(C₁-C₆)-alkyl, (C₁-C₆)-Alkylthio-(C₁-C₆)-alkyl, (C₁-C₆)-Alkylsulfinyl-(C₁-C₆)-alkyl, (C₁-C₆)-Haloalkylsulfonyl-(C₁-C₆)-alkyl, (C₁-C₆)-Haloalkylthio-(C₁-C₆)-alkyl, (C₁-C₆)-Haloalkylsulfinyl-(C₁-C₆)-alkyl, (C₁-C₆)-Alkylsulfonyl-(C₁-C₆)-haloalkyl, (C₁-C₆)-Alkylthio-(C₁-C₆)-haloalkyl, (C₁-C₆)-Alkylsulfinyl-(C₁-C₆)-haloalkyl, (C₁-C₆)-Haloalkylsulfonyl-(C₁-C₆)-haloalkyl, (C₁-C₆)-Haloalkylthio-(C₁-C₆)-haloalkyl, (C₁-C₆)-Haloalkylsulfinyl-(C₁-C₆)-haloalkyl, (C₁-C₆)-Alkylsulfonyloxy, (C₁-C₆)-Halogenalkylsulfonyloxy, (C₁-C₆)-Alkylthiocarbonyl, (C₁-C₆)-Haloalkylthiocarbonyl, (C₁-C₆)-Alkylthiocarbonyloxy, (C₁-C₆)-Haloalkylthiocarbonyloxy, (C₁-C₆)-Alkylthio-(C₁-C₆)-alkyl, (C₁-C₆)-Alkylthio-(C₁-C₆)-alkoxy, (C₁-C₆)-Alkylthio-(C₁-C₆)-alkylcarbonyl, (C₁-C₆)-Alkylthio-(C₁-C₆)-alkylcarbonyloxy; (C₄-C₁₄)-Arylsulfonyl, (C₆-C₁₄)-Arylthio, (C₆-C₁₄)-Arylsulfinyl, (C₃-C₈)-Cycloalkylthio, (C₃-C₈)-Alkenylthio, (C₃-C₈)-Cycloalkenylthio, (C₃-C₆)-Alkinylthio; und
R^{2a} und R^{2b}, jeweils unabhängig voneinander, ausgewählt sind aus der Gruppe, bestehend aus
- Wasserstoff, Halogen, Hydroxy, Cyano, Nitro, C(O)OH, C(O)NH₂;
- (C₁-C₆)-Alkyl, (C₁-C₆)-Halogenalkyl, (C₁-C₆)-Alkylcarbonyl, (C₁-C₆)-Haloalkylcarbonyl, (C₁-C₆)-Alkylcarbonyloxy, (C₁-C₆)-Halogenalkylcarbonyloxy, (C₁-C₆)-Alkylcarbonyl-(C₁-C₄)-alkyl;
- (C₁-C₆)-Alkoxy, (C₁-C₆)-Halogenalkoxy, (C₁-C₆)-Alkoxycarbonyl, (C₁-C₆)-Haloalkoxycarbonyl, (C₁-C₆)-Alkoxycarbonyl-(C₁-C₆)-alkyl, (C₁-C₆)-Halo-alkoxycarbonyl-(C₁-C₆)-alkyl, (C₁-C₆)-Alkoxycarbonyl-(C₁-C₆)-halogenalkyl, (C₁-C₆)-Halogenalkoxycarbonyl-(C₁-C₆)-halogenalkyl;
- (C₂-C₆)-Alkenyl, (C₂-C₆)-Halogenalkenyl, (C₂-C₆)-Alkenylcarbonyl, (C₂-C₆)-Haloalkenylcarbonyl, (C₂-C₆)-Alkenyloxy, (C₂-C₆)-Haloalkenyloxy, (C₂-C₆)-Alkenyloxycarbonyl, (C₂-C₆)-Haloalkenyloxycarbonyl;
- (C₂-C₆)-Alkinyl, (C₂-C₆)-Halogenalkinyl, (C₂-C₆)-Alkinylcarbonyl, (C₂-C₆)-Haloalkinylcarbonyl, (C₂-C₆)-Alkinyloxy, (C₂-C₆)-Haloalkinyloxy, (C₂-C₆)-Alkinyloxycarbonyl, (C₂-C₆)-Halogenalkinyloxycarbonyl;
- Tri-(C₁-C₆)-alkylsilyl-(C₂-C₆)-alkinyl, Di-(C₁-C₆)-alkylsilyl-(C₂-C₆)-alkinyl, Mono-(C₁-C₆)-alkylsilyl-(C₂-C₆)-alkinyl; Phenylsilyl-(C₂-C₆)-alkinyl;
- (C₆-C₁₄)-Aryl, (C₆-C₁₄)-Aryloxy, (C₆-C₁₄)-Arylcarbonyl und (C₆-C₁₄)-Aryloxycarbonyl, welche jeweils am Arylteil mit Halogen, (C₁-C₆)-Alkyl und/oder (C₁-C₆)-Haloalkyl substituiert sein können;
- (C₆-C₁₄)-Aryl-(C₁-C₆)-alkyl, (C₆-C₁₄)-Aryl-(C₁-C₆)-alkoxy, (C₆-C₁₄)-Aryl-(C₁-C₆)-alkyl-carbonyl, (C₆-C₁₄)-Aryl-(C₁-C₆)-alkyl-carbonyloxy, (C₆-C₁₄)-Aryl-(C₁-C₆)-alkoxycarbonyl, (C₆-C₁₄)-Aryl-(C₁-C₆)-alkoxycarbonyloxy;
- N-((C₁-C₆)-Haloalkanoyl)-amino, Aminocarbonyl-(C₁-C₆)-alkyl, Di-(C₁-C₆)-Alkylaminocarbonyl-(C₁-C₆)-alkyl;
- N-((C₁-C₆)-Haloalkanoyl)-amino-carbonyl, Mono-((C₆-C₁₄)-aryl)-aminocarbonyl, Di-((C₆-C₁₄)-aryl)-amino-carbonyl;
- (C₁-C₆)-Alkoxy-(C₁-C₆)-alkyl, (C₁-C₆)-Alkoxy-(C₁-C₆)-alkoxy, (C₁-C₆)-Alkoxycarbonyl-(C₁-C₆)-alkoxy;
- (C₃-C₈)-Cycloalkyl, welches gegebenenfalls am Cycloalkylrest durch (C₁-C₆)-Alkyl und/oder Halogen substituiert sein kann; (C₃-C₈)-Cycloalkoxy, (C₃-C₈)-Cycloalkyl-(C₁-C₆)-alkyl, (C₃-C₈)-Cycloalkyl-(C₁-C₆)-haloalkyl, (C₃-C₈)-Cycloalkyl-(C₁-C₆)-alkoxy, (C₃-C₈)-Cycloalkyl-(C₁-C₆)-haloalkoxy, (C₃-C₈)-Cycloalkylcarbonyl, (C₃-C₈)-Cycloalkoxycarbonyl, (C₃-C₈)-Cycloalkyl-(C₁-C₆)-alkylcarbonyl, (C₃-C₈)-Cycloalkyl-(C₁-C₆)-haloalkylcarbonyl, (C₃-C₈)-Cycloalkyl-(C₁-C₆)-alkoxycarbonyl, (C₃-C₈)-Cycloalkyl-(C₁-C₆)-haloalkoxycarbonyl, (C₃-C₈)-Cycloalkylcarbonyloxy, (C₃-C₈)-Cycloalkoxycarbonyloxy, (C₃-C₈)-Cycloalkyl-(C₁-C₆)-alkylcarbonyloxy, (C₃-C₈)-Cycloalkyl-(C₁-C₆)-halogenalkylcarbonyloxy, (C₃-C₈)-Cycloalkyl-(C₁-C₆)-alkoxycarbonyloxy, (C₃-C₈)-Cycloalkyl-(C₁-C₆)-haloalkoxycarbonyloxy;
- (C₃-C₈)-Cycloalkenyl, (C₃-C₈)-Cycloalkenyloxy, (C₃-C₈)-Cycloalkenyl-(C₁-C₆)-alkyl, (C₃-C₈)-Cycloalkenyl-(C₁-C₆)-halogenalkyl, (C₃-C₈)-Cycloalkenyl-(C₁-C₆)-alkoxy, (C₃-C₈)-Cycloalkenyl-(C₁-C₆)-halogenalkoxy, (C₃-C₈)-Cycloalkenylcarbonyl, (C₃-C₈)-Cycloalkenyloxycarbonyl, (C₃-C₈)-Cycloalkenyl-(C₁-C₆)-alkylcarbonyl, (C₃-C₈)-Cycloalkenyl-(C₁-C₆)-halogenalkylcarbonyl, (C₃-C₈)-Cycloalkenyl-(C₁-C₆)-alkoxycarbonyl, (C₃-C₈)-Cycloalkenyl-(C₁-C₆)-haloalkoxycarbonyl, (C₃-C₈)-Cycloalkenylcarbonyloxy, (C₃-C₈)-Cycloalkenyloxycarbonyloxy, (C₃-C₈)-Cycloalkenyl-(C₁-C₆)-alkylcarbonyloxy, (C₃-C₈)-Cycloalkenyl-(C₁-C₆)-halogenalkylcarbonyloxy, (C₃-C₈)-Cycloalkenyl-(C₁-C₆)-alkoxycarbonyloxy, (C₃-C₈)-Cycloalkenyl-(C₁-C₆)-haloalkoxycarbonyloxy;
- Hydroxy-(C₁-C₆)-alkyl, Hydroxy-(C₁-C₆)-alkoxy Cyano-(C₁-C₆)-alkoxy, Cyano-(C₁-C₆)-alkyl;
- (C₁-C₆)-Alkylsulfonyl, (C₁-C₆)-Alkylthio, (C₁-C₆)-Alkylsulfinyl, (C₁-C₆)-Haloalkylsulfonyl, (C₁-C₆)-Halogenalkylthio, (C₁-C₆)-Halogenalkylsulfinyl, (C₁-C₆)-Alkylsulfonyl-(C₁-C₆)-alkyl, (C₁-C₆)-Alkylthio-(C₁-C₆)-alkyl, (C₁-C₆)-Alkylsulfinyl-(C₁-C₆)-alkyl, (C₁-C₆)-Haloalkylsulfonyl-(C₁-C₆)-alkyl, (C₁-C₆)-Haloalkylthio-(C₁-C₆)-alkyl, (C₁-C₆)-Haloalkylsulfinyl-(C₁-C₆)-alkyl, (C₁-C₆)-Alkylsulfonyl-(C₁-C₆)-haloalkyl, (C₁-C₆)-Alkylthio-(C₁-C₆)-haloalkyl, (C₁-C₆)-Alkylsulfinyl-(C₁-C₆)-haloalkyl, (C₁-C₆)-Haloalkylsulfonyl-(C₁-C₆)-haloalkyl, (C₁-C₆)-Haloalkylthio-(C₁-C₆)-haloalkyl, (C₁-C₆)-Haloalkylsulfinyl-(C₁-C₆)-haloalkyl, (C₁-C₆)-Alkylsulfonyloxy, (C₁-C₆)-Halogenalkylsulfonyloxy, (C₁-C₆)-Alkylthiocarbonyl, (C₁-C₆)-Haloalkylthiocarbonyl, (C₁-C₆)-Alkylthiocarbonyloxy, (C₁-C₆)-Haloalkylthiocarbonyloxy, (C₁-C₆)-Alkylthio-(C₁-C₆)-alkyl, (C₁-C₆)-Alkylthio-(C₁-C₆)-alkoxy, (C₁-C₆)-Alkylthio-(C₁-C₆)-alkylcarbonyl, (C₁-C₆)-Alkylthio-(C₁-C₆)-alkylcarbonyloxy; (C₄-C₁₄)-Arylsulfonyl, (C₆-C₁₄)-Arylthio, (C₆-C₁₄)-Arylsulfinyl, (C₃-C₈)-Cycloalkylthio, (C₃-C₈)-Alkenylthio, (C₃-C₈)-Cycloalkenylthio, (C₃-C₆)-Alkinylthio; und
R³ ausgewählt ist aus der Gruppe, bestehend aus
- Wasserstoff, Cyano, C(O)OH, C(O)NH₂;
- (C₁-C₆)-Alkyl und (C₁-C₆)-Haloalkyl,
- (C₁-C₆)-Alkoxycarbonyl, und
R⁴ und R⁵, jeweils unabhängig voneinander, ausgewählt sind aus der Gruppe, bestehend aus Wasserstoff, (C₁-C₆)-Alkyl, (C₁-C₆)-Haloalkyl, Hydroxy, (C₁-C₆)-Alkoxy und (C₁-C₆)-Halogenalkoxy, oder die Reste R⁴ und R⁵ zusammen mit dem Kohlenstoffatom, an welchem sie gebunden sind, einen drei- bis siebengliedrigen Ring bilden; und
R⁶ und R⁷, jeweils unabhängig voneinander, ausgewählt sind aus der Gruppe, bestehend aus Wasserstoff, (C₁-C₆)-Alkyl, (C₁-C₆)-Haloalkyl, (C₁-C₆)-Alkoxy, (C₁-C₆)-Halogenalkoxy, (C₆-C₁₄)-Aryl, (C₆-C₁₄)-Aryloxy, (C₆-C₁₄)-Arylcarbonyl und (C₆-C₁₄)-Aryloxycarbonyl, oder die Reste R⁶ und R⁷ zusammen eine (C₁-C₇)-Alkylengruppe bilden, die ein oder mehrere Sauerstoff- und/oder Schwefelatome enthalten können, wobei die (C₁-C₇)-Alkylengruppe durch Halogen einfach oder mehrfach substituiert sein kann und die jeweilgen Halogensubstituenten gleich oder verschieden sein können, und
n die Laufzahl 0, 1 oder 2 ist; und
R⁸, R⁹, R¹⁰ und R¹¹, jeweils unabhängig voneinander, ausgewählt sind aus der Gruppe, bestehend aus Wasserstoff, Halogen, Cyano, C(O)OH, C(O)NH₂, (C₁-C₆)-Alkyl, (C₁-C₆)-Alkylcarbonyl, (C₁-C₆)-Alkyloxycarbonyl, (C₁-C₆)-Alkylaminocarbonyl, (C₁-C₆)-di-Alkylaminocarbonyl, (C₁-C₆)-Haloalkyl, (C₁-C₆)-Alkoxy, (C₁-C₆)-Halogenalkoxy, (C₂-C₆)-Alkenyl, (C₂-C₆)-Alkinyl, (C₂-C₆)-Halogenalkinyl, (C₂-C₆)-Alkinylcarbonyl, (C₂-C₆)-Haloalkinylcarbonyl, (C₂-C₆)-Alkinyloxy, (C₂-C₆)-Haloalkinyloxy, (C₂-C₆)-Alkinyloxycarbonyl und (C₂-C₆)-Halogenalkinyloxycarbonyl und Nitro;
X für eine Bindung, CH₂, O, S, Carbonyl, NH, CR¹²R¹³ und NR¹⁴ oder CH₂O, CH₂S, wobei bei den beiden letztgenannten Gruppen das Kohlenstoffatom an den aromatischen Teil und das Heteroatom O oder S an den teilhydrierten Teil des Amins gebunden ist,
steht; und
R¹² und R¹³, jeweils unabhängig voneinander, ausgewählt sind aus der Gruppe, bestehend aus Wasserstoff, (C₁-C₆)-Alkyl und (C₁-C₆)-Haloalkyl, und
R¹⁴ ausgewählt ist aus der Gruppe, bestehend aus
Wasserstoff, (C₁-C₆)-Alkyl, (C₁-C₆)-Haloalkyl.

2. Verbindungen der allgemeinen Formel (I) nach Anspruch 1, **dadurch gekennzeichnet, dass** R¹ ausgewählt ist aus der Gruppe, bestehend aus, Cyano (CN), NO₂, (C₁-C₃)-Alkyl, (C₁-C₆)-Haloalkyl, (C₁-C₆)-Alkoxy, (C₁-C₆)-Haloalkoxy, (C₁-C₆)-Alkylthio, (C₁-C₆)-Alkylsulfoxid, (C₁-C₆)-Alkylsulfonyl, (C₁-C₆)-Haloalkylsulfonyl, (C₁-C₆)-Alkoxy-carbonyl, Amino-Carbonyl, Mono-((C₁-C₆)-alkyl)-amino-carbonyl, Di-((C₁-C₆)-alkyl)-aminocarbonyl, (C₆-C₁₄)-Aryl, (C₆-C₁₄)-Aryl-Haloalkyl, (C₁-C₆)-Aryloxy, (C₆-C₁₄)-Haloaryl-(C₁-C₆)alkyl, (C₂-C₆)-Alkinyl-(C₁-C₆)-alkoxy, (C₂-C₆)-Alkinyl-(C₁-C₆)alkyl-(C₁-C₆)-alkoxy und (C₁-C₆)Alkyl-(C₂-C₆)-Alkinyl-(C₁-C₆)-Alkoxy.

3. Verbindungen der allgemeinen Formel (I) nach Anspruch 1 oder 2, **dadurch gekennzeichnet, dass** die Reste R^{2a} und R^{2b}, jeweils unabhängig voneinander, ausgewählt sind aus der Gruppe, bestehend aus Wasserstoff, CN, Hydroxy (OH), (C₁-C₆)-Alkyl, (C₁-C₆)-Haloalkyl, (C₁-C₆)-Cycloalkyl, (C₁-C₆)-Alkoxy, Aminocarbonyl, Hydroxycarbonyl COOH, (C₁-C₆)-Alkoxycarbonyl, (C₁-C₆)-Alkylsulfonyl, Mono-((C₁-C₆)-alkyl)-amino-carbonyl, Di-((C₁-C₆)-alkyl)-aminocarbonyl, Mono-((C₆-C₁₄)-Aryl)-amino-carbonyl, Mono-((C₂-C₆)-Alkylen)-aminocarbonyl, Mono-((C₆-C₁₄)-Aryl-(C₁-C₆)-Alkyl)-amino-carbonyl, (C₁-C₆)-Alkoxy-(C₁-C₆)-alkoxy-carbonyl und Di-((C₁-C₆)-alkyl)-amino-(C₁-C₆)-alkoxycarbonyl.

4. Verbindungen der allgemeinen Formel (I) nach einem der Ansprüche 1 bis 3, **dadurch gekennzeichnet, dass** der Rest R³ ausgewählt ist aus der Gruppe, bestehend aus Wasserstoff, CH₃, CH₂CH₂OCH₃, COOCH₃, CONH₂, und C₆H₅.

5. Verbindungen der allgemeinen Formel (I) nach einem der Ansprüche 1 bis 4, **dadurch gekennzeichnet, dass** die Reste R⁴ und R⁵ jeweils unabhängig voneinander, ausgewählt sind aus der Gruppe, bestehend aus Wasserstoff, Cyano, Hydroxy, (C₁-C₆)-Alkyl und (C₁-C₆)-Alkoxy.

6. Verbindungen der allgemeinen Formel (I) nach einem der Ansprüche 1 bis 4, **dadurch gekennzeichnet, dass** die Reste R⁴ und R⁵ für eine (C₁-C₃)-Alkylengruppe stehen und zusammen mit dem Kohlenstoffatom, an welches sie gebunden sind, einen drei oder viergliedrigen Ring bilden.

7. Verbindungen der allgemeinen Formel (I) nach einem der Ansprüche 1 bis 6, **dadurch gekennzeichnet, dass** R⁶ und R⁷ unabhängig voneinander, ausgewählt sind aus der Gruppe, bestehend aus Wasserstoff, Methyl und Phenyl.

8. Verbindungen der allgemeinen Formel (I) nach einem der Ansprüche 1 bis 7, **dadurch gekennzeichnet, dass** der Rest R⁸ ausgewählt ist aus der Gruppe, bestehend aus Wasserstoff, Halogen, (C₁-C₆)-Alkyl, (C₁-C₆)Alkoxy, und Di-((C₁-C₆)alkylamino.

9. Verbindungen der allgemeinen Formel (I) nach einem der Ansprüche 1 bis 8, **dadurch gekennzeichnet, dass** der Rest R⁹ ausgewählt ist aus der Gruppe, bestehend aus Wasserstoff, Chlor, Fluor, (C₁-C₆)-Alkyl und (C₁-C₆)-Alkoxy.

10. Verbindungen der allgemeinen Formel (I) nach einem der Ansprüche 1 bis 9, **dadurch gekennzeichnet, dass** der Rest R¹⁰ ausgewählt ist aus der Gruppe, bestehend aus Wasserstoff, Fluor, Chlor, Brom, Cyano, CONH₂, COOH, Methyl (CH₃), Ethyl (CH₂CH₃), Methoxy (OCH₃), CH=CH₂, C=CH und C=CCH₃.

11. Verbindung der allgemeinen Formel (I) nach einem der Ansprüche 1 bis 10, **dadurch gekennzeichnet, dass** der Rest R¹¹ ausgewählt ist aus der Gruppe, bestehend aus Wasserstoff und Methyl.

12. Verbindung der allgemeinen Formel (I) nach einem der Ansprüche 1 bis 11, **dadurch gekennzeichnet, dass** der Rest X ausgewählt ist aus der Gruppe, bestehend aus O, S, Carbonyl, CH₂, NH, CHCH₃, NCH₃, C(CH₃)2, OCH₂, SCH₂, oder in welchen X für eine chemische Bindung steht.

13. Verfahren zur Herstellung von Verbindungen der allgemeinen Formel (I) und/oder deren agrochemisch verträgliche Salze und/oder deren agrochemisch verträgliche quaternierte Stickstoff-Derivate: 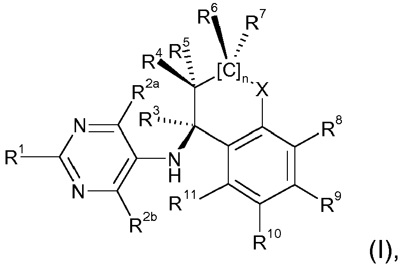 worin die Reste R¹ bis R¹¹ und X sowie n wie in einem der Ansprüche 1 bis 12 definiert sind, und wobei
- eine Verbindung der allgemeinen Formel (II) 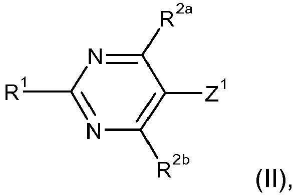 worin R¹ und R^{2a} sowie R^{2b} wie in einem der Ansprüche 1 bis 12 definiert sind, und Z¹ für einen austauschfähigen Rest oder eine Abgangsgruppe steht,
- umgesetzt wird mit einem Amin der allgemeinen Formel (III) oder mit einem Säureadditionssalz des Amins der allgemeinen Formel (III) 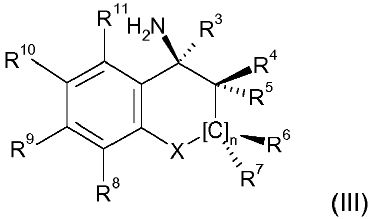 worin die Reste R³ bis R¹¹ und X sowie n die vorstehende Bedeutung aufweisen.

14. Verfahren nach Anspruch 13, wobei der austauschfähige Rest, bzw. die Abgangsgruppe Z¹ für Fluor, Chlor, Brom, Jod, ein (C₁-C₄)-Alkylsulfonyl, ein unsubstituiertes oder substituiertes Phenyl-(C₁-C₄)-alkylsulfonyl oder ein (C₁-C₄)-Alkylphenylsulfonyl steht.

15. Herbizides oder pflanzenwachstumsregulierendes Mittel, **dadurch gekennzeichnet, dass** es eine oder mehrere Verbindungen der allgemeinen Formel (I) oder deren Salze nach einem der Ansprüche 1 bis 12 enthält.

16. Verfahren zur Bekämpfung von Schadpflanzen oder zur Wachstumsregulierung von Pflanzen, **dadurch gekennzeichnet, dass** man eine wirksame Menge von einer oder mehreren Verbindungen der allgemeinen Formel (I) oder deren Salzen nach einem der Ansprüche 1 bis 12 auf Pflanzen, Pflanzenteile, Pflanzensamen oder auf eine Anbaufläche appliziert.

17. Verwendung von Verbindungen der allgemeinen Formel (I) oder deren Salzen nach einem der Ansprüche 1 bis 12 als Herbizide oder Pflanzenwachstumsregulatoren.

18. Verwendung nach Anspruch 17, **dadurch gekennzeichnet, dass** die Verbindungen der allgemeinen Formel (I) oder deren Salze zur Bekämpfung von Schadpflanzen oder zur Wachstumsregulierung von Pflanzen in Kulturen von Nutz- oder Zierpflanzen eingesetzt werden.

19. Verwendung nach Anspruch 18, **dadurch gekennzeichnet, dass** die Kulturpflanzen transgene Kulturpflanzen sind.

20. Verwendung nach Anspruch 17 oder 18, **dadurch gekennzeichnet, dass** die Kulturpflanzen aus Plantagenkulturen ausgewählt sind.

## Claims

1. Compounds of the general formula (I) and their agrochemically acceptable salts thereof 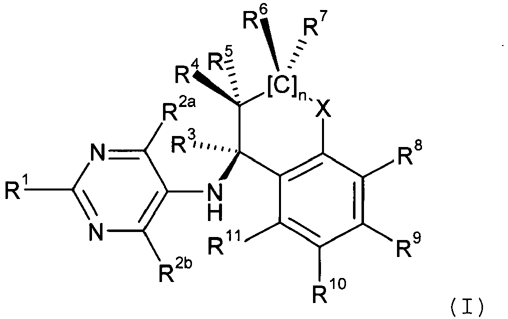 in which
R¹ is selected from the group consisting of
- cyano, nitro, C(O)OH, C(O)NH₂;
- (C₁-C₆)-alkyl, (C₁-C₆)-haloalkyl, (C₁-C₆)-alkylcarbonyl, (C₁-C₆)-haloalkylcarbonyl, (C₁-C₆)-alkylcarbonyloxy, (C₁-C₆)-haloalkylcarbonyloxy, (C₁-C₆)-alkylcarbonyl-(C₁-C₄)-alkyl;
- (C₁-C₆)-alkoxy, (C₁-C₆)-haloalkoxy, (C₁-C₆)-alkoxycarbonyl, (C₁-C₆)-haloalkoxycarbonyl; (C₁-C₆)-alkoxycarbonyl- (C₁-C₆)-alkyl, (C₁-C₆)-haloalkoxycarbonyl- (C₁-C₆)-alkyl, (C₁-C₆)-alkoxycarbonyl- (C₁-C₆)-haloalkyl, (C₁-C₆) haloalkoxycarbonyl-(C₁-C₆)-haloalkyl;
- (C₂-C₆)-alkenyl, (C₂-C₆) -haloalkenyl, (C₂-C₆)-alkenylcarbonyl, (C₂-C₆)-haloalkenylcarbonyl, (C₂-C₆) -alkenyloxy, (C₂-C₆) -haloalkenyloxy, (C₂-C₆) -alkenyloxycarbonyl, (C₂-C₆) haloalkenyloxycarbonyl;
- (C₂-C₆)-alkynyl, (C₂-C₆) -haloalkynyl, (C₂-C₆)-alkynylcarbonyl, (C₂-C₆)-haloalkynylcarbonyl, (C₂-C₆) -alkynyloxy, (C₂-C₆) -haloalkynyloxy, (C₂-C₆) -alkynyloxycarbonyl, (C₂-C₆)-haloalkynyloxycarbonyl;
- tri-(C₁-C₆)-alkylsilyl-(C₂-C₆)-alkynyl, di-(C₁-C₆)-alkylsilyl-(C₂-C₆)-alkynyl, mono-(C₁-C₆)-alkylsilyl-(C₂-C₆)-alkynyl; phenylsilyl-(C₂-C₆)-alkynyl;
- (C₆-C₁₄)-aryl, (C₆-C₁₄)-aryloxy, (C₆-C₁₄)-arylcarbonyl and (C₆-C₁₄)-aryloxycarbonyl, each of which may be substituted at the aryl moiety by halogen, (C₁-C₆)-alkyl and/or (C₁-C₆)-haloalkyl;
- (C₆-C₁₄)-aryl-(C₁-C₆)-alkyl, (C₆-C₁₄) -aryl- (C₁-C₆)-alkoxy, (C₆-C₁₄) -aryl- (C₁-C₆)-alkylcarbonyl, (C₆-C₁₄) -aryl- (C₁-C₆)-alkylcarbonyloxy, (C₆-C₁₄) -aryl-(C₁-C₆)-alkoxycarbonyl, (C₆-C₁₄) -aryl- (C₁-C₆) alkoxycarbonyloxy;
- aminocarbonyl-(C₁-C₆)-alkyl, di-(C₁-C₆)-alkylaminocarbonyl-(C₁-C₆)-alkyl;
- mono-((C₁-C₆)-alkyl)-amino-carbonyl, mono-((C₁-C₆)-haloalkyl)-amino-carbonyl, di-((C₁-C₆)-alkyl)-amino-carbonyl, di-((C₁-C₆)-haloalkyl)-amino-carbonyl, ((C₁-C₆)-alkyl-(C₁-C₆)-haloalkyl)-amino-carbonyl, N-((C₁-C₆)-alkanoyl)-amino-carbonyl, N-((C₁-C₆)-haloalkanoyl)-amino-carbonyl, mono-((C₆-C₁₄)-aryl)-amino-carbonyl, di- (C₆-C₁₄)-aryl)-amino-carbonyl;
- (C₁-C₆)-alkoxy-(C₁-C₆)-alkyl, (C₁-C₆)-alkoxy-(C₁-C₆)-alkoxy, (C₁-C₆)-alkoxycarbonyl-(C₁-C₆)-alkoxy;
- (C₃-C₈)-cycloalkyl which may optionally be substituted at the cycloalkyl radical by (C₁-C₆)-alkyl and/or halogen; (C₃-C₈)-cycloalkoxy, (C₃-C₈) -cycloalkyl- (C₁-C₆)-alkyl, (C₃-C₈)-cycloalkyl- (C₁-C₆)-haloalkyl, (C₃-C₈)-cycloalkyl- (C₁-C₆)-alkoxy, (C₃-C₈)-cycloalkyl-(C₁-C₆)-haloalkoxy, (C₃-C₈)-cycloalkylcarbonyl, (C₃-C₈)-cycloalkoxycarbonyl, (C₃-C₈)-cycloalkyl- (C₁-C₆)-alkylcarbonyl, (C₃-C₈)-cycloalkyl- (C₁-C₆)-haloalkylcarbonyl, (C₃-C₈)-cycloalkyl- (C₁-C₆)-alkoxycarbonyl, (C₃-C₈)-cycloalkyl- (C₁-C₆)-haloalkoxycarbonyl, (C₃-C₈)-cycloalkylcarbonyloxy, (C₃-C₈)-cycloalkoxycarbonyloxy, (C₃-C₈-cycloalkyl-(C₁-C₆)-alkylcarbonyloxy, (C₃-C₈)-cycloalkyl-(C₁-C₆)-haloalkylcarbonyloxy, (C₃-C₈)-cycloalkyl- (C₁-C₆)-alkoxycarbonyloxy, (C₃-C₈)-cycloalkyl-(C₁-C₆)-haloalkoxycarbonyloxy;
- (C₃-C₈)-cycloalkenyl, (C₃-C₈)-cycloalkenyloxy, (C₃-C₈)-cycloalkenyl- (C₁-C₆)-alkyl, (C₃-C₈)-cycloalkenyl- (C₁-C₆)-haloalkyl, (C₃-C₈)-cycloalkenyl- (C₁-C₆)-alkoxy, (C₃-C₈)-cycloalkenyl- (C₁-C₆)-haloalkoxy, (C₃-C₈)-cycloalkenylcarbonyl, (C₃-C₈)-cycloalkenyloxycarbonyl, (C₃-C₈)-cycloalkenyl-(C₁-C₆)-alkylcarbonyl, (C₃-C₈)-cycloalkenyl-(C₁-C₆)-haloalkylcarbonyl, (C₃-C₈)-cycloalkenyl- (C₁-C₆)-alkoxycarbonyl, (C₃-C₈)-cycloalkenyl-(C₁-C₆)-haloalkoxycarbonyl, (C₃-C₈) -cycloalkenylcarbonyloxy, (C₃-C₈)-cycloalkenyloxycarbonyloxy, (C₃-C₈)-cycloalkenyl- (C₁-C₆)-alkylcarbonyloxy, (C₃-C₈)-cycloalkenyl-(C₁-C₈)-haloalkylcarbonyloxy, (C₃-C₈)-cycloalkenyl-(C₁-C₆)-alkoxycarbonyloxy, (C₃-C₈)-cycloalkenyl-(C₁-C₆)-haloalkoxycarbonyloxy;
- hydroxy-(C₁-C₆)-alkyl, hydroxy-(C₁-C₆)-alkoxy, cyano-(C₁-C₆)-alkoxy, cyano-(C₁-C₆)-alkyl;
- (C₁-C₆)-alkylsulfonyl, (C₁-C₆)-alkylthio, (C₁-C₆)-alkylsulfinyl, (C₁-C₆)-haloalkylsulfonyl, (C₁-C₆)-haloalkylthio, (C₁-C₆)-haloalkylsulfinyl, (C₁-C₆)-alkylsulfonyl-(C₁-C₆)-alkyl, (C₁-C₆)-alkylthio- (C₁-C₆)-alkyl, (C₁-C₆)-alkylsulfinyl-(C₁-C₆)-alkyl, (C₁-C₆)-haloalkylsulfonyl-(C₁-C₆)-alkyl, (C₁-C₆)-haloalkylthio-(C₁-C₆)-alkyl, (C₁-C₆)-haloalkylsulfinyl-(C₁-C₆)-alkyl, (C₁-C₆)-alkylsulfonyl-(C₁-C₆)-haloalkyl, (C₁-C₆)-alkylthio- (C₁-C₆)-haloalkyl, (C₁-C₆)-alkylsulfinyl-(C₁-C₆)-haloalkyl, (C₁-C₆)-haloalkylsulfonyl-(C₁-C₆)-haloalkyl, (C₁-C₆)-haloalkylthio- (C₁-C₆)-haloalkyl, (C₁-C₆)-haloalkylsulfinyl-(C₁-C₆)-haloalkyl, (C₁-C₆)-alkylsulfonyloxy, (C₁-C₆)-haloalkylsulfonyloxy, (C₁-C₆)-alkylthiocarbonyl, (C₁-C₆)-haloalkylthiocarbonyl, (C₁-C₆) alkylthiocarbonyloxy, (C₁-C₆)-haloalkylthiocarbonyloxy, (C₁-C₆)-alkylthio-(C₁-C₆)-alkyl, (C₁-C₆)-alkylthio- (C₁-C₆) alkoxy, (C₁-C₆)-alkylthio-(C₁-C₆)-alkylcarbonyl, (C₁-C₆)-alkylthio- (C₁-C₆) alkylcarbonyloxy; (C₄-C₁₄)-arylsulfonyl, (C₆-C₁₄)-arylthio, (C₆-C₁₄)-arylsulfinyl, (C₃-C₈)-cycloalkylthio, (C₃-C₈)-alkenylthio, (C₃-C₈)-cycloalkenylthio, (C₃-C₆)-alkynylthio; and
R^{2a} and R^{2b}, each independently of one another, are selected from the group consisting of
- hydrogen, halogen, hydroxy, cyano, nitro, C(O)OH, C(O)NH₂;
- (C₁-C₆)-alkyl, (C₁-C₆)-haloalkyl, (C₁-C₆)-alkylcarbonyl, (C₁-C₆)-haloalkylcarbonyl, (C₁-C₆)-alkylcarbonyloxy, (C₁-C₆)-haloalkylcarbonyloxy, (C₁-C₆)-alkylcarbonyl-(C₁-C₄) -alkyl;
- (C₁-C₆)-alkoxy, (C₁-C₆)-haloalkoxy, (C₁-C₆)-alkoxycarbonyl, (C₁-C₆)-haloalkoxycarbonyl, (C₁-C₆)-alkoxycarbonyl- (C₁-C₆)-alkyl, (C₁-C₆) - haloalkoxycarbonyl- (C₁-C₆)-alkyl, (C₁-C₆) - alkoxycarbonyl- (C₁-C₆)-haloalkyl, (C₁-C₆) - haloalkoxycarbonyl-(C₁-C₆)-haloalkyl;
- (C₂-C₆)-alkenyl, (C₂-C₆) -haloalkenyl, (C₂-C₆)-alkenylcarbonyl, (C₂-C₆)-haloalkenylcarbonyl, (C₂-C₆) -alkenyloxy, (C₂-C₆)-haloalkenyloxy, (C₂-C₆) -alkenyloxycarbonyl, (C₂-C₆)-haloalkenyloxycarbonyl;
- (C₂-C₆)-alkynyl, (C₂-C₆)-haloalkynyl, (C₂-C₆)-alkynylcarbonyl, (C₂-C₆)-haloalkynylcarbonyl, (C₂-C₆) -alkynyloxy, (C₂-C₆) -haloalkynyloxy, (C₂-C₆) -alkynyloxycarbonyl, (C₂-C₆)-haloalkynyloxycarbonyl;
- tri-(C₁-C₆)-alkylsilyl-(C₂-C₆)-alkynyl, di-(C₁-C₆)-alkylsilyl-(C₂-C₆)-alkynyl, mono-(C₁-C₆)-alkylsilyl-(C₂-C₆)-alkynyl; phenylsilyl-(C₂-C₆)-alkynyl;
- (C₆-C₁₄)-aryl, (C₆-C₁₄)-aryloxy, (C₆-C₁₄)-arylcarbonyl and (C₆-C₁₄)-aryloxycarbonyl, each of which may be substituted at the aryl moiety by halogen, (C₁-C₆)-alkyl and/or (C₁-C₆)-haloalkyl;
- (C₆-C₁₄)-aryl-(C₁-C₆)-alkyl, (C₆-C₁₄) -aryl- (C₁-C₆)-alkoxy, (C₆-C₁₄)-aryl-(C₁-C₆)-alkylcarbonyl, (C₆-C₁₄) -aryl- (C₁-C₆)-alkylcarbonyloxy, (C₆-C₁₄) -aryl- (C₁-C₆)-alkoxycarbonyl, (C₆-C₁₄) -aryl- (C₁-C₆) alkoxycarbonyloxy;
- N-((C₁-C₆)-haloalkanoyl)-amino, aminocarbonyl-(C₁-C₆-alkyl, di-(C₁-C₆)-alkylaminocarbonyl-(C₁-C₆)-alkyl;
- N-((C₁-C₆)-haloalkanoyl)-amino-carbonyl, mono-((C₆-C₁₄) -aryl) -amino-carbonyl, di- ((C₆-C₁₄) - aryl)-amino-carbonyl;
- (C₁-C₆)-alkoxy-(C₁-C₆)-alkyl, (C₁-C₆)-alkoxy-(C₁-C₆)-alkoxy, (C₁-C₆)-alkoxycarbonyl-(C₁-C₆)-alkoxy;
- (C₃-C₈)-cycloalkyl which may optionally be substituted at the cycloalkyl radical by (C₁-C₆)-alkyl and/or halogen; (C₃-C₈)-cycloalkoxy, (C₃-C₈)-cycloalkyl- (C₁-C₆)-alkyl, (C₃-C₈)-cycloalkyl- (C₁-C₆)-haloalkyl, (C₃-C₈)-cycloalkyl- (C₁-C₆)-alkoxy, (C₃-C₈)-cycloalkyl-(C₁-C₆)-haloalkoxy, (C₃-C₈-cycloalkylcarbonyl, (C₃-C₈)-cycloalkoxycarbonyl, (C₃-C₈)-cycloalkyl- (C₁-C₆)-alkylcarbonyl, (C₃-C₈)-cycloalkyl-(C₁-C₆)-haloalkylcarbonyl, (C₃-C₈)-cycloalkyl- (C₁-C₆)-alkoxycarbonyl, (C₃-C₈)-cycloalkyl- (C₁-C₆)-haloalkoxycarbonyl, (C₃-C₈)-cycloalkylcarbonyloxy, (C₃-C₈)-cycloalkoxycarbonyloxy, (C₃-C₈)-cycloalkyl-(C₁-C₆)-alkylcarbonyloxy, (C₃-C₈)-cycloalkyl-(C₁-C₆)-haloalkylcarbonyloxy, (C₃-C₈)-cycloalkyl- (C₁-C₆) -alkoxycarbonyloxy, (C₃-C₈)-cycloalkyl-(C₁-C₆)-haloalkoxycarbonyloxy;
- (C₃-C₈)-cycloalkenyl, (C₃-C₈)-cycloalkenyloxy, (C₃-C₈)-cycloalkenyl-(C₁-C₈)-alkyl, (C₃-C₈)-cycloalkenyl- (C₁-C₆)-haloalkyl, (C₃-C₈)-cycloalkenyl- (C₁-C₆)-alkoxy, (C₃-C₈)-cycloalkenyl-(C₁-C₆)-haloalkoxy, (C₃-C₈)-cycloalkenylcarbonyl, (C₃-C₈)-cycloalkenyloxycarbonyl, (C₃-C₈)-cycloalkenyl-(C₁-C₆)-alkylcarbonyl, (C₃-C₈)-cycloalkenyl-(C₁-C₆)-haloalkylcarbonyl, (C₃-C₈)-cycloalkenyl- (C₁-C₆)-alkoxycarbonyl, (C₃-C₈)-cycloalkenyl-(C₁-C₆)-haloalkoxycarbonyl, (C₃-C₈)-cycloalkenylcarbonyloxy, (C₃-C₈)-cycloalkenyloxycarbonyloxy, (C₃-C₈)-cycloalkenyl-(C₁-C₈)-alkylcarbonyloxy, (C₃-C₈)-cycloalkenyl-(C₁-C₆)-haloalkylcarbonyloxy, (C₃-Ca) -cycloalkenyl- (C₁-C₆) -alkoxycarbonyloxy, (C₃-C₈)-cycloalkenyl-(C₁-C₆)-haloalkoxycarbonyloxy;
- hydroxy-(C₁-C₆)-alkyl, hydroxy-(C₁-C₆)-alkoxy, cyano-(C₁-C₆)-alkoxy, cyano-(C₁-C₆)-alkyl;
- (C₁-C₆)-alkylsulfonyl, (C₁-C₆)-alkylthio, (C₁-C₆)-alkylsulfinyl, (C₁-C₆)-haloalkylsulfonyl, (C₁-C₆)-haloalkylthio, (C₁-C₆)-haloalkylsulfinyl, (C₁-C₆)-alkylsulfonyl-(C₁-C₆)-alkyl, (C₁-C₆)-alkylthio-(C₁-C₆)-alkyl, (C₁-C₆)-alkylsulfinyl-(C₁-C₆)-alkyl, (C₁-C₆)-haloalkylsulfonyl-(C₁-C₆)-alkyl, (C₁-C₆)-haloalkylthio- (C₁-C₆)-alkyl, (C₁-C₆)-haloalkylsulfinyl-(C₁-C₆)-alkyl, (C₁-C₆)-alkylsulfonyl-(C₁-C₆)-haloalkyl, (C₁-C₆)-alkylthio- (C₁-C₆)-haloalkyl, (C₁-C₆)-alkylsulfinyl-(C₁-C₆)-haloalkyl, (C₁-C₆)-haloalkylsulfonyl-(C₁-C₆)-haloalkyl, (C₁-C₆)-haloalkylthio- (C₁-C₆)-haloalkyl, (C₁-C₆)-haloalkylsulfinyl-(C₁-C₆)-haloalkyl, (C₁-C₆)-alkylsulfonyloxy, (C₁-C₆)-haloalkylsulfonyloxy, (C₁-C₆)-alkylthiocarbonyl, (C₁-C₆)-haloalkylthiocarbonyl, (C₁-C₆)-alkylthiocarbonyloxy, (C₁-C₆)-haloalkylthiocarbonyloxy, (C₁-C₆)-alkylthio-(C₁-C₆)-alkyl, (C₁-C₆)-alkylthio- (C₁-C₆)-alkoxy, (C₁-C₆)-alkylthio- (C₁-C₆)-alkylcarbonyl, (C₁-C₆)-alkylthio- (C₁-C₆)-alkylcarbonyloxy; (C₉-C₁₄)-arylsulfonyl, (C₆-C₁₄)-arylthio, (C₆-C₁₄)-arylsulfinyl, (C₃-C₈)-cycloalkylthio, (C₃-C₈)-alkenylthio, (C₃-C₈)-cycloalkenylthio, (C₃-C₆)-alkynylthio; and
R³ is selected from the group consisting of
- hydrogen, cyano, C(O)OH, C(O)NH₂;
- (C₁-C₆)-alkyl and (C₁-C₆)-haloalkyl;
- (C₁-C₆)-alkoxycarbonyl; and
R⁴ and R⁵, each independently of one another, are selected from the group consisting of hydrogen, (C₁-C₆) -alkyl, (C₁-C₆) -haloalkyl, hydroxy, (C₁-C₆)-alkoxy and (C₁-C₆)-haloalkoxy; or the radicals R⁴ and R⁵ together with the carbon atom to which they are attached form a three- to seven-membered ring; and
R⁶ and R⁷, each independently of one another, are selected from the group consisting of hydrogen, (C₁-C₆) -alkyl, (C₁-C₆)-haloalkyl, (C₁-C₆)-alkoxy, (C₁-C₆)-haloalkoxy, (C₆-C₁₄)-aryl, (C₆-C₁₄)-aryloxy, (C₆-C₁₄)-arylcarbonyl and (C₆-C₁₄)-aryloxycarbonyl; or the radicals R⁶ and R⁷ together form a (C₁-C₇)-alkylene group which may contain one or more oxygen and/or sulfur atoms, where the (C₁-C₇)-alkylene group may be mono- or polysubstituted by halogen and the halogen substituents in question may be identical or different, and
n is the running number 0, 1 or 2 and
R⁸, R⁹, R¹⁰ and R¹¹, each independently of one another, are selected from the group consisting of hydrogen, halogen, cyano, C(O)OH, C(O)NH₂, (C₁-C₆)-alkyl, (C₁-C₆)-alkylcarbonyl, (C₁-C₆)-alkyloxycarbonyl, (C₁-C₆)-alkylaminocarbonyl, (C₁-C₆)-dialkylaminocarbonyl, (C₁-C₆)-haloalkyl, (C₁-C₆)-alkoxy, (C₁-C₆)-haloalkoxy, (C₂-C₆) -alkenyl, (C₂-C₆)-alkynyl, (C₂-C₆) -haloalkynyl, (C₂-C₆)-alkynylcarbonyl, (C₂-C₆)-haloalkynylcarbonyl, (C₂-C₆)-alkynyloxy, (C₂-C₆)-haloalkynyloxy, (C₂-C₆)-alkynyloxycarbonyl and (C₂-C₆)-haloalkynyloxycarbonyl and nitro;
X is a bond, CH₂, O, S, carbonyl, NH, CR¹²R¹³ and NR¹⁴ or CH₂O, CH₂S, where in the two last-mentioned groups the carbon atom is attached to the aromatic moiety and the heteroatom O or S is attached to the partially hydrogenated moiety of the amine;
and
R¹² and R¹³, each independently of one another, are selected from the group consisting of hydrogen, (C₁-C₆)-alkyl and (C₁-C₆)-haloalkyl; and
R¹⁴ is selected from the group consisting of hydrogen, (C₁-C₆)-alkyl, (C₁-C₆)-haloalkyl.

2. Compounds of the general formula (I) according to Claim 1, **characterized in that** R¹ is selected from the group consisting of
cyano (CN), NO₂, (C₁-C₃)-alkyl, (C₁-C₆)-haloalkyl, (C₁-C₆)-alkoxy,
(C₁-C₆)-haloalkoxy, (C₁-C₆)-alkylthio, (C₁-C₆)-alkyl sulfoxide, (C₁-C₆)-alkylsulfonyl, (C₁-C₆)-haloalkylsulfonyl, (C₁-C₆)-alkoxy-carbonyl, amino-carbonyl, mono-((C₁-C₆)-alkyl)-amino-carbonyl, di-((C₁-C₆)-alkyl)-amino-carbonyl, (C₆-C₁₄)-aryl, (C₆-C₁₄)-aryl-haloalkyl, (C₁-C₆)-aryloxy, (C₆-C₁₄)-haloaryl- (C₁-C₆)-alkyl, (C₂-C₆) -alkynyl- (C₁-C₆)-alkoxy, (C₂-C₆)-alkynyl-(C₁-C₆)-alkyl-(C₁-C₆)-alkoxy and (C₁-C₆)-alkyl-(C₂-C₆)-alkynyl-(C₁-C₆)-alkoxy.

3. Compounds of the general formula (I) according to Claim 1 or 2, **characterized in that** the radicals R^{2a} and R^{2b}, each independently of one another, are selected from the group consisting of hydrogen, CN, hydroxy (OH), (C₁-C₆) -alkyl, (C₁-C₆)-haloalkyl, (C₁-C₆)-cycloalkyl, (C₁-C₆)-alkoxy, aminocarbonyl, hydroxycarbonyl COOH, (C₁-C₆)-alkoxycarbonyl, (C₁-C₆)-alkylsulfonyl, mono-((C₁-C₆)-alkyl)-amino-carbonyl, di-((C₁-C₆)-alkyl)-amino-carbonyl, mono-((C₆-C₁₄) -aryl) -amino-carbonyl, mono- ((C₂-C₆)-alkylene)-amino-carbonyl, mono- (C₆-C₁₄)-aryl-(C₁-C₆)-alkyl) -amino-carbonyl, (C₁-C₆)-alkoxy-(C₁-C₆)-alkoxy-carbonyl and di-((C₁-C₆)-alkyl)-amino-(C₁-C₆)-alkoxycarbonyl.

4. Compounds of the general formula (I) according to any of Claims 1 to 3, **characterized in that** the radical R³ is selected from the group consisting of hydrogen, CH₃, CH₂CH₂OCH₃, COOCH₃, CONH₂ and C₆H₅.

5. Compounds of the general formula (I) according to any of Claims 1 to 4, **characterized in that** the radicals R⁴ and R⁵, each independently of one another, are selected from the group consisting of hydrogen, cyano, hydroxy, (C₁-C₆)-alkyl and (C₁-C₆)-alkoxy.

6. Compounds of the general formula (I) according to any of Claims 1 to 4, **characterized in that** the radicals R⁴ and R⁵ are a (C₁-C₃)-alkylene group and together with the carbon atom to which they are attached form a three- or four-membered ring.

7. Compounds of the general formula (I) according to any of Claims 1 to 6, **characterized in that** R⁶ and R⁷ independently of one another are selected from the group consisting of hydrogen, methyl and phenyl.

8. Compounds of the general formula (I) according to any of Claims 1 to 7, **characterized in that** the radical R⁸ is selected from the group consisting of hydrogen, halogen, (C₁-C₆)-alkyl, (C₁-C₆)-alkoxy and di-(C₁-C₆)-alkylamino.

9. Compounds of the general formula (I) according to any of Claims 1 to 8, **characterized in that** the radical R⁹ is selected from the group consisting of hydrogen, chlorine, fluorine, (C₁-C₆)-alkyl and (C₁-C₆)-alkoxy.

10. Compounds of the general formula (I) according to any of Claims 1 to 9, **characterized in that** the radical R¹⁰ is selected from the group consisting of hydrogen, fluorine, chlorine, bromine, cyano, CONH₂, COOH, methyl (CH₃), ethyl (CH₂CH₃), methoxy (OCH₃), CH=CH₂, C=CH and C≡CCH₃.

11. Compounds of the general formula (I) according to any of Claims 1 to 10, **characterized in that** the radical R¹¹ is selected from the group consisting of hydrogen and methyl.

12. Compounds of the general formula (I) according to any of Claims 1 to 11, **characterized in that** the radical X is selected from the group consisting of O, S, carbonyl, CH₂, NH, CHCH₃, NCH₃, C(CH₃)₂, OCH₂, SCH₂, or in which X is a chemical bond.

13. Process for preparing compounds of the general formula (I) and/or agrochemically acceptable salts thereof and/or agrochemically acceptable quaternized nitrogen derivatives thereof: 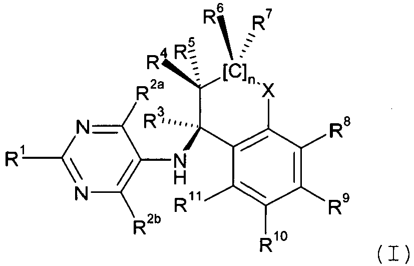 in which the radicals R¹ to R¹¹ and X and also n are as defined in any of Claims 1 to 12, and where
- a compound of the general formula (II) 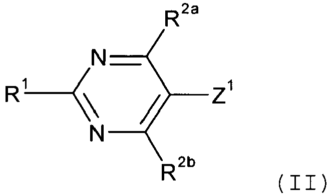 in which R¹ and R^{2a} and also R^{2b} are as defined in any of Claims 1 to 12 and Z¹ is an exchangeable radical or a leaving group,
- is reacted with an amine of the general formula (III) or an acid addition salt of the amine of the general formula (III) 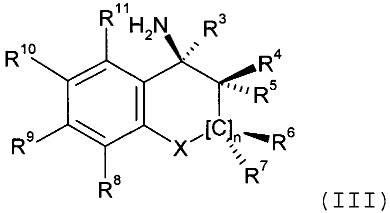 in which the radicals R³ to R¹¹ and X and also n have the above meaning.

14. Process according to Claim 13, where the exchangeable radical or the leaving group Z¹ is fluorine, chlorine, bromine, iodine, a (C₁-C₄)-alkylsulfonyl, an unsubstituted or a substituted phenyl-(C₁-C₄)-alkylsulfonyl or a (C₁-C₄)-alkylphenylsulfonyl.

15. Herbicidal or plant growth-regulating composition, **characterized in that** it comprises one or more compounds of the general formula (I) or salts thereof according to any of Claims 1 to 12.

16. Method for controlling harmful plants or for regulating the growth of plants, **characterized in that** an effective amount of one or more compounds of the general formula (I) or salts thereof according to any of Claims 1 to 12 is applied onto the plants, plant parts, plant seeds or an area under cultivation.

17. Use of compounds of the general formula (I) or the salts thereof according to any of Claims 1 to 12 as herbicides or plant growth regulators.

18. Use according to Claim 17, **characterized in that** the compounds of the general formula (I) or salts thereof are employed for controlling harmful plants or for regulating the growth of plants in crops of useful plants or ornamental plants.

19. Use according to Claim 18, **characterized in that** the crop plants are transgenic crop plants.

20. Use according to Claim 17 or 18, **characterized in that** the crop plants are selected from plantation crops.

## Revendications

1. Composés de formule générale (I) et leurs sels acceptables sur le plan agrochimique, 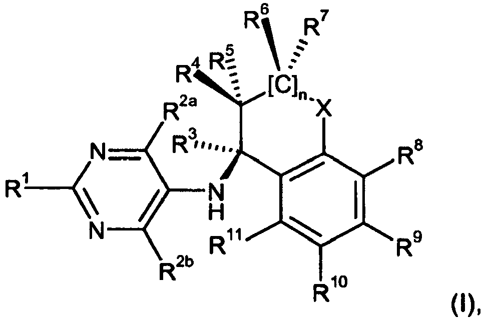 dans laquelle
R¹ est choisi dans le groupe constitué par
- cyano, nitro, C(O)OH, C(O)NH₂;
- (C₁-C₆)-alkyle, (C₁-C₆)-halogénoalkyle, (C₁-C₆)-alkylcarbonyle, (C₁-C₆)-halogénoalkylcarbonyle, (C₁-C₆) -alkylcarbonyloxy, (C₁-C₆)-halogénoalkylcarbonyloxy, (C₁-C₆)-alkylcarbonyl-(C₁-C₄)-alkyle ;
- (C₁-C₆)-alcoxy, (C₁-C₆)-halogénoalcoxy, (C₁-C₆)-alcoxycarbonyle, (C₁-C₆)-halogénoalcoxycarbonyle, (C₁-C₆) -alcoxycarbonyl- (C₁-C₆) -alkyle, (C₁-C₆)-halogénoalcoxycarbonyl- (C₁-C₆) -alkyle, (C₁-C₆)-alcoxycarbonyl- (C₁-C₆) -halogénoalkyle, (C₁-C₆)-halogénoalcoxycarbonyl-(C₁-C₆)-halogénoalkyle ;
- (C₂-C₆)-alcényle, (C₂-C₆)-halogénoalcényle, (C₂-C₆)-alcénylcarbonyle, (C₂-C₆)-halogénoalcénylcarbonyle, (C₂-C₆)-alcényloxy, (C₂-C₆)-halogénoalcényloxy, (C₂-C₆) -alcényloxycarbonyle, (C₂-C₆)-halogénoalcényloxycarbonyle ;
- (C₂-C₆)-alcynyle, (C₂-C₆)-halogénoalcynyle, (C₂-C₆)-alcynylcarbonyle, (C₂-C₆)-halogénoalcynylcarbonyle, (C₂-C₆)-alcynyloxy, (C₂-C₆)-halogénoalcynyloxy, (C₂-C₆)-alcynyloxycarbonyle, (C₂-C₆)-halogénoalcynyloxycarbonyle ;
- tri-(C₁-C₆)-alkylsilyl-(C₂-C₆)-alcynyle, di-(C₁-C₆)-alkylsilyl-(C₂-C₆)-alcynyle, mono-(C₁-C₆)-alkylsilyl-(C₂-C₆)-alcynyle, phénylsilyl-(C₂-C₆)-alcynyle ;
- (C₆-C₁₄)-aryle, (C₆-C₁₄)-aryloxy, (C₆-C₁₄)-arylcarbonyle et (C₆-C₁₄)-aryloxycarbonyle, qui peuvent à chaque fois être substitués sur la partie aryle par halogène, (C₁-C₆)-alkyle et/ou (C₁-C₆)-halogénoalkyle ;
- (C₆-C₁₄)-aryl-(C₁-C₆)-alkyle, (C₆-C₁₄)-aryl-(C₁-C₆)-alcoxy, (C₆-C₁₄)-aryl-(C₁-C₆)-alkylcarbonyle, (C₆-C₁₄) -aryl- (C₁-C₆) -alkylcarbonyloxy, (C₆-C₁₄) -aryl-(C₁-C₆) -alcoxycarbonyle, (C₆-C₁₄) -aryl- (C₁-C₆)-alcoxycarbonyloxy ;
- aminocarbonyl-(C₁-C₆)-alkyle, di-(C₁-C₆)-alkylaminocarbonyl-(C₁-C₆)-alkyle ;
- mono- (C₁-C₆)-alkyl)-aminocarbonyle, mono-((C₁-C₆)-halogénoalkyl)-aminocarbonyle, di-((C₁-C₆)-alkyl)-aminocarbonyle, di-((C₁-C₆)-halogénoalkyl)-aminocarbonyle, ((C₁-C₆)-alkyl-(C₁-C₆)-halogénoalkyl)-aminocarbonyle, N-((C₁-C₆)-alcanoyl)-aminocarbonyle, N-((C₁-C₆)-halogénoalcanoyl)-aminocarbonyle, mono-((C₆-C₁₄)-aryl)-aminocarbonyle, di- ((C₆-C₁₄)-aryl)-aminocarbonyle ;
- (C₁-C₆)-alcoxy-(C₁-C₆)-alkyle, (C₁-C₆)-alcoxy-(C₁-C₆)-alcoxy, (C₁-C₆)-alcoxycarbonyl-(C₁-C₆)-alcoxy ;
- (C₃-C₈)-cycloalkyle, qui peut le cas échéant être substitué sur le radical cycloalkyle par (C₁-C₆)-alkyle et/ou halogène ; (C₃-C₈)-cycloalcoxy, (C₃-C₈) -cycloalkyl- (C₁-C₆) -alkyle, (C₃-C₈) -cycloalkyl-(C₁-C₆) -halogénoalkyle, (C₃-C₈) -cycloalkyl- (C₁-C₆)-alcoxy, (C₃-C₈) -cycloalkyl- (C₁-C₆) -halogénoalcoxy, (C₃-C₈) -cycloalkylcarbonyle, (C₃-C₈)-cycloalcoxycarbonyle, (C₃-C₈)-cycloalkyl-(C₁-C₆)-alkylcarbonyle, (C₃-C₈) -cycloalkyl- (C₁-C₆)-halogénoalkylcarbonyle, (C₃-C₈) -cycloalkyl- (C₁-C₆)-alcoxycarbonyle, (C₃-C₈) -cycloalkyl- (C₁-C₆)-halogénoalcoxycarbonyle, (C₃-C₈)-cycloalkylcarbonyloxy, (C₃-C₈)-cycloalcoxycarbonyloxy, (C₃-C₈) -cycloalkyl- (C₁-C₆)-alkylcarbonyloxy, (C₃-C₈) -cycloalkyl- (C₁-C₆)-halogénoalkylcarbonyloxy, (C₃-C₈)-cycloalkyl-(C₁-C₆) -alcoxycarbonyloxy, (C₃-C₈) -cycloalkyl- (C₁-C₆)-halogénoalcoxycarbonyloxy ;
- (C₃-C₈) -cycloalcényle, (C₃-C₈) -cycloalcényloxy, (C₃-C₈) -cycloalcényl- (C₁-C₆) -alkyle, (C₃-C₈)-cycloalcényl- (C₁-C₆) -halogénoalkyle, (C₃-C₈)-cycloalcényl- (C₁-C₆) -alcoxy, (C₃-C₈) -cycloalcényl-(C₁-C₆)-halogénoalcoxy, (C₃-C₈)-cycloalcénylcarbonyle, (C₃-C₈)-cycloalcényloxycarbonyle, (C₃-C₈)-cycloalcényl-(C₁-C₆)-alkylcarbonyle, (C₃-C₈)-cycloalcényl-(C₁-C₆)-halogénoalkylcarbonyle, (C₃-C₈)-cycloalcényl-(C₁-C₆) -alcoxycarbonyle, (C₃-C₈) -cycloalcényl- (C₁-C₆) halogénoalcoxycarbonyle, (C₃-C₈)-cycloalcénylcarbonyloxy, (C₃-C₈)-cycloalcényloxycarbonyloxy, (C₃-C₈)-cycloalcényl-(C₁-C₆) -alkylcarbonyloxy, (C₃-C₈) -cycloalcényl- (C₁-C₆) -halogénoalkylcarbonyloxy, (C₃-C₈)-cycloalcényl-(C₁-C₆) -alcoxycarbonyloxy, (C₃-C₈) -cycloalcényl- (C₁-C₆)-halogénoalcoxycarbonyloxy ;
- hydroxy-(C₁-C₆)-alkyle, hydroxy-(C₁-C₆)-alcoxy; cyano-(C₁-C₆)-alcoxy, cyano-(C₁-C₆)-alkyle ;
- (C₁-C₆)-alkylsulfonyle, (C₁-C₆)-alkylthio, (C₁-C₆)-alkylsulfinyle, (C₁-C₆)-halogénoalkylsulfonyle, (C₁-C₆) -halogénoalkylthio, (C₁-C₆)-halogénoalkylsulfinyle, (C₁-C₆)-alkylsulfonyl-(C₁-C₆)-alkyle, (C₁-C₆) -alkylthio- (C₁-C₆) -alkyle, (C₁-C₆) -alkylsulfinyl- (C₁-C₆) -alkyle, (C₁-C₆)-halogénoalkylsulfonyl-(C₁-C₆) -alkyle, (C₁-C₆)-halogénoalkylthio- (C₁-C₆) -alkyle, (C₁-C₆)-halogénoalkylsulfinyl-(C₁-C₆)-alkyle, (C₁-C₆)-alkylsulfonyl-(C₁-C₆)-halogénoalkyle, (C₁-C₆)-alkylthio- (C₁-C₆) -halogénoalkyle, (C₁-C₆)-alkylsulfinyl-(C₁-C₆)-halogénoalkyle, (C₁-C₆)-halogénoalkylsulfonyl-(C₁-C₆)-halogénoalkyle, (C₁-C₆) -halogénoalkylthio- (C₁-C₆) -halogénoalkyle, (C₁-C₆)-halogénoalkylsulfinyl-(C₁-C₆)-halogénoalkyle, (C₁-C₆)-alkylsulfonyloxy, (C₁-C₆)-halogénoalkylsulfonyloxy, (C₁-C₆)-alkylthiocarbonyle, (C₁-C₆)-halogénoalkylthiocarbonyle, (C₁-C₆)-alkylthiocarbonyloxy, (C₁-C₆)-halogénoalkylthiocarbonyloxy, (C₁-C₆)-alkylthio-(C₁-C₆) -alkyle, (C₁-C₆) -alkylthio- (C₁-C₆) -alcoxy, (C₁-C₆) -alkylthio- (C₁-C₆) -alkylcarbonyle, (C₁-C₆)-alkylthio- (C₁-C₆) -alkylcarbonyloxy, (C₄-C₁₄)-arylsulfonyle, (C₆-C₁₄)-arylthio, (C₆-C₁₄)-arylsulfinyle, (C₃-C₈)-cycloalkylthio, (C₃-C₈)-alcénylthio, (C₃-C₈)-cycloalcénylthio, (C₃-C₆)-alcynylthio ; et
R^{2a} et R^{2b}, à chaque fois indépendamment l'un de l'autre, sont choisis dans le groupe constitué par
- hydrogène, halogène, hydroxy, cyano, nitro, C(O)OH, C(O)NH₂ ;
- (C₁-C₆)-alkyle, (C₁-C₆)-halogénoalkyle, (C₁-C₆)-alkylcarbonyle, (C₁-C₆)-halogénoalkylcarbonyle, (C₁-C₆) -alkylcarbonyloxy, (C₁-C₆)-halogénoalkylcarbonyloxy, (C₁-C₆)-alkylcarbonyl-(C₁-C₄)-alkyle ;
- (C₁-C₆) -alcoxy, (C₁-C₆) -halogénoalcoxy, (C₁-C₆)-alcoxycarbonyle, (C₁-C₆)-halogénoalcoxycarbonyle, (C₁-C₆) -alcoxycarbonyl- (C₁-C₆) -alkyle, (C₁-C₆)-halogénoalcoxycarbonyl- (C₁-C₆) -alkyle, (C₁-C₆)-alcoxycarbonyl- (C₁-C₆) -halogénoalkyle, (C₁-C₆)-halogénoalcoxycarbonyl-(C₁-C₆)-halogénoalkyle ;
- (C₂-C₆)-alcényle, (C₂-C₆)-halogénoalcényle, (C₂-C₆)-alcénylcarbonyle, (C₂-C₆)-halogénoalcénylcarbonyle, (C₂-C₆)-alcényloxy, (C₂-C₆)-halogénoalcényloxy, (C₂-C₆) -alcényloxycarbonyle, (C₂-C₆)-halogénoalcényloxycarbonyle ;
- (C₂-C₆)-alcynyle, (C₂-C₆)-halogénoalcynyle, (C₂-C₆)-alcynylcarbonyle, (C₂-C₆)-halogénoalcynylcarbonyle, (C₂-C₆)-alcynyloxy, (C₂-C₆)-halogénoalcynyloxy, (C₂-C₆) -alcynyloxycarbonyle, (C₂-C₆)-halogénoalcynyloxycarbonyle ;
- tri-(C₁-C₆)-alkylsilyl-(C₂-C₆)-alcynyle, di-(C₁-C₆)-alkylsilyl-(C₂-C₆)-alcynyle, mono-(C₁-C₆)-alkylsilyl-(C₂-C₆)-alcynyle, phénylsilyl-(C₂-C₆)-alcynyle ;
- (C₆-C₁₄)-aryle, (C₆-C₁₄)-aryloxy, (C₆-C₁₄)-arylcarbonyle et (C₆-C₁₄)-aryloxycarbonyle, qui peuvent à chaque fois être substitués sur la partie aryle par halogène, (C₁-C₆)-alkyle et/ou (C₁-C₆)-halogénoalkyle ;
- (C₆-C₁₄)-aryl-(C₁-C₆)-alkyle, (C₆-C₁₄)-aryl- (C₁-C₆) alcoxy, (C₆-C₁₄)-aryl-(C₁-C₆)-alkylcarbonyle, (C₆-C₁₄) -aryl- (C₁-C₆) -alkylcarbonyloxy, (C₆-C₁₄) -aryl-(C₁-C₆) -alcoxycarbonyle, (C₆-C₁₄) -aryl- (C₁-C₆)-alcoxycarbonyloxy ;
- N-((C₁-C₆)-halogénoalcanoyl)-amino, aminocarbonyl-(C₁-C₆)-alkyle, di-(C₁-C₆)-alkylaminocarbonyl-(C₁-C₆)-alkyle ;
- N-((C₁-C₆)-halogénoalcanoyl)-aminocarbonyle, mono-((C₆-C₁₄) -aryl) -aminocarbonyle, di- ((C₆-C₁₄)-aryl)-aminocarbonyle ;
- (C₁-C₆)-alcoxy-(C₁-C₆)-alkyle, (C₁-C₆) -alcoxy-(C₁-C₆)-alcoxy, (C₁-C₆)-alcoxycarbonyl-(C₁-C₆)-alcoxy ;
- (C₃-C₈)-cycloalkyle, qui peut le cas échéant être substitué sur le radical cycloalkyle par (C₁-C₆)-alkyle et/ou halogène ; (C₃-C₈)-cycloalcoxy, (C₃-C₈) -cycloalkyl- (C₁-C₆) -alkyle, (C₃-C₈) -cycloalkyl-(C₁-C₆) -halogénoalkyle, (C₃-C₈) -cycloalkyl- (C₁-C₆)-alcoxy, (C₃-C₈) -cycloalkyl- (C₁-C₆) -halogénoalcoxy, (C₃-C₈) -cycloalkylcarbonyle, (C₃-C₈)-cycloalcoxycarbonyle, (C₃-C₈) -cycloalkyl- (C₁-C₆)-alkylcarbonyle, (C₃-C₈) -cycloalkyl- (C₁-C₆)-halogénoalkylcarbonyle, (C₃-C₈)-cycloalkyl-(C₁-C₆)-alcoxycarbonyle, (C₃-C₈)-cycloalkyl-(C₁-C₆)-halogénoalcoxycarbonyle, (C₃-C₈)-cycloalkylcarbonyloxy, (C₃-C₈)-cycloalcoxycarbonyloxy, (C₃-C₈) -cycloalkyl- (C₁-C₆) alkylcarbonyloxy, (C₃-C₈)-cycloalkyl-(C₁-C₆)-halogénoalkylcarbonyloxy, (C₃-C₈)-cycloalkyl-(C₁-C₆) -alcoxycarbonyloxy, (C₃-C₈) -cycloalkyl- (C₁-C₆) halogénoalcoxycarbonyloxy ;
- (C₃-C₈) -cycloalcényle, (C₃-C₈) -cycloalcényloxy, (C₃-C₈) -cycloalcényl- (C₁-C₆) -alkyle, (C₃-C₈)-cycloalcényl-(C₁-C₆)-halogénoalkyle, (C₃-C₈)-cycloalcényl- (C₁-C₆) -alcoxy, (C₃-C₈) -cycloalcényl-(C₁-C₆) -halogénoalcoxy, (C₃-C₈)-cycloalcénylcarbonyle, (C₃-C₈)-cycloalcényloxycarbonyle, (C₃-C₈)-cycloalcényl-(C₁-C₆) -alkylcarbonyle, (C₃-C₈) -cycloalcényl- (C₁-C₆) halogénoalkylcarbonyle, (C₃-C₈)-cycloalcényl-(C₁-C₆) -alcoxycarbonyle, (C₃-C₈) -cycloalcényl- (C₁-C₆) halogénoalcoxycarbonyle, (C₃-C₈)-cycloalcénylcarbonyloxy, (C₃-C₈)-cycloalcényloxycarbonyloxy, (C₃-C₈)-cycloalcényl-(C₁-C₆) -alkylcarbonyloxy, (C₃-C₈) -cycloalcényl- (C₁-C₆)-halogénoalkylcarbonyloxy, (C₃-C₈)-cycloalcényl-(C₁-C₆) -alcoxycarbonyloxy, (C₃-C₈) -cycloalcényl-(C₁-C₆)-halogénoalcoxycarbonyloxy ;
- hydroxy-(C₁-C₆)-alkyle, hydroxy-(C₁-C₆)-alcoxy cyano-(C₁-C₆)-alcoxy, cyano-(C₁-C₆)-alkyle ;
- (C₁-C₆)-alkylsulfonyle, (C₁-C₆)-alkylthio, (C₁-C₆)-alkylsulfinyle, (C₁-C₆)-halogénoalkylsulfonyle, (C₁-C₆) -halogénoalkylthio, (C₁-C₆)-halogénoalkylsulfinyle, (C₁-C₆)-alkylsulfonyl-(C₁-C₆)-alkyle, (C₁-C₆)-alkylthio- (C₁-C₆) -alkyle, (C₁-C₆) -alkylsulfinyl- (C₁-C₆) -alkyle, (C₁-C₆)-halogénoalkylsulfonyl-(C₁-C₆) -alkyle, (C₁-C₆)-halogénoalkylthio- (C₁-C₆) -alkyle, (C₁-C₆)-halogénoalkylsulfinyl- (C₁-C₆) -alkyle, (C₁-C₆)-alkylsulfonyl-(C₁-C₆)-halogénoalkyle, (C₁-C₆)-alkylthio- (C₁-C₆) -halogénoalkyle, (C₁-C₆)-alkylsulfinyl-(C₁-C₆)-halogénoalkyle, (C₁-C₆)-halogénoalkylsulfonyl-(C₁-C₆)-halogénoalkyle, (C₁-C₆) -halogénoalkylthio- (C₁-C₆) -halogénoalkyle, (C₁-C₆)-halogénoalkylsulfinyl-(C₁-C₆)-halogénoalkyle, (C₁-C₆)-alkylsulfonyloxy, (C₁-C₆)-halogénoalkylsulfonyloxy, (C₁-C₆)-alkylthiocarbonyle, (C₁-C₆) halogénoalkylthiocarbonyle, (C₁-C₆)-alkylthiocarbonyloxy, (C₁-C₆)-halogénoalkylthiocarbonyloxy, (C₁-C₆)-alkylthio-(C₁-C₆)-alkyle, (C₁-C₆) -alkylthio- (C₁-C₆) -alcoxy, (C₁-C₆) -alkylthio- (C₁-C₆) -alkylcarbonyle, (C₁-C₆) alkylthio- (C₁-C₆) -alkylcarbonyloxy, (C₄-C₁₄)-arylsulfonyle, (C₆-C₁₄)-arylthio, (C₆-C₁₄)-arylsulfinyle, (C₃-C₆)-cycloalkylthio, (C₃-C₈)-alcénylthio, (C₃-C₈)-cycloalcénylthio, (C₃-C₆)-alcynylthio ; et
R³ est choisi dans le groupe constitué par
- hydrogène, cyano, C(O)OH, C(O)NH₂ ;
- (C₁-C₆)-alkyle et (C₁-C₆)-halogénoalkyle ;
- (C₁-C₆)-alcoxycarbonyle ; et
R⁴ et R⁵, à chaque fois indépendamment l'un de l'autre, sont choisis dans le groupe constitué par hydrogène, (C₁-C₆)-alkyle, (C₁-C₆)-halogénoalkyle, hydroxy, (C₁-C₆)-alcoxy et (C₁-C₆)-halogénoalcoxy ; ou les radicaux R⁴ et R⁵, ensemble avec l'atome de carbone auquel ils sont liés, forment un cycle de trois à sept chaînons ; et
R⁶ et R⁷, à chaque fois indépendamment l'un de l'autre, sont choisis dans le groupe constitué par hydrogène, (C₁-C₆)-alkyle, (C₁-C₆)-halogénoalkyle, (C₁-C₆)-alcoxy, (C₁-C₆)-halogénoalcoxy, (C₆-C₁₄)-aryle, (C₆-C₁₄)-aryloxy, (C₆-C₁₄)-arylcarbonyle et (C₆-C₁₄)-aryloxycarbonyle ; ou les radicaux R⁶ et R⁷ forment ensemble un groupe (C₁-C₇)-alkylène qui peut contenir un ou plusieurs atomes d'oxygène et/ou de soufre, le groupe (C₁-C₇)-alkylène pouvant être monosubstitué ou polysubstitué par halogène et les différents substituants halogène pouvant être identiques ou différents, et
n vaut le nombre 0, 1 ou 2 ; et
R⁸, R⁹, R¹⁰ et R¹¹, à chaque fois indépendamment les uns des autres, sont choisis dans le groupe constitué par hydrogène, halogène, cyano, C(O)OH, C(O)NH₂, (C₁-C₆)-alkyle, (C₁-C₆) -alkylcarbonyle, (C₁-C₆) -alkyloxycarbonyle, (C₁-C₆) alkylaminocarbonyle, (C₁-C₆)-di-alkylaminocarbonyle, (C₁-C₆)-halogénoalkyle, (C₁-C₆)-alcoxy, (C₁-C₆) -halogénoalcoxy, (C₂-C₆)-alcényle, (C₂-C₆)-alcynyle, (C₂-C₆)-halogénoalcynyle, (C₂-C₆)-alcynylcarbonyle, (C₂-C₆)-halogénoalcynylcarbonyle, (C₂-C₆)-alcynyloxy, (C₂-C₆)-halogénoalcynyloxy, (C₂-C₆)-alcynyloxycarbonyle et (C₂-C₆)-halogénoalcynyloxycarbonyle et nitro ;
X représente une liaison, CH₂, O, S, carbonyle, NH, CR¹²R¹³ et NR¹⁴ ou CH₂O, CH₂S, où, dans les deux derniers groupes mentionnés, l'atome de carbone est lié à la partie aromatique et l'hétéroatome O ou S est lié à la partie partiellement hydrogénée de l'amine ; et
R¹² et R¹³, à chaque fois indépendamment l'un de l'autre, sont choisis dans le groupe constitué par hydrogène, (C₁-C₆)-alkyle et (C₁-C₆)-halogénoalkyle ; et
R¹⁴ est choisi dans le groupe constitué par hydrogène, (C₁-C₆)-alkyle, (C₁-C₆) -halogénoalkyle.

2. Composés de formule générale (I) selon la revendication 1, **caractérisés en ce que** R¹ est choisi dans le groupe constitué par cyano (CN), NO₂, (C₁-C₃)-alkyle, (C₁-C₆) -halogénoalkyle, (C₁-C₆)-alcoxy, (C₁-C₆)-halogénoalcoxy, (C₁-C₆) -alkylthio, (C₁-C₆)-alkylsulfoxyde, (C₁-C₆)-alkylsulfonyle, (C₁-C₆)-halogénoalkylsulfonyle, (C₁-C₆)-alcoxycarbonyle, aminocarbonyle, mono-((C₁-C₆)-alkyl)-aminocarbonyle, di-((C₁-C₆)-alkyl)-aminocarbonyle, (C₆-C₁₄)-aryle, (C₆-C₁₄)-arylhalogénoalkyle, (C₁-C₆)-aryloxy, (C₆-C₁₄)-halogénoaryl- (C₁-C₆) -alkyle, (C₂-C₆)-alcynyl- (C₁-C₆)-alcoxy, (C₂-C₆)-alcynyl-(C₁-C₆)-alkyl-(C₁-C₆)-alcoxy et (C₁-C₆)-alkyl-(C₂-C₆)-alcynyl-(C₁-C₆)-alcoxy.

3. Composés de formule générale (I) selon la revendication 1 ou 2, **caractérisés en ce que** les radicaux R^{2a} et R^{2b}, à chaque fois indépendamment l'un de l'autre, sont choisis dans le groupe constitué par hydrogène, CN, hydroxy (OH), (C₁-C₆)-alkyle, (C₁-C₆)-halogénoalkyle, (C₁-C₆)-cycloalkyle, (C₁-C₆)-alcoxy, aminocarbonyle, hydroxycarbonyle COOH, (C₁-C₆)-alcoxycarbonyle, (C₁-C₆)-alkylsulfonyle, mono-((C₁-C₆)-alkyl)-aminocarbonyle, di-((C₁-C₆)-alkyl)-aminocarbonyle, mono-((C₆-C₁₄)-aryl)-aminocarbonyle, mono- ((C₂-C₆)-alkylène) -aminocarbonyle, mono- ((C₆-C₁₄)-aryl- (C₁-C₆) -alkyl) -aminocarbonyle, (C₁-C₆) -alcoxy-(C₁-C₆)-alcoxycarbonyle et di- ((C₁-C₆)-alkyl)-amino-(C₁-C₆)-alcoxycarbonyle.

4. Composés de formule générale (I) selon l'une quelconque des revendications 1 à 3, **caractérisés en ce que** le radical R³ est choisi dans le groupe constitué par hydrogène, CH₃, CH₂CH₂OCH₃, COOCH₃, CONH₂, et C₆H₅.

5. Composés de formule générale (I) selon l'une quelconque des revendications 1 à 4, **caractérisés en ce que** les radicaux R⁴ et R⁵, à chaque fois indépendamment l'un de l'autre, sont choisis dans le groupe constitué par hydrogène, cyano, hydroxy, (C₁-C₆)-alkyle et (C₁-C₆)-alcoxy.

6. Composés de formule générale (I) selon l'une quelconque des revendications 1 à 4, **caractérisés en ce que** les radicaux R⁴ et R⁵ représentent un groupe (C₁-C₃)-alkylène et forment, ensemble avec l'atome de carbone auquel ils sont liés, un cycle de trois ou quatre chaînons.

7. Composés de formule générale (I) selon l'une quelconque des revendications 1 à 6, **caractérisés en ce que** R⁶ et R⁷ sont choisis, indépendamment l'un de l'autre, dans le groupe constitué par hydrogène, méthyle et phényle.

8. Composés de formule générale (I) selon l'une quelconque des revendications 1 à 7, **caractérisés en ce que** le radical R⁸ est choisi dans le groupe constitué par hydrogène, halogène, (C₁-C₆)-alkyle, (C₁-C₆)-alcoxy et di-((C₁-C₆)-alkyl)-amino.

9. Composés de formule générale (I) selon l'une quelconque des revendications 1 à 8, **caractérisés en ce que** le radical R⁹ est choisi dans le groupe constitué par hydrogène, chlore, fluor, (C₁-C₆)-alkyle et (C₁-C₆)-alcoxy.

10. Composés de formule générale (I) selon l'une quelconque des revendications 1 à 9, **caractérisés en ce que** le radical R¹⁰ est choisi dans le groupe constitué par hydrogène, fluor, chlore, brome, cyano, CONH₂, COOH, méthyle (CH₃), éthyle (CH₂CH₃), méthoxy (OCH₃), CH=CH₂, C=CH et C≡CCH₃.

11. Composé de formule générale (I) selon l'une quelconque des revendications 1 à 10, **caractérisé en ce que** le radical R¹¹ est choisi dans le groupe constitué par hydrogène et méthyle.

12. Composé de formule générale (I) selon l'une quelconque des revendications 1 à 11, **caractérisé en ce que** le radical X est choisi dans le groupe constitué par O, S, carbonyle, CH₂, NH, CHCH₃, NCH₃, C(CH₃)₂, OCH₂, SCH₂, ou dans lequel X représente une liaison chimique.

13. Procédé pour la préparation de composés de formule générale (I) et/ou de leurs sels acceptables sur le plan agrochimique et/ou de leurs dérivés d'azote quaternaire acceptables sur le plan agrochimique : 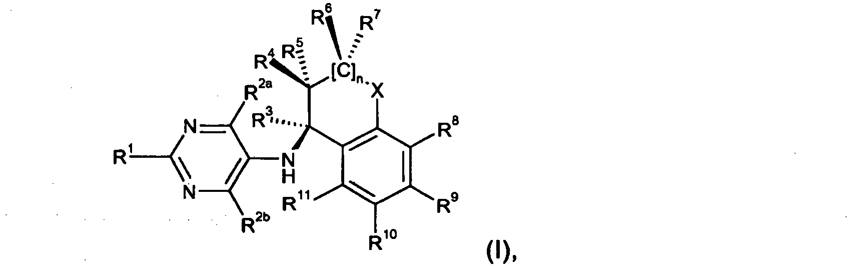 dans laquelle les radicaux R¹ à R¹¹ et X ainsi que n sont définis comme dans l'une quelconque des revendications 1 à 12, et dans lequel
- un composé de formule générale (II) 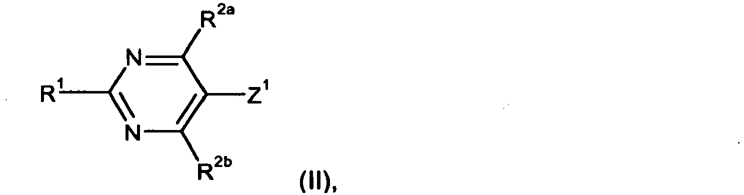 dans laquelle R¹ et R^{2a} ainsi que R^{2b} sont définis comme dans l'une quelconque des revendications 1 à 12 et Z¹ représente un radical échangeable ou un groupe partant,
- est transformé avec une amine de formule générale (III) ou avec un sel d'addition d'acide de l'amine de formule générale (III) 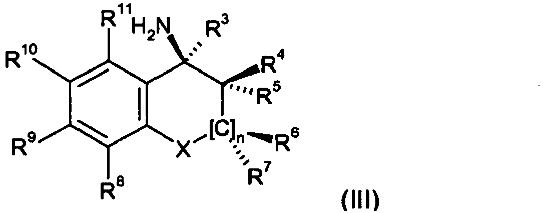 dans laquelle les radicaux R³ à R¹¹ et X ainsi que n ont la signification indiquée ci-dessus.

14. Procédé selon la revendication 13, le radical échangeable ou le groupe partant Z¹ représentant fluor, chlore, brome, iode, (C₁-C₄)-alkylsulfonyle ; phényl-(C₁-C₄)-alkylsulfonyle substitué ou non substitué ou (C₁-C₄)-alkylphénylsulfonyle.

15. Agent herbicide ou de régulation de la croissance de plantes, **caractérisé en ce qu'**il contient un ou plusieurs composés de formule générale (I) ou leurs sels selon l'une quelconque des revendications 1 à 12.

16. Procédé pour lutter contre des plantes nuisibles ou pour la régulation de la croissance de plantes, **caractérisé en ce qu'**on applique une quantité active d'un ou de plusieurs composés de formule générale (I) ou de leurs sels selon l'une quelconque des revendications 1 à 12 sur des plantes, des parties de plantes, des graines de plantes ou sur une surface de culture.

17. Utilisation de composés de formule générale (I) ou de leurs sels selon l'une quelconque des revendications 1 à 12 comme herbicides ou régulateurs de la croissance de plantes.

18. Utilisation selon la revendication 17, **caractérisée en ce que** les composés de formule générale (I) ou leurs sels sont utilisés pour lutter contre des plantes nuisibles ou pour la régulation de la croissance de plantes dans des cultures de plantes utiles ou décoratives.

19. Utilisation selon la revendication 18, **caractérisée en ce que** les plantes de culture sont des plantes de culture transgéniques.

20. Utilisation selon la revendication 17 ou 18, **caractérisée en ce que** les plantes de culture sont choisies parmi les cultures en plantation.
